# EUROPEAN PATENT APPLICATION

(11) **EP 4 070 832 A1**
(43) Date of publication of application: **12.10.2022**
(21) Application number: 21167293.6
(22) Date of filing: 07.04.2021
(51) Int. Cl.: A61M 5/24

(54) **KEYED CONNECTORS**

(71) Applicant: medmix Switzerland AG, 9469 Haag (Rheintal) (CH)
(72) Inventor: KEITEL, Joachim, 73728 Esslingen (DE)
(74) Representative: Manitz Finsterwald Patent- und Rechtsanwaltspartnerschaft mbB

(57) **Abstract**

A set of at least two drug delivery devices (10) comprising a first drug delivery device and a second drug delivery device, wherein the first drug delivery device and the second drug delivery device each comprise a first set of mutual members that are identical in both the first drug delivery device and the second drug delivery device, and wherein the first drug delivery device and the second drug delivery device each comprise a second set of distinguishing members that differ among the first and second drug delivery device. The first drug delivery device comprises first connection means (510) for attaching a first dispensing unit (410) to the first drug delivery device and the second drug delivery device comprises second connection means for attaching a second dispensing unit to the second drug delivery device, wherein the first and second connection means differ from each other so that the second dispensing unit is not attachable to the first connection means and the first dispensing unit is not attachable to the second connection means.

## Description

The invention relates to a set of at least two drug delivery devices comprising at least a first injection device and a second injection device. Furthermore, the invention relates to a set of dispensing units comprising a first dispensing unit and a second dispensing unit, each dispensing unit configured to have a drug compartment containing a drug to be delivered by one of the drug delivery devices of said sets.

To date, drug delivery devices which can be used by medically non-trained people, such as for example patients, to self-administer medicaments are becoming more and more sophisticated in view of their dose setting mechanisms and/or their dose delivery mechanisms. Uses of such devices may include, for example, diabetics, where medication management, i.e. the degree to which a patient follows medical instructions and protocols which may originate from a medically trained person such as a doctor, is often of extreme importance. Of the known types of drug delivery devices, which can be actuated manually, semi-automatically or automatically to eject a drug out of a drug compartment, the pen-type device has become very popular such that it is now available both in reusable and disposable designs.

Disposable drug delivery devices are completely discarded once the drug compartment of the device has been emptied to a degree that no further dose of medicament can be ejected from the device. With single use devices, the device is discarded after a single dose has been ejected, while multi-use devices allow the repeated ejection of several doses from the same medicament container or drug compartment.

With reusable devices, the drug delivery device includes the possibility to reset the delivery device such that the medicament container can be replaced with a new one when the last dose has been delivered from the container. Said emptying of the container may happen after one dose ejection or after several dose ejections. Thus, in reusable devices it can be of particular interest not only to be able to set a precise amount of drug to be ejected but also to being able to attach a new drug compartment to an existing drug delivery device.

To date there exists a great amount of different medications and/or medications of different concentrations which can be self-administered with such reusable delivery devices. Therefore, it has shown to be crucial for the patient to be particularly mindful of which medicament container, i. e. which medicament or medicament concentration, is attached to the delivery device.

Therefore, it is an object of the invention to provide a set of delivery devices as well as a set of dispensing units, with which the safety of use as well as the precise medication management can be improved. This object is solved by the subject matter of the independent claims.

Particularly, according to the invention a set of at least two drug delivery devices comprising at least a first drug delivery device and a second drug delivery device is provided. Each one of the first and second drug delivery devices comprises a housing, a dosing mechanism within said housing comprising a dose setting member for setting an injection dose, a dose indication member for visual indication of the set dose, and a piston rod configured to move in a proximal direction out of the housing for ejecting the drug, the piston rod further being operatively coupled to the dose setting member. The piston rod may be rotationally fixed with respect to the housing at least during dose delivery. Alternatively, it also may be configured to rotate with respect to the housing during dose delivery. The first drug delivery device and the second drug delivery device each comprise a first set of mutual members that are identical in both the first drug delivery device and the second drug delivery device, the first set comprising at least one mutual member. Furthermore, the first drug delivery device and the second drug delivery device each comprise a second set of distinguishing members that differ among the first and second drug delivery device, the second set comprising at least one distinguishing member. Additionally, the first drug delivery device comprises first connection means for attaching a first dispensing unit to the housing of the first drug delivery device and the second drug delivery device comprises second connection means for attaching a second dispensing unit to the housing of the second drug delivery device, wherein the first and second connection means differ from each other so that the second dispensing unit is not attachable to the first connection means and the first dispensing unit is not attachable to the second connection means.

In this connection it is noted that the first and second drug delivery devices may be designed as reusable drug delivery devices.

Furthermore, it is noted that when the piston rod moves in a proximal direction for ejecting the drug, it can be regarded that said set dose is ejected from a drug compartment of a dispensing unit attached to the housing.

Furthermore, it is noted that throughout the application text, the expressions "proximal" and "distal" refer to parts of the delivery device, which are closer to or further away from the body of a patient, respectively, and which are therefore closer to or further away from a delivery or injection site, respectively. Hence, the proximal end of the drug delivery device is the part which is connected to the dispensing unit, and thus optionally closest to a needle that may be attached to the dispensing unit, whereas the distal end of the drug delivery device is the part which may comprise the dose setting member that may be configured to be actuated by the user.

As can be seen from the above, the invention provides a set of drug delivery devices, i.e. at least a first drug delivery device and a second drug delivery device, which drug delivery devices consist of several members. Some of said members may be identical for both drug delivery devices, thereby forming a part of each set of mutual members.

Hence, in this connection the term "mutual members" means that there exist components in all, e. g. both, drug delivery devices of the set of drug delivery devices that are formed to be (mechanically) identical and interchangeable in assembly. This means mutual members are members that can be exchanged between two different delivery devices and thereby maintain the function and/or appearance thereof. Despite from being mechanically identical, mutual members are also identical in appearance.

Furthermore, each drug delivery device may further comprise a second set of members, i.e. a set of distinguishing members, with said distinguishing members being different in some way among the first drug delivery device and the second drug delivery device. This can, for example, mean that there exist members which are mechanically different among the two drug delivery devices. Additionally or alternatively, this could also mean that said distinguishing members of the first drug delivery device and the second drug delivery device differ from each other in their appearance. That is, they may comprise a different colour or may have different prints on them such that the user can be able to distinguish the two drug delivery devices from the set of devices from each other.

Hence, said distinguishing members are members that differ mechanically, and/or in appearance and are not interchangeable in assembly and use. This means that they cannot simply be exchanged for parts of the same or similar function in the respective other drug delivery device without the function being compromised in one way or another. In this connection it is noted that a function can also include differences in appearance, such as a colour coding or labelled coding or the like, which visually distinguishes the drug delivery devices among each other.

As a further security feature the first drug delivery device comprises first connection means for attaching a first dispensing unit to the housing of the first drug delivery device and the second drug delivery device comprises second connection means for attaching a second dispensing unit to the housing of the second drug delivery device, wherein the first and second connection means differ from each other so that the second dispensing unit is not attachable to the first connection means and the first dispensing unit is not attachable to the second connection means. This further helps the user to distinguish the two drug delivery devices from the set of devices since the first dispensing unit can simply not be attached to the second drug delivery device and vice versa. Furthermore, it allows to easily recognize the dispensing unit attached to the device and also the drug contained within the dispensing unit based on the visual appearance of the drug delivery device, for example, with a cap attached to the device.

Stated differently, the first and second connection means are configured as keyed connectors that prevent a cross-use of the second dispensing unit with the first drug delivery device or a cross-use of the first dispensing unit with the second drug delivery device. This enhances the safety during use of the first and second drug delivery device as it is assured that only a specific dispensing unit is attachable to a given one of the first and second drug delivery devices.

There exist several types of diseases, such as for example diabetes, for which a patient needs to receive different types, doses or concentrations of medication throughout the day. Said patients often carry several drug delivery devices with them with which they can self-inject the respective medication at a given time. Different drug delivery devices for different medications may often be needed since such delivery devices may include dosing mechanisms with which different doses depending on the application field, the patient or the precise medication (concentration) can be set. Hence, it is crucial to guarantee that the correct medication is attached to the drug delivery device which is about to be used.

To make things worse, some patients may further suffer from poor eyesight or the like such that it is difficult for them to distinguish their different drug delivery devices purely visually. For such cases it is even more crucial to have an additional safety mechanism which ensures that only the right dispensing unit containing the right medication can be mounted on the drug delivery device which is suited for said medication.

The invention can solve this issue by not only providing a set of distinguishing members for each one of the two drug delivery devices with which the delivery devices may be distinguished by the user. According to the invention, as already mentioned above, the first and second drug delivery device comprise first and second connection means, respectively, which guarantee that only the right dispensing unit can be attached to the corresponding drug delivery device.

The first connection means and second connection means can generally be designed in different ways such as threads, bayonet locks, snap locks or the like. It can further be either possible that the first drug delivery device and the second drug delivery device comprise the same type of connection means or a different type of connection means. It seems to be quite obvious that if the devices comprise connection means of different types a distinction between the two is easy.

It is pointed out that even if both devices comprise the same type of connection means, they nevertheless differ in such a way that the first drug delivery device cannot be connected with the second dispensing unit and the second drug delivery device cannot connected with the first dispensing unit.

Providing at least one mutual member that is identical among the first and second drug delivery device allows efficient manufacturing of the individual drug delivery devices of the set according to the present disclosure. The mutual members may be manufactured and held available for the manufacturing of all drug delivery devices of the set so that separate production runs or stock-keeping is not necessary.

According to an embodiment the dosing mechanisms of the first drug delivery device and the second delivery device each comprise, for example consist of, a same set of functional members that includes the first set and the second set. That is, in some embodiments the dosing mechanisms of the first drug delivery device and the second drug delivery device may be the same, especially in view of their mechanical functions.

The functional members constitute the individual parts of which the drug delivery devices are assembled. While the individual parts may differ in their exact shape and appearance, for example to provide different dose increments among the individual drug delivery devices, they perform the same function and are located at the same positions within the dosing mechanisms of the individual drug delivery devices. Furthermore, they interact and engage with the same further functional members of the dosing mechanism among all drug delivery devices of the set. Functional members may be composed of several sub-parts that are rigidly connected to each other to form a single mechanical part. With one embodiment of the present disclosure, a dosing member may, for example, constitute a functional member that is composed of two sub-parts, namely a dose sleeve and a snap element.

According to another embodiment the first connection means and the second connection means both comprise a female part or a male part. Such connection means are usually comparatively easy to use such that the patient does not need to worry a lot about the handling upon attaching a dispensing unit to its respective drug delivery device.

It may also be preferable to design the first connection means and/or the second connection means as threads. Connection means of this kind are usually rather easy to produce such that manufacturing costs can be reduced. Furthermore, connection means which are designed as threads are also easy to handle for patients using the set of drug delivery devices according to the invention.

In this connection it is noted that the first connection means and the second connection means can either both be designed as threads or only one of them may be designed as a thread such that the respective other connection means are designed in a different way, such as for example a snap lock or bayonet lock or anything suitable.

In this connection it is further noted that the thread of the first connection means may have a pitch that is identical to the pitch of the thread of the second connection means. That is, in this embodiment the first and second connection means do not differ in the pitch of the thread from each other but rather in any other thread dimension. It has shown that if threads of different pitches were provided, it sometimes was not enough to ensure that a dispensing unit of a first kind could only be connected to the first drug delivery device and not to the second drug delivery device.

This can be due to the fact that when threads of a definite pitch are being produced, they always comprise inaccuracies which may lead to two threads, which are supposed to comprise different pitches but are nevertheless similar enough that, for example, dispensing units of a second kind could be attached to a drug delivery device of a first kind.

Having the same pitches on both the first and second thread furthermore allows a secure connection between the first and second dispensing unit and the corresponding drug delivery device since both threads require the same number of turns to attach the dispensing unit to the corresponding drug delivery device. Furthermore, an excessive length of one of the threads compared to the other one may be avoided.

According to a further embodiment ridges of the thread of the first connection means have different dimensions than ridges of the second connection means such that the two connection means can clearly be differentiated and it can be ensured that the first (second) dispensing unit cannot be attached to the second (first) drug delivery device since the different dimensions of the ridges of the thread do not allow such a connection. This means that generally each drug delivery device can be configured to only connect to a respective dispensing unit which comprises a unique drug or drug concentration or anything alike. Thus, each drug delivery device can comprise unique connection means such that medications of different concentrations, for example, cannot accidentally be mixed up.

According to another embodiment ridges of the thread of the first connection means have a width that differs from a width of ridges of the thread of the second connection means. That is, the ridges of the thread of the first connection means may have a width that differs among the first and the second connection means whereas, for example, the pitch and the other dimensions of the thread may be identical. Just by varying the width of the thread, it can be ensured that the first and the second connection means can be distinguished from one another.

In this connection it is noted that it may further be possible that the width of the ridges of the thread do not match with a width of their neighbouring valleys or troughs. Usually, the widths of the ridges of a thread and the width of the valleys of said thread are identical. According to this embodiment, on the other hand, the widths of the ridges may not match with the widths of the valleys, thereby defining another possibility to distinguish the first connection means from the second connection means when they comprise different valley widths.

The ridges of the thread of the first connection means may further have a height that differs from a height of the ridges of the second connection means such that the two connection means differ from each other in way that ensures that only the "right" dispensing unit can be attached to the "right" drug delivery device, i.e. the first dispensing unit to the first drug delivery device and the second dispensing unit to the second drug delivery device, respectively.

In this connection it is further noted that it may be possible that the two connection means only differ in the height of the ridges whereas all other dimensions can be identical among the two connection means. In a different embodiment additional dimensions, such as for example the width of the ridges, can additionally be different among the two in order to ensure a safer distinction compared to connection means which only differ in one single attribute or thread dimension.

It can also be preferred that the threads of the first and second connection means have the same minor diameter and/or the same major diameter. That is, the height of the ridges, as mentioned above, can be different between the two connection means while still keeping one dimension identical. For example, two connection means can comprise the same major diameter but a different minor diameter or vice versa. This obviously leads to a different height of the ridges while still keeping one dimension identical.

For example, the minor diameter, which can also be called core diameter, of the threads of the first and second connection means may be identical and the major diameter, which can also be called outer diameter, of the threads of the first and second connection means may differ. This allows efficient production of a female part of the threaded connection, for example by injection molding. A cast used for molding may then comprise a first element that defines the core diameter of the thread. The differing outer diameters may then be defined by a multitude of second elements that may be positioned around the first element and that define the valleys or troughs between the ridges of the female part. For each connection means, there may be provided a different second part that defines a trough having a width that differs from the widths of the troughs of the other connection means and/or that defines a trough having a height that differs from the heights of the troughs of the other connection means.

In one embodiment, the threads of the first and second connection means may differ in the widths and in the heights of their ridges. In this case, the differences in height may result from differences in the outer diameters with the core diameters being equal. Furthermore, all other thread dimensions may be equal. The width of the ridges of the male part of one of the threads may be a multiple of, for example two times or three times, the width of the ridges of the male part of other one of the threads. Furthermore, the male part of the one of the threads having the smaller width of its ridges may have a height of its ridges that is a multiple of, for example two times or three times, the height of the ridges of the other one of the threads.

For example, the male part of the thread of the connection means of the first drug delivery device may have ridges with a first width and a first height and the male part of the thread of the connection means of the second drug delivery device may have ridges with a second width and a second height. The first height may be two times the second height and the second width may be two times the first width. Additional to or instead of the second drug delivery device, the set of drug delivery devices may then comprise a third drug delivery device having connection means with a thread, the male part of which has ridges with a third width and a third height. The first height may then be three times the third height and the third width may be three times the first width. A pitch, core diameter and angle of the threads of the first drug delivery device and the threads of the second and/or third drug delivery device may then be equal.

Unless stated otherwise, the term "ridges" used in the present disclosure always refers to the ridges of the thread part of a given threaded connection, irrespective of whether the part being described actually comprises a male thread or a female thread.

According to an alternative embodiment both the minor and the major diameter can differ such that two completely different threads can be realized.

According to still another embodiment both the minor and the major diameter can be identical. With this embodiment, the threads of the first connection means and the second connection means differ from each other in another manner or thread dimension, such as, for example, in the width of the ridges, the pitch of the thread or anything alike.

According to an embodiment, the dosing mechanisms each comprise a dose definition mechanism that acts between the dose setting member and the housing and wherein the dose definition mechanisms are configured to define dose positions that are settable with the dose setting members, wherein the dose definition mechanisms define relative positions, for example relative rotational positions, of the dose setting member with respect to the housing that correspond to the settable dose positions. That is, by actuating, like for example turning, the dose setting member with respect to the housing, the dose definition mechanism defines precise positions of said dose setting member with respect to the housing which correspond to definite settable dose positions, i.e. to definite doses of drug to be ejected. This provides a rather simple way for the user to define precise doses of medication such that also a medically non-trained person is able to self-administer the right amount of drug.

Hence, the dose setting member may further be configured to be actuated by the user, i.e. the patient, to set a definite dose of drug which is supposed to be ejected out of the dispensing unit.

In this connection it is noted that the dose definition mechanisms of the first and second drug delivery device may further define the same number of settable doses, for example per revolution of the dose setting member. For some cases, i.e. for some drugs, it may be possible that the dose definition mechanism defines the same amount of settable doses.

Nevertheless, this may not necessarily mean that the exact same medication is dispensed out of both the first and the second drug delivery device. It may be coincidence that, for example, for two different drugs or one drug of two different concentrations, two drug delivery devices can be used which comprise dose definition mechanisms that are configured to define the same amount of settable doses when, for example, rotating the dose setting member, which may be a knob or a sleeve, once around its axis of rotation. The set of drug delivery devices according to the present disclosure nevertheless ensures that the two medications can be differentiated because of the distinguishable connection means of the drug delivery devices.

According to an alternative embodiment the dose definition mechanisms of the first and second drug delivery device may define different numbers of settable doses, for example per revolution of the dose setting member. That is, contrary to the above, it may also be possible that, for example, the first drug delivery device is configured to provide a number of settable doses which exceeds the number of settable doses of the second drug delivery device. This may be necessary for certain medications.

Thus, the precise number of settable doses may be chosen according to the application field, the patient's conditions or even according to the drug concentrations.

Furthermore, it can be preferred that the dose definition mechanisms of the first and/or second drug delivery device define an uneven number of settable doses, for example per revolution of the dose setting member. This can ensure that the dose setting member does always comprise a definite position relative to the housing since an uneven number of settable doses obviously cannot provide, for example, any kind of, e.g. rotational, symmetry

In another embodiment of the invention, the dosing mechanism further comprises a dose selector member which is rotationally fixed to the housing and axially movable with respect to the housing, wherein the dose definition mechanism is provided between the dose selector member and the dose setting member. That is, when actuating the dose definition mechanism with the dose setting member, the dose selector member is free to move axially with respect to the housing.

If the dose setting member is further configured to be free to rotate with respect to the housing, it may be necessary to ensure that the dose setting member is also free to rotate with respect to the dose selector member since the dose selector member is rotationally fixed to the housing. Thereby, the dose setting member does not only rotate with respect to the housing but also with respect to the dose selector member.

Also, the dose definition mechanisms may further comprise elastic elements which are in connection with functional features, for example teeth or dose stops, such that upon actuation of the dose setting member the elastic elements engage with the functional features to provide an audible and/or tactile feedback for a user. Such elastic members can provide a visible, tactile and/or audible feedback to the user for helping him or her during the dose setting and dose injection procedure.

In this connection it can be possible that the first drug delivery device and/or the second drug delivery device can comprise an even number of elastic elements, for example four elastic elements. The precise number may not necessarily have functional reasons but may, for example, be chosen such that the manufacturing costs and/or time of the drug delivery devices can be reduced. The first and/or second drug delivery device may also comprise a single elastic element.

The dose indication member may further comprise optical markers visualizing the dose set upon actuation of the dose setting member, wherein the optical markers of the first drug delivery device may be different from the optical markers of the second drug delivery device. Thus, said optical markers can help the user to clearly recognize the set dose. Furthermore, such optical markers can further also contribute to the group of distinguishing members, if said markers differ among the first and second drug delivery device, such that the at least two drug delivery devices of the set of drug delivery devices can be distinguished from one another.

The optical markers may be configured to display an amount or dose of an active pharmaceutical ingredient of the drug that is delivered by the drug delivery device when injecting the set dose. The amount of the active pharmaceutical ingredient that is delivered thereby depends on the concentration of the active pharmaceutical ingredient in the drug liquid or fluid contained in the dispensing units and the amount of liquid expelled by the drug delivery device when ejecting the set dose.

The first drug delivery device may be configured to be used with the first dispensing unit containing an active pharmaceutical ingredient in a first concentration and the second drug delivery device may be configured to be used with the second dispensing unit containing an active pharmaceutical ingredient in a second concentration that differs from the first concentration. Furthermore, the first and second drug delivery device may be configured to expel the same amount of liquid per settable dose increment. Due to the different concentrations of the active pharmaceutical ingredient, the same amount of liquid then corresponds to a different dose of the active pharmaceutical ingredient among the first and second drug delivery device.

In this case, the dose indication members may be mechanically identical. The dose indication members may however then carry different dose markings to account for the different doses of the active pharmaceutical ingredient corresponding to the individual set doses of ejected liquid. The dose markings may only differ by their numerical value while their position on the dose indication member may be the same with the first and second drug delivery device. Due to the differences in their appearance, the dose indication members then constitute distinguishing members of the dose setting mechanisms.

All other functional members of the dosing mechanisms of the first and second drug delivery device, except for the members carrying the first and second connection means, may then also be mechanically identical among the first and second drug delivery device. For example, all members of the dose definition mechanism of the dosing mechanism that defines the settable doses, may be mechanically identical.

According to another embodiment, the dose indication member of the first drug delivery device is mechanically identical to the dose indication member of the second drug delivery device. Nevertheless, it may be possible that two dose indication members which are mechanically identical comprise different optical markers to visualize the dose set depending on the drug (concentration) which is filled in the drug compartment of the drug delivery device.

According to an alternative embodiment the dose indication member of the first drug delivery device is mechanically different from the dose indication member of the second drug delivery device. That is, for some applications (e. g. for certain drugs) it can be necessary to provide different dose increments. Therefore, the dose indication members of the first and second drug delivery devices can also be mechanically different such that an adaption of the drug delivery device to its precise application field is provided.

Additionally, apart from being mechanically different, the two dose indication members of the first and second drug delivery device may comprise different optical markers. Alternatively, said optical markers of the mechanically different dose indication members can also be identical in their numbering and/or their position on the dose indication member.

In one embodiment, the optical markers of the dose indication members of the first and second drug delivery device are identical in their numbering and in their positions on the dose indication member. In such an embodiment, the first drug delivery device may be configured to be used with a first drug contained within the first dispensing unit and the second drug delivery device may be configured to be used with a second drug contained within the second dispensing unit. A first active pharmaceutical ingredient of the first drug and a second active pharmaceutical ingredient of the second drug as well as the amount of liquid expelled per dose increment settable with the first and second drug delivery device may match in a way that both the first and second drug delivery device expel the same dose of active pharmaceutical ingredient per settable dose increment. The first and second drug delivery device may then have distinguishing elements that differ in their outer appearance, such as their colour or labelling. This allows a user to easily distinguish between the first drug delivery device containing the first drug and the second drug delivery device containing the second drug. That distinguishing elements may, for example, be outer housings and/or caps of the first and second drug delivery devices.

In another embodiment, the optical markers of the dose indication members of the first and second drug delivery device may be identical in their numbering but differ in their positions on the dose indication member. In a further embodiment, the optical markers of the dose indication members of the first and second drug delivery device may be different in their numbering but identical in their positions on the dose indication member. In yet another embodiment, the optical markers of the dose indication members of the first and second drug delivery device may differ in both their numbering and in their positions on the dose indication member. With these embodiments, the first and second drug delivery devices may be configured to be used with the dispensing units containing drugs that have active pharmaceutical ingredients in different concentrations and/or the first and second drug delivery device may be configured to expel different amounts of liquid per settable doses.

With a further embodiment of the invention, the dose setting mechanisms comprises a nut and a clutch mechanism, wherein the nut is configured to axially advance the piston rod during dose delivery, wherein the clutch mechanism is configured to rotationally couple the nut to a dosing member in distinct relative rotational positions and to rotationally decouple the nut from the housing during dose setting, and wherein the clutch mechanism is configured to rotationally decouple the nut from the dosing member and to rotationally couple the nut to the housing in distinct relative rotational positions during dose delivery, wherein, for example, the dosing member is the dose indication member.

That is, during dose setting the clutch mechanism rotationally couples the nut to the dosing member, wherein the dosing member may contribute to defining the size, e. g. the quantity, of the settable dose. Therefore, the nut rotates with the dosing member during the dose setting process.

During dose delivery, on the other hand, the clutch mechanism rotationally decouples the nut from the dosing member, while at the same time rotationally couples the nut to the housing. This means that during dose delivery the nut does not rotate but is configured to axially advance the piston rod.

It is further noted that in some embodiments the dosing member and the dose indication member may be one and the same member.

In this connection it is noted that the clutch mechanism may be provided between the dose setting member and the dosing member. Hence, during dose setting an actuation of the dose setting member may lead to a rotation of the dosing member and also to a rotation of the nut since the dosing member and the nut are rotationally coupled through the clutch mechanism. During dose delivery, on the other hand, an actuation of the dose setting member may lead to the nut advancing the piston rod in an axial direction.

This could for example be realized by providing a dose setting member which can be rotated for the dose setting process and pressed, i.e. axially advanced, for the dose delivery process. Such a dose setting member forms an actuation member of the dosing mechanism.

The relative rotational positions in which the nut and the dose setting sleeve are coupled to each other during dose setting may differ from each other among the first and second drug delivery device. This may ensure that with the different drug delivery devices different doses such as amount of liquid (as measured in ml), may be set. The precise design of the dose setting sleeve may be chosen according to the application of the drug delivery device, e.g. according to the drug which is supposed to be filled in the drug compartment.

Furthermore, the relative rotational positions in which the nut and the housing are coupled to each other during dose delivery may differ among the first and second drug delivery device. This may arise due to the fact that the dosing mechanisms of the two drug delivery devices are mechanically different such that, for example, a thread pitch of some of the connections between the different components of the dosing mechanisms differ from one another.

Alternatively, the relative rotational positions in which the nut and the housing are coupled to each other during dose delivery of the first and second drug delivery device are identical.

According to a different embodiment the clutch mechanisms each comprise a clutch member that is connected to the respective dose setting member, wherein the dose setting member comprises a first set of clutch elements and the clutch member comprises a second set of clutch elements, and wherein a connection between the clutch member and the dose setting member is configured to restrict a relative rotational orientation of the clutch elements of the dose setting member with respect to the clutch elements of the clutch member.

In this connection it is noted that in some embodiments the drug delivery devices may comprise an even number of clutch elements whereas in other cases the drug delivery devices may comprise an odd number of clutch elements on the clutch member.

In this connection it is further noted that the connection between the clutch member and the dose setting member of the first delivery device may be mechanically different from the connection between the clutch member and the dose setting member of the second delivery device. This may be realized, for example, by providing connections which have different radial cross-section in planes perpendicular to longitudinal axes of the devices.

Alternatively, the connection between the clutch member and the dose setting member of the first delivery device is mechanically identical to the connection between the clutch member and the dose setting member of the second delivery device. This may be the case when the only differences between the drug delivery devices lie in their appearances, by e. g. comprising different optical markers on their dose indication member or by comprising differently coloured or printed housings or anything the like.

The connection of the first and/or second drug delivery device may further be configured to restrict the relative rotational orientation to a single relative rotational orientation. This can ensure that the dose setting member can only be attached to the clutch member in one single orientation. For cases, where the clutch member comprises an odd number of clutch elements it can be necessary that the dose setting member can only be provided in one single rotational orientation with respect to the clutch member.

According to an alternative embodiment the connection of the first and/or second drug delivery device is configured to restrict the relative rotational orientation to two relative rotational orientations that differ by 180°. This may be the case when the clutch member comprise an even number of clutch elements. By providing an even number of clutch elements it may not make any difference if the connection is symmetrical with respect to an axis.

It may further be preferable for the dosing mechanisms to further comprise an inner body to support the dosing mechanism and to transfer the movement of the dose setting member to the piston rod, wherein the inner body is, for example, in threaded connection with the dosing member and with at least one member from said first subset of mutual members. Hence, the inner body may contribute to translate the rotation of the clutch mechanism to an axial movement of the piston rod, preferably without rotation of the piston rod during dose delivery.

Therefore, a threaded connection between the inner body and the dosing member and between the inner body and at least one member of said first subset of mutual members may be provided which can ensure that a rotation of the dosing member is translated, via the inner body, to an axial movement of said dosing member. This can ultimately lead to an axial movement of said mutual member, i.e. for example a driver, since said mutual member is also in a threaded connection with said inner body.

According to another embodiment the dosing mechanisms of the first and second drug delivery device have a dialling resolution that is the same for both devices such that they are configured to expel the same amount of fluid or liquid per dose increment settable by the dose setting member, for example 0.010 ml or 0.015 ml. That is, the first and second drug delivery device may comprise identical mechanical functionalities such that they are configured to expel the same amount of fluid per dose increment, i.e. for example per step, which may be settable by the dose setting member. With such a dosing mechanism, all functional members, despite the members comprising the connection means, may be mechanically identical among the first and second drug delivery device.

Nevertheless, if, for example, a drug of two different concentrations of active pharmaceutical ingredient is provided, this may lead to two different doses of active pharmaceutical ingredient settable per dose increment. Therefore, the drug delivery devices according to the invention are provided with their respective distinguishing member, which may for example be distinguishable by their appearance, as well as with their distinguishable connection means such that said two drug delivery devices provided with the drugs of different concentrations can be clearly distinguished from one another.

Alternatively, the dosing mechanism of the first and second drug delivery device may have a dialling resolution that is different for both devices such that they are configured to expel a different amount of fluid or liquid per dose increment settable by the dose setting members, for example 0.010 ml and 0.015 ml. In contrast to the previous embodiment, the first and second drug delivery devices can also have dialling resolutions that differ from one another such that a different amount of fluid is expelled. This may be necessary when different medications are used. The precise amount of fluid which is expelled per dose increment may be chosen according to the application (i.e. according to the precise medication etc.).

According to another embodiment the piston rods of the first and second drug delivery device comprise a gearing ratio or mechanical advantage between the movement of the piston rod with respect to the axial movement of an actuation member, which may be for example the dose setting member, that is identical among the first and second drug delivery device. This means that a tactile feedback for the user may always be the same independently of the dosing mechanisms being mechanically identical or different since the axial movement of the actuation member is always translated to the same amount of movement of the piston rod.

According to an embodiment, the piston rod is rotationally fixed with respect to the housing at least during dose delivery. Other embodiments of drug delivery devices of the sets according to the present disclosure may have a piston rod that is rotationally movable with respect to the housing during dose delivery and/or during dose setting.

Furthermore, all functional members of the dose definition mechanism of the first drug delivery device may be mechanically identical to the functional members of the dose definition mechanism of the second drug delivery device. This may be the case when the first and second drug delivery device only differ in their appearance such as their looks (e. g. colouring) and/or their optical markers on their dose indication members but provide the same dose increments and expel the same amount of fluid per dose increment.

The invention further relates to a set of dispensing units comprising a first dispensing unit and a second dispensing unit. Each dispensing unit is configured to have a drug compartment containing a drug to be delivered by one of the drug delivery devices of the set according the invention, wherein the first dispensing unit comprises first connection means that are different from second connection means of the second dispensing unit. The first connection means are configured to only connect to the first connection means of the first drug delivery device and not to the second connection means of the second drug delivery device. The second connection means are configured to only connect to the second connection means of the second drug delivery device and not to the first connection means of the first drug delivery device.

The first connection means and the second connection means can generally be designed in different ways such as threads, bayonet locks, snap locks or the like. It can further be either possible that the first dispensing unit and the second dispensing unit comprise the same type of connection means or a different type of connection means. It seems to be quite obvious that if the devices comprise connection means of different types a distinction between the two is easy.

Nevertheless, even if the two dispensing units comprise the same type of connection means they can be designed such that the first dispensing unit can only be connected to the first drug delivery device and the second dispensing unit can only be connected to the second drug delivery device.

The first connection means and the second connection means may both comprise a female part. Alternatively, the may both comprise a male part. As already mentioned above, such connection means are usually comparatively easy to use such that the patient does not need to worry a lot about the handling upon attaching a dispensing unit to its respective drug delivery device.

According to an embodiment the first and second connection means are designed as threads, in particular wherein the thread of the first connection means has a pitch that is identical to the pitch of the thread of the second connection means. Threads are usually easy to handle such that also patients which may already suffer from handicaps are able to use such connection means rather easily.

In this connection it is noted that the first connection means and the second connection means can either both be designed as threads or only one of them may be designed as a thread such that the respective other connection means are designed in a different way, such as for example a snap lock or bayonet lock or anything suitable.

According to another embodiment ridges of the thread of the first connection means have different dimensions than ridges of the second connection means such that the two connection means can clearly be differentiated and it can be ensured that the first (second) dispensing unit cannot be attached to the second (first) drug delivery device since the different dimensions of the ridges of the thread do not allow such a connection.

This means that generally each drug delivery device can be configured to only connect to a respective dispensing unit which comprises a unique drug or drug concentration or anything alike. Thus, each drug delivery device can comprise unique connection means such that medications of different concentrations, for example, cannot accidentally be mixed up.

Furthermore, ridges of the thread of the first connection means may have a width that differs from a width of ridges of the thread of the second connection means. That is, the ridges of the thread of the first connection means may have a width that differs among the first and the second connection means, whereas, for example, the pitch and the other dimensions of the thread may be identical. Just by varying the width of the thread, it can be ensured that the first and the second connection means can be distinguished from one another.

In this connection it is noted that it may further be possible that the width of the ridges of the thread do not match with a width of their neighbouring valleys. Usually, with conventional threads, the widths of the ridges of a thread and the width of the valleys of said thread are identical.

According to this embodiment, on the other hand, the widths of the ridges may not match with the widths of the valleys, thereby defining another possibility to distinguish the first connection means from the second connection means when they comprise different valley widths.

Also, ridges of the thread of the first connection means may have a height that differs from a height of the ridges of the second connection means such that the two connection means differ from each other in a way that ensures that only the "right" dispensing unit can be attached to the "right" drug delivery device, i.e. the first dispensing unit to the first drug delivery device and the second dispensing unit to the second drug delivery device, respectively.

In this connection it is further noted that it may be possible that the two connection means only differ in the height of the ridges whereas all other dimensions can be identical among the two connection means. In a different embodiment other dimensions, such as for example the width of the ridges, can additionally be different among the two in order to ensure a safer distinction compared to connection means which only differ in one single attribute or thread dimension.

It can also be preferred that the threads of the first and second connection means have the same minor diameter and/or the same major diameter. That is, the height of the ridges, as mentioned above, can be different between the two connection means while still keeping one dimension identical. For example, the two connection means can comprise the same major diameter but a different minor diameter or vice versa. This obviously leads to a different height of the ridges while still keeping one dimension identical.

According to an alternative embodiment both the minor and the major diameter can differ such that two completely different threads can be realized.

According to still another embodiment both the minor and the major diameter can be identical such that it needs to be ensured that the threads of the first connection means and the second connection means differ from each other in another manner, such as for example the width of the ridges, the pitch of the thread or anything alike.

According to another embodiment of the invention, the dispensing units comprise cartridge holders for receiving a cartridge which comprises the drug compartment filled with the drug.

In this connection it should be noted that a drug stored in the cartridge may be selected from the group of members consisting of diabetes medication, such as insulin, growth hormones, fertility hormones, osteoporosis medication, blood thinners, such as heparin.

In this connection it is noted that the first connection means and the second connection means can be provided at the cartridge holder. This means that only the cartridge holder is directly engaged with the respective drug delivery device and not the cartridge itself. This can ensure that standard cartridges, which are already common state of the art, can be used with the new set of dispensing units according to the invention since the different connection means are provided at the cartridge holder and not the cartridge itself.

The cartridge holder may further comprise a connector that is configured to non-releasably, for example permanently, connect the cartridge to the cartridge holder during use of the dispensing unit. This can be regarded as a further security features which can ensure that only the right cartridge containing the right drug can be attached to the respective drug delivery device. By providing a non-releasable attachment of the cartridge to the cartridge holder, it can be ensured that different cartridges cannot be mixed up accidentally.

The dispensing unit may be provided, for example to a user of the device, with the cartridge already inserted into the cartridge holder. This assures that the drug contained within the cartridge is already permanently assigned to a connection means that only allows attachment to a drug delivery device that is configured to be used with the respective drug. The present disclosure therefore also relates to a dispensing unit having a cartridge according to the present disclosure inserted into a cartridge container according the present disclosure.

It this connection it is further noted that the connector may be designed as a snap fit connection, for example as a snap hook. Such a hook can ensure that the cartridge is safely secured inside the cartridge holder and can further not be moved in an axial direction when a needle is mounted onto the dispensing unit.

According to another embodiment, the connector is provided at a needle end of the dispensing unit. Such a positioning of the connector may be advantageous since the space conditions at the other end, i.e. the side which is attached to the drug delivery device, may be limited.

It can also be preferred that the connector is configured to engage with an annular rim of the cartridge, for example to abut against a distal surface of the annular rim that faces away from a needle end of the cartridge holder.

The cartridge holder can further comprise a push element, for example a flexible member, that is configured to push the cartridge in the proximal direction and/or into the cartridge holder after insertion. Since the dispensing unit is connected to the drug delivery device via connection means that may be provided at the cartridge holder, the drug delivery device cannot directly push the cartridge in a proximal direction, i.e. away from the drug delivery device, in order to safely secure the cartridge during attachment of a cannula to the cartridge holder. Said additional push element can therefore be provided to push the cartridge further into the cartridge holder and to prevent distal movement of the cartridge when piercing a sealing member of the cartridge by the cannula during attachment of the cannula to the cartridge holder.

In this connection it is further noted that the push element is configured to push the cartridge against a stop that is located at a needle end of the cartridge holder. Said stop feature may, for example, be configured as an end surface of a cavity of the cartridge holder, in which cavity the cartridge is received after insertion into the cartridge holder.

The push element also prevents a plunger of the cartridge from being pressed distally against the piston rod when the user attaches a cannula to the cartridge container. Such distal movement could otherwise compress the plunger before the cannula penetrates a sealing means of the cartridge. Such a compressed plunger would then relax once a fluid path out of the cartridge is established by the cannula piercing the sealing means and would thus expel some drug through the needle. In general, the push element avoids that the plunger is compressed when the cannula is about to penetrate the sealing means.

According to a further embodiment, the first dispensing unit comprises a fluid containing a drug or an active pharmaceutical ingredient, for example insulin or HGH, in a first concentration and the second dispensing unit comprises a fluid containing the drug or the active pharmaceutical ingredient in a second concentration that is different from the first concentration. That is, the two dispensing units according to the invention may generally comprise the same drug or active pharmaceutical ingredient, such as, for example, insulin, but in different concentrations.

Since, according to the invention, different connection means are provided at the different dispensing units, said units can clearly be separated from each other since they can only be attached to their respective drug delivery device which is suitable for said precise fluid concentration.

The invention further relates to a kit comprising at least the first drug delivery device of the set according to the invention and the first dispensing unit of the set according to the invention and the second drug delivery device of the set according to the invention and the second dispensing unit of the set according to the invention.

According to an embodiment of the invention the dialling resolution of first drug delivery device differs from the dialling resolution of the second drug delivery device, and the first dispensing unit comprises a fluid containing a drug or an active pharmaceutical ingredient, for example insulin or HGH, in a first concentration and the second dispensing unit comprises a fluid containing the drug or the active pharmaceutical ingredient in a second concentration that is different from the first concentration.

Since the two drug delivery devices as well as the two respective dispensing units according to the invention comprise different connection means it can be ensured that only the right combination of dispensing unit and drug delivery device can be connected to one another thereby ensuring that a patient can only eject the right dose out of said drug delivery device.

According to one embodiment of the invention, said set of mutual members of the first and second drug delivery device may comprise one or more of the following: cap clip, driver, clutch member, inner body, dose setting member, nut and piston rod.

The corresponding sets of distinguishing members may then comprise one or more of the following: housing, cap body, cartridge holder, dose indication member and dose selector member. In this connection it is noted that said distinguishing members may only be distinguished by their appearance but not by their functionality.

According to another embodiment of the invention the set of mutual members of the first and second drug delivery device may comprise one or more of the following: cap body, driver, inner body, nut and piston rod.

The corresponding sets of distinguishing members may then comprise one or more of the following: housing, cap body, cartridge holder, dose indication member, dose selector member, clutch member and dose setting member. In this connection it is further noted that the distinguishing members may not only be distinguished by their appearance but also by their mechanical functionalities.

According to a third embodiment of the invention the set of mutual members of the first and second drug delivery device may comprise one or more of the following: cap clip, driver, inner body, nut and piston rod.

The corresponding sets of distinguishing members may then comprise one or more of the following: housing, cap body, cartridge holder, dose indication member, dose selector sleeve, clutch member, dose setting member. In this connection it is noted that the distinguishing members may differ in their appearance as well as in their mechanical functionalities.

Exemplary embodiments and functions of the present disclosure are described herein in conjunction with the following drawings, showing schematically:
- Fig. 1: a perspective view of a drug delivery device according to the present invention with an attached cap;
- Fig. 2: a perspective view of the drug delivery device with the cap removed and an attached dispensing unit;
- Fig. 3: a perspective view of the drug delivery device, the cap and the dispensing unit;
- Fig. 4: a side view of the dispensing unit comprising a cartridge holder and a cartridge and a pen needle attachable to the dispensing unit;
- Fig. 5: a longitudinal cut of the drug delivery device, the dispensing unit and the cap through a first cutting plane with the drug delivery device being in a dose setting state;
- Fig. 6: a longitudinal cut of the first drug delivery device, the first dispensing unit and the cap through a second cutting plane perpendicular to the first cutting plane with the drug delivery device being in the dose setting state;
- Fig. 7: an exploded partial view of a dosing mechanism of the drug delivery device;
- Fig. 8: a longitudinal cut of the dosing mechanism of the drug delivery device through the first cutting plane prior to setting a dose;
- Fig. 9: a longitudinal cut of the dosing mechanism through the first cutting plane after setting a dose, the dosing mechanism still being in a dose setting state;
- Fig. 10: a longitudinal cut of the dosing mechanism through the first cutting plane after setting the dose, the dosing mechanism being in a dose delivery state;
- Fig. 11: a longitudinal cut of the dosing mechanism through the first cutting plane after delivering the dose, the dosing mechanism being in the dose setting state;
- Fig. 12: a clutch mechanism of the dosing mechanism in a dose setting state;
- Fig. 13: the clutch mechanism in a dose delivery state;
- Fig. 14: a radial cut through a dose definition mechanism of the drug delivery device;
- Fig. 15: a perspective view of a proximal side of a dose setting member of the drug delivery device;
- Fig. 16: a perspective view of a distal side of a clutch member of the drug delivery device;
- Fig. 17: a perspective view of a proximal side of the clutch member of the drug delivery device;
- Fig. 18: a longitudinal cut through a dosing member and a dose selector member of the drug delivery device with a first friction reduction mechanism;
- Fig. 19: a perspective view of a connection between a nut and a driver of the drug delivery device with a second friction reduction mechanism;
- Fig. 20: a perspective view of a dosing member of the drug delivery device;
- Fig. 21: a longitudinal cut through an inner housing of the drug delivery device;
- Fig. 22: a perspective view of the inner housing with the dosing member in a zero-dose position;
- Fig. 23: a perspective view of the inner housing with the dosing member in a maximum dose position;
- Fig. 24: a longitudinal cut through an outer housing of the drug delivery device;
- Fig. 25: a longitudinal cut of the inner housing mounted within the outer housing of the drug delivery device;
- Fig. 26: a radial cut through the outer and inner housing of the drug delivery device;
- Fig. 27: an exploded partial view of a resetting mechanism of the drug delivery device;
- Fig. 28: a longitudinal cut through the resetting mechanism of the drug delivery device with a resetting element in a proximal position;
- Fig. 29: a distal perspective view of the resetting element of the resetting mechanism;
- Fig. 30: a proximal perspective view of the resetting element;
- Fig. 31: a proximal perspective view of a coupling part of the resetting mechanism;
- Fig. 32: a perspective view of the coupling part and the inner housing;
- Fig. 33: a longitudinal cut through the resetting mechanism with the dispensing unit attached to the drug delivery device and the resetting element located in a distal position;
- Fig. 34: a longitudinal cut through a proximal end of a cartridge holder attachable to the drug delivery device;
- Fig. 35: a perspective distal view of a radial cut through a proximal part of the cartridge holder;
- Fig. 36: a longitudinal cut through a first dispensing unit attachable to a first drug delivery device, a longitudinal cut through a second dispensing unit attachable to a second drug delivery device, and a longitudinal cut through a third dispensing unit attachable to a third drug delivery device;
- Fig. 37: a longitudinal cut through a first connection means of the first drug delivery device and a perspective view of the first connection means, a longitudinal cut through a second connection means of the second drug delivery device and a perspective view of the second connection means, and a longitudinal cut through a third connection means of the third drug delivery device and a perspective view of the third connection means;
- Fig. 38: a perspective view of a further drug delivery device;
- Fig. 39: the further drug delivery device with a removed cap;
- Fig. 40: an exploded view of the further drug delivery device;
- Fig. 41: a clutch mechanism of the further drug delivery device;
- Fig. 42: a dose setting member of the further drug delivery device;

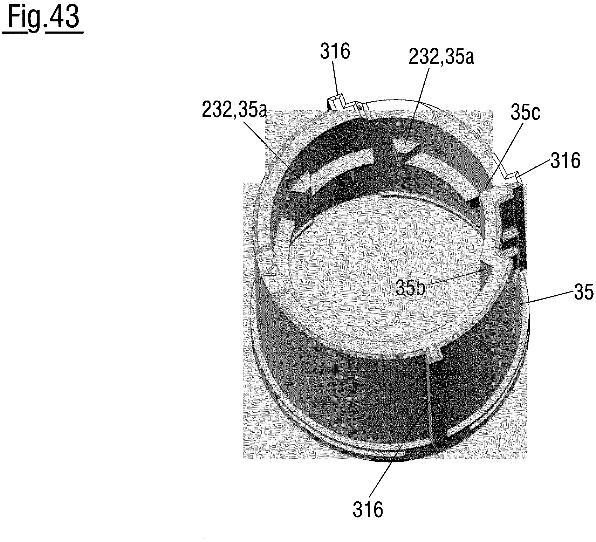

- Fig. 43: a dose selector member of the further drug delivery device.

In the present application, the term "distal part/end" refers to the part/end of the device, or the parts/ends of the components or members thereof, which in accordance with the use of the device, is located the furthest away from a delivery/injection site of a patient. Correspondingly, the term "proximal part/end" refers to the part/end of the device, or the parts/ends of the members thereof, which in accordance with the use of the device is located closest to the delivery/injection site of the patient.

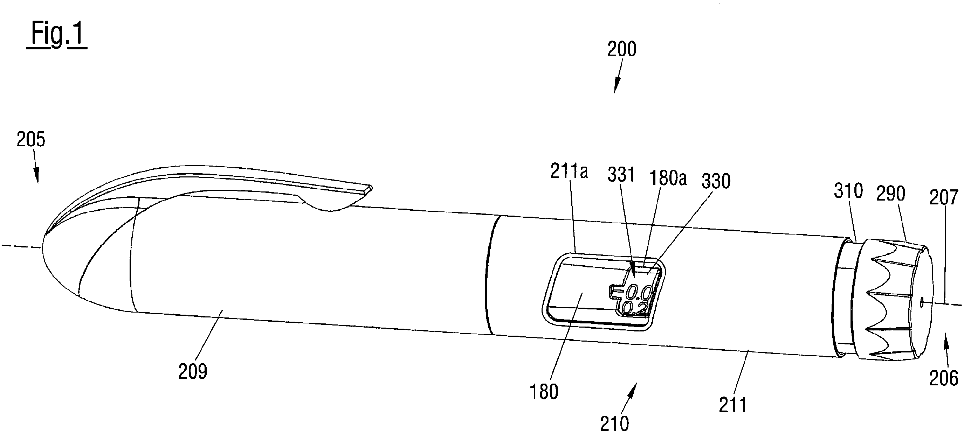

The present disclosure of keyed connection means is applicable with a number of medicament delivery devices, for example, injection devices. One possible injection device is the pen-type design illustrated in Fig. 1.

Fig. 1 shows a drug delivery device 200 that comprises connection means for attaching a dispensing unit according to the present disclosure. The drug delivery device has a generally tubular housing 210, which is elongated along a longitudinal axis 207. A generally tubular cap 209 is attached to a proximal end 205 of the housing 210. At a distal end 206 of the housing 210, which distal end 206 is located opposite to the proximal end 205 along the longitudinal axis 207, the drug delivery device 200 comprises a dose setting member 290.

The dose setting member 290 is rotatable around the longitudinal axis 207 and is configured to be gripped and rotated by a user of the device 210 to set a dose to be delivered by the device 210. In the embodiment shown in Fig. 1, the dose setting member 290 is configured as a knob that terminates the drug delivery device 200 at its distal end 206. With other embodiments, the dose setting member 290 may also be, for example, configured as a rotatable sleeve or ring that surrounds the longitudinal axis 207.

The dose setting member 290 is connected to the housing 210 via a dose selector member 310 that is rotationally locked and axially movable relative to the housing 210 both during dose setting and during dose delivery. When increasing a set dose by turning the dose setting member 290, the dose selector member 310 moves distally out of the housing 210, thereby also moving the dose setting member 290 in the distal direction.

The housing 210 comprises an outer housing 211, which, in the present embodiment, is made from metal, and an inner housing 180. The inner housing 180 is located within the outer housing 211. In the present embodiment, it is made from a plastic material. The outer housing 211 comprises a window 211 a through which a part of the inner housing 180 and a window 180a within the inner housing 118 is visible to a user of the device 200. Through the windows 211a and 180a, a dose indication member 330, which is located inside the generally tubular inner housing 180, is visible to the user.

The dose indication member 330 is also configured as a generally tubular member and carries on its outer cylindrical surface a dose scale comprising several optical markers 331 that correspond to the respective set dose. When setting a dose, the dose indication member 330 rotates within the inner housing 180, which changes the location of the scale and thus also the optical markers 331 visible through the windows 211a and 180a.

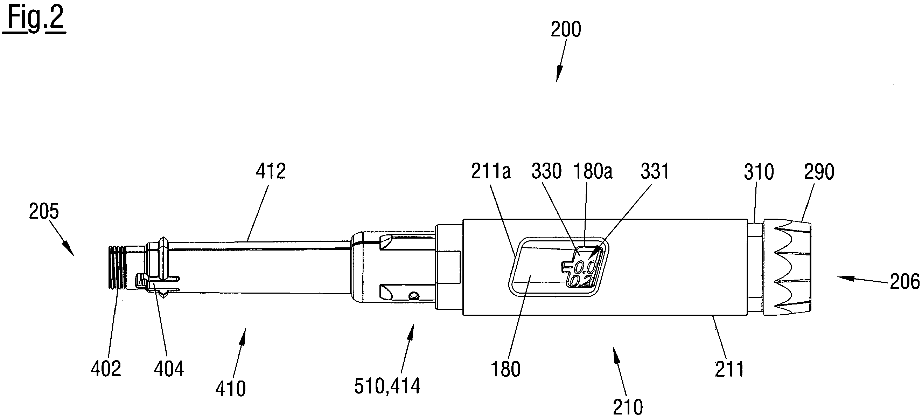

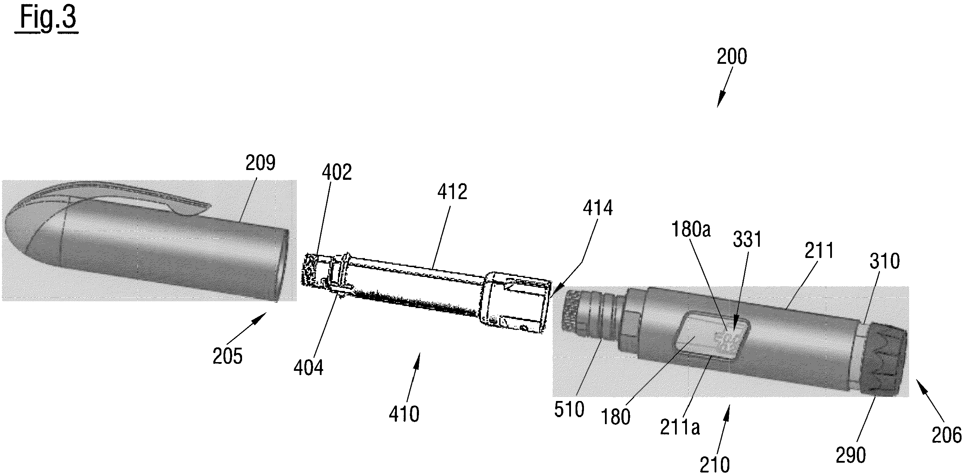

**Fig. 2** shows the drug delivery device 200 with the cap 209 removed. A dispensing unit 410 that comprises the drug to be delivered by the device 200 is removably attached to the proximal end 205 of the housing 210. **Fig. 3** shows the cap 209 and the dispensing unit 410 removed from the drug delivery device 200. With the cap 209 and the dispensing unit 410 attached to the housing 210 of the device 200, the dispensing unit 410 is fully received within the cap 209.

The dispensing unit 410 comprises a cartridge holder 412, which, in the current embodiment, is made from a plastic material. The cartridge holder 412 attaches to the outer housing 211 of the drug delivery device 200 via a connection, which comprises first connection means 510 located at the proximal end of the housing 210 and corresponding first connection means 414 located at the distal end of the dispensing unit 410. The first connection means 510 of the housing 210 are formed as integral part of the outer housing 211 and the first connection means 414 of the dispensing unit 410 are formed as integral part of the cartridge holder 412.

At its proximal end, the cartridge holder 412 of the dispensing unit 410 comprises a needle connector 402 that is configured to receive a hollow needle or cannula through which the drug is delivered by the drug delivery device 200. In the present embodiment, the needle connector 402 is configured as a threaded connector. With other embodiments, the needle connector 402 may also be configured as, for example, a snap-fit, bayonet or Luer-Lok connection.

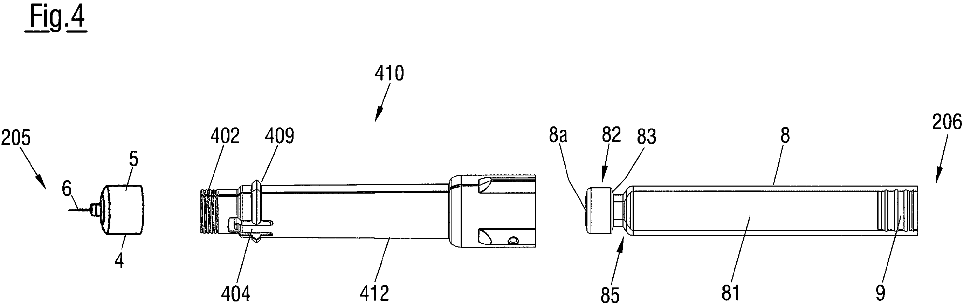

**Fig. 4** shows the cartridge holder 412 of the dispensing unit 410 and a cartridge 8 that may be inserted into the cartridge holder 412, as well as a pen needle 4 attachable to the needle connector 402.

The cartridge 8 has a generally cylindrical body, which, in the present embodiment, is made from glass, and which surrounds a drug compartment 81 that contains a liquid drug to be delivered by the drug delivery device 200. The drug compartment 81 is sealed at its distal end by an elastic plunger 9, which is movable along the longitudinal axis within the body of the cartridge 8. At its proximal end, the cartridge 8 comprises an annular rim 82, which is separated from the body by an annular detent 85 located distally from the annular rim 82. At a proximal front surface of the cartridge 8, which is orientated perpendicular to the longitudinal axis 207, the cartridge 8 comprises a sealing means or septum 8a, which seals the drug compartment 81 in the proximal direction.

When being fully inserted into the cartridge holder 412, the sealing means 8a is located at the proximal end of the cartridge holder 412 and accessible through an opening of the cartridge holder 412. The cartridge 8 is non-releasably held in its inserted position by a snap hook 404. The snap hook 404 is configured as a flexible member. In the present embodiment, the snap hook 404 is formed by a cut-out portion of the cartridge holder 412. Upon insertion of the cartridge 8 into the cartridge holder 412, the snap hook 404 snaps over the annular rim 82 of the cartridge 8. A radially inwardly protruding finger of the snap hook 404 is then located within the annular detent 85 of the cartridge 8 and prevents distal movement of the cartridge 8 by abutting against a distal surface 83 of the annular rim 82.

This non-releasable connection between the cartridge 8 and the cartridge holder 412 prevents a removal of the cartridge 8 from the cartridge holder 412 during intended use of the dispensing unit 410. For example, it prevents removal of the cartridge container 8 unless the snap hook 404 is intentionally and forcefully brought out of engagement with the annular rim 82. The snap hook 404 is thereby configured in a way that such disengagement is only possible using tools or excessive forces that are higher than the forces acting on the snap hook 404 during normal and/or intended use of the dispensing unit 410, for example during mounting of the dispensing unit 410 to the housing 210, during attachment of the pen needle 4 to the cartridge container 412 or during handling of the dispensing unit 410 with the cartridge 8 inserted into the cartridge holder 412. This handling may also comprise shock forces that may occur during transport and/or unintentional dropping of the dispensing unit and that do not exert forces that would destroy the dispensing unit 410 and/or the cartridge holder 412 and/or the cartridge 8. The non-releasable connection between the cartridge 8 and the cartridge holder 412 allows to provide and sell the dispensing unit 410 with an inserted cartridge 8 as a single, premounted unit.

The pen needle 4 comprises a hub 5 that carries a double-ended cannula 6. The cannula 6 is longitudinally received within the hub 5. The hub 5 comprises at its distal end a hub connector that matches the needle connector 402 of the cartridge holder 412. In the present embodiment, the hub connector is configured as an inner thread matching the outer thread of the needle connector 402. The cannula 6 protrudes from the proximal end of the hub 5. It has sharp ends at both its proximal and distal ends. With its distal end, the cannula 6 penetrates the sealing means 8a of the cartridge 8 and thus establishes a fluid connection between the drug compartment 81 and the proximal end of the cannula 6. The proximal end of the cannula 6 is configured to be inserted into a delivery site, such as a skin of the user of the device 200, thereby permitting injection of the drug into the delivery site.

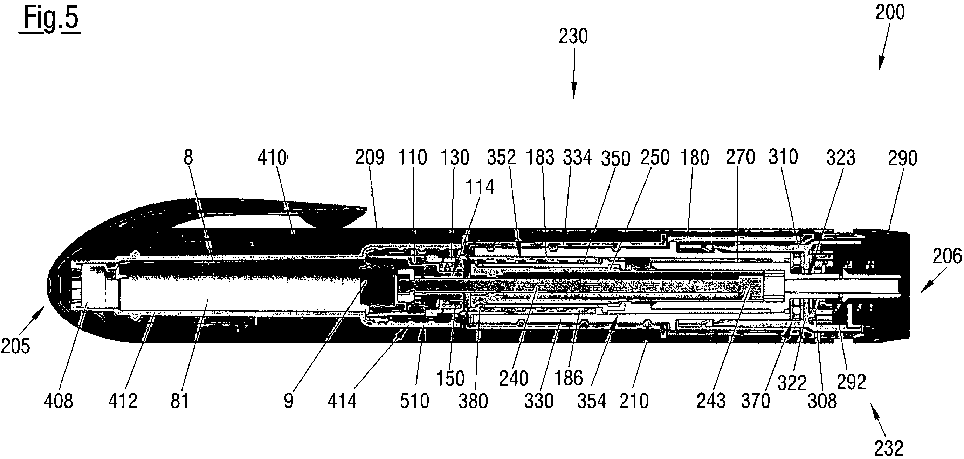

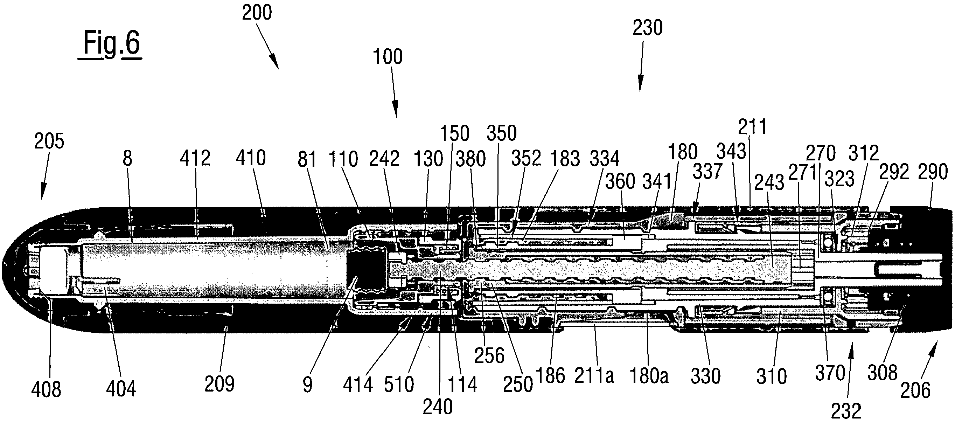

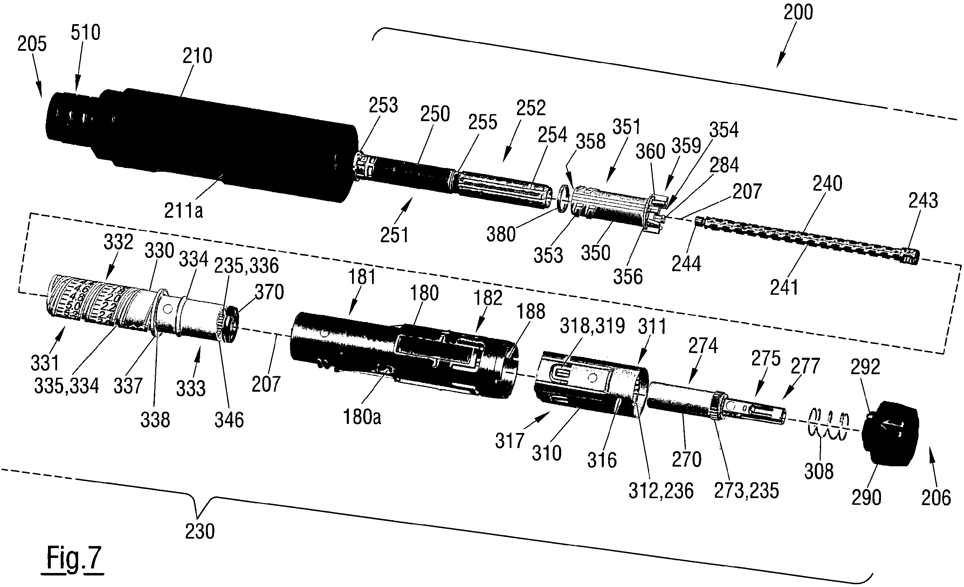

**Fig. 5** and **Fig. 6** show longitudinal cuts through the drug delivery device 200 along two different cutting planes that are orientated perpendicular to each other. **Fig. 7** shows a partial exploded view of the components of the drug delivery device 200 that are visible in Fig. 5 and Fig. 6. The drug delivery device 200 comprises a dosing mechanism 230 that is configured to set a dose of drug to be delivered by the drug delivery device 200 and to expel the set dose by moving the plunger 9 in the proximal direction.

The dosing mechanism 230 comprises a piston rod assembly with a piston rod 240, which is elongated along the longitudinal axis 207, and a plunger disc 242 mounted to the proximal end of the piston rod 240. The piston rod assembly is configured to directly contact the plunger 9 by the piston disc 242 and to advance the plunger 9 within the cartridge 8 upon movement of the piston rod assembly in the proximal direction. The piston rod 240 has a non-circular cross-section and an outer thread 241 that essentially covers its entire length. At its proximal end, the piston rod 240 comprises a disc connector 244 for receiving the piston disc 242. At its distal end, the piston rod 240 comprises a stop feature 243, which terminates the outer thread 241 and is exemplarily configured as a thickened portion of the piston rod 240 having a larger radial extent than the minor diameter of the thread 241.

The piston rod 240 is located within the housing 210 and protrudes from the proximal end of the housing 210 such that the piston disc 242 may be completely moved out of the housing 210. Thereby, the piston disc 242 is permanently located outside the inner housing 211.

The piston rod 240 is rotationally locked with respect to the housing 210 during both dose setting and dose delivery. In the present embodiment, the piston rod 240 is connected to the housing 210 via a resetting element 110 of a resetting mechanism 100 of the drug delivery device 200. The resetting element 110 is rotationally fixed with respect to the housing 210 during both dose delivery and dose setting. It comprises a longitudinal opening 114 that receives the piston rod such that the piston disc 242 is located at a proximal side of the opening 114 and the stop feature 243 is located at a distal side of the opening 114. The opening 114 is configured as a through hole with a non-circular cross section that matches the non-circular cross-section of the piston rod 240 thereby allowing axial movement but preventing rotational movement of the piston rod 240.

The piston rod 240 is surrounded by a generally cylindrical nut 250. The nut 250 is threadedly engaged with the thread 241 of the piston rod 240. In the present embodiment, the nut 250 comprises a threaded section with an inner thread 256 that engages the outer thread of the piston rod 240. The threaded section is located in a proximal part 251 of the nut 250, at the proximal end of the nut 250. With other embodiments, the threaded section may also cover other parts of the nut 250 or be located at other portions of the nut 250. The nut 250 further permanently surrounds the stop feature 243 of the piston rod 240, irrespective of the set and/or delivered doses.

The nut 250 has a distal part 252 that is surrounded by a proximal part 274 of a clutch member 270 of the dosing mechanism 230. The nut 250 is rotationally fixed to the clutch member 270 and axially movable with respect to the clutch member 270.

In the present embodiment, the nut 250 is engaged with the clutch member 270 by a splined connection between the nut 250 and the clutch member 270. The splined connection exemplarily comprises longitudinal grooves 254 that are located on the outer surface of the distal part 252 of the nut 250 and that are distributed around the circumference of the nut 250. The grooves 254 run parallel to the longitudinal axis 207 and are engaged by corresponding longitudinal ridges 271 that are distributed on an inner surface of the clutch member 270.

With other embodiments, a rotationally fixed and axially movable connection between the nut 250 and the clutch member 270 may also be achieved by different means, for example by a splined connection between longitudinal ridges on the outer surface of the nut 250 and corresponding longitudinal grooves on the inner surface of the clutch member 270. Additionally or alternatively, the connection may also be mediated by one or more intermediate members.

The clutch member 270 is, at its distal end, fixedly connected to the dose setting member 290 by a connection 277 that prevents both relative axial and relative rotational movement between the clutch member 270 and the dose setting member 290. With other embodiments of the drug delivery device 200, the dose setting member 290 and the clutch member 217 may also be configured as a single component. Alternatively, the connection between the clutch member 270 and the dose setting member 290 may also be mediated by one or more intermediate members.

In its proximal part 251, the nut 250 is surrounded by a driver 350. The driver 350 is configured as a generally cylindrical member. Furthermore, the driver 350 is axially fixed and rotationally movable with respect to the nut 250 and both axially and rotationally movable with respect to the housing 210 during both dose setting and dose delivery. Thereby, the driver 350 is threadedly engaged with the housing 210.

The inner housing 180 comprises at its proximal end an inner sleeve 183 that receives a proximal part 351 of the driver 350. The driver 350 comprises a thread 353 that engages with a drive thread 186 of the inner sleeve 183. In the exemplary embodiment, the thread 353 of the driver 350 is configured as an outer thread and the drive thread 186 is configured as an inner thread. The thread 353 is located on the proximal part 351 of the driver 350. With other embodiments, a threaded connection between the driver 350 and the housing 210 may also be achieved by other ways, for example by an outer thread on the housing 210 and an inner thread on the driver 350.

The dosing mechanism 230 furthermore comprises a dosing member 330. The dosing member 330 is configured as a hollow generally cylindrical member. It surrounds both the driver 350 and the clutch member 270.

The driver 350 is located within a proximal part 331 of the dosing member 330 and the clutch member 270 is located with its proximal part 274 in a distal part 333 of the dosing member 330.

The dosing member 330 is axially and rotationally movable with respect to the housing 210 during both dose setting and dose delivery. It is furthermore threadedly engaged with the housing 210 so that it is forced to move on a helical path with respect to the housing 210.

The dosing member 330 is located between the inner sleeve 183 and an outer wall of the inner housing 180. It has a thread 335 that is engaged with a dose thread 185 of the housing 210. With the exemplary embodiment, the thread 335 of the dosing member 330 is configured as an outer thread and the dose thread 185 is configured as an inner thread located on an inner surface of the outer wall of the inner housing 180. With other embodiments, a threaded connection between the dosing member 330 and the housing 210 may also be realized in different ways. For example, the threaded connection could be provided between the dosing member 330 and the inner sleeve 183 of the inner housing 180.

The dosing member 330 is configured as a dose indication member and comprises the optical markers 331 on its outer surface. The optical markers 331 form a helical scale with a pitch that corresponds to the pitch of the thread 334 on the outer surface of the dosing member 330.

The driver 350 is axially movable and rotationally fixed with respect to the dosing member 330 during both dose setting and dose delivery. With the exemplary embodiment, this is achieved by a splined connection between the driver 350 and the dosing member 330.

The driver 350 comprises radially extending longitudinal splines 360 that engage with corresponding longitudinal grooves 341 provided on an inner surface of the dosing member 330. The splines 360 are located in the distal part 359 of the driver 350 and the grooves are located in a proximal part 332 of the dosing member 330. With other embodiments, the splined connection between the driver 350 and the dosing member 330 may also be achieved in different ways. For example, the driver 350 may comprise grooves that are engaged by corresponding splines of the dosing member 330.

The dose selector member 310 is axially fixed and rotationally movable with respect to the dosing member 330. Therefore, the dose selector member 310 is forced to axially follow a movement of the dosing member 330 while the dosing member 330 is free to rotate with respect to the dose selector member 310, which itself is rotationally fixed with respect to the housing 210.

The dosing member 330 is received within the dose selector member 310. With the current embodiment, a proximal part 317 of the dose selector member 310 receives the distal part 333 of the dosing member 330. The clutch member 270, the proximal part 274 of which is located within the dosing member 330, axially extends with its distal part 275 from the dosing member 330. The distal part 275 of the clutch member 270 thereby extends through an opening 323 in a radially orientated inner wall 322 of the dose selector member 310, which inner wall 322 separates the proximal part 317 of the dose selector member 310 from a distal part 311.

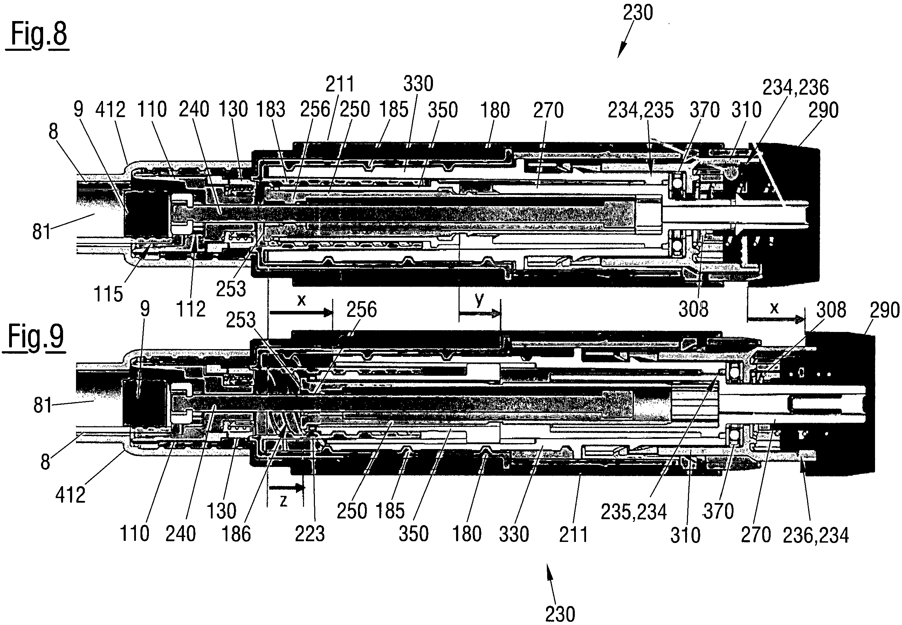

**Fig.** 8 shows a longitudinal cut of the dosing mechanism 230 of the drug delivery device 200 through the first cutting plane prior to setting a dose to be delivered by the drug delivery device 200. To set the dose, the dose setting member 290 is gripped by a user and rotated with respect to the housing 210. This causes the clutch member 270 to rotate together with the dose setting member 290. Due to the rotationally fixed connection between the clutch member 270 and the nut 250, the nut 250 also rotates together with the dose setting member 290. Since the piston rod 240 is rotationally fixed with respect to the housing 210 and the piston rod 240 is threadedly engaged with the nut 250, rotation of the nut 250 causes the nut 250 to axially advance along the piston rod 240. When increasing the set dose, the nut 250 travels in the distal direction, and when decreasing the set dose, the nut 250 travels in the proximal direction.

During dose setting, the dose setting member 290 is rotationally fixed with respect to the dosing member 330. This is achieved by a clutch mechanism 234 that comprises a first part 235 that acts between the dose setting member 290 and the dosing member 330.

The first part 235 of the clutch mechanism 234 comprises clutch elements 336 that are located on the dosing member 330 and that engage, during dose setting, with corresponding clutch elements 273 located on the clutch member 270. The engagement between these clutch elements 336, 273 prevents relative rotational movement between the dose setting member 290 and the dosing member 330 while allowing axial movement for disengagement of the first part 235 of the clutch mechanism 234.

Since the first part 235 of the clutch mechanism 234 is closed during dose setting, the dosing member 330 rotates together with the dose setting member 270. The threaded engagement between the dosing member 330 and the housing 210 then causes the dosing member 330 to axially travel within the housing 210. Upon increasing the set dose, the dosing member 330 travels in the distal direction, and upon decreasing the set dose, the dosing member 330 travels in the proximal direction.

Since the dose selector member 310 is axially fixed with respect to the dosing member 330, distal movement of the dosing member 330 causes the dose selector member 310 to axially travel out of the housing 310 in the distal direction, thereby also moving the dose setting member 290 into the distal direction, while proximal movement of the dosing member 330 causes the dose selector member 310 to axially travel into the housing 210 thereby also moving the dose setting member 290 into the proximal direction.

As the dosing member 330 is rotationally fixed with respect to the driver 350, rotation of the dosing member 330 also causes the driver 350 to rotate together with the dose setting member 290. The threaded connection between the driver 350 and the housing 210 then causes the driver 350 to move in the distal direction when increasing the set dose and to move in the proximal direction when decreasing the set dose.

A first pitch of the threaded connection between the piston rod 240 and the nut 250 and a second pitch of the threaded connection between the driver 350 and the housing 210 are matched to each other to cause the nut 250 and the driver 350 to travel essentially the same axial distance upon movement of the dose setting member 290. The first and second pitches are smaller than a third pitch of the threaded connection between the dosing member 330 and the housing 210. This causes the dosing member 330 to travel a larger axial distance upon rotation of the dose setting member 290 than the nut 250 and the driver 350.

**Fig. 9** shows the dose setting mechanism 232 after a dose has been set. During dose setting, the dosing member 330 has traveled a first distance x in the distal direction, while the driver 350 has traveled a second distance y and the nut 250 has traveled a third distance z. The first distance x is larger than the second and third distances y, z.

Due to manufacturing tolerances, the first pitch of the threaded connection between the piston rod 240 and the nut 250 varies among different threaded connections between a minimum first pitch and a maximum first pitch and the second pitch of the threaded connection between the driver 350 and the housing 210 varies among different threaded connections between a minimum second pitch and a maximum second pitch. With the drug delivery device 200, the maximum first pitch is smaller or at least equals the minimum second pitch. This ensures that the second distance y traveled by the driver 350 in the distal direction is always slightly larger than the third distance z traveled by the nut 250.

The dose setting member 290, which also acts as an actuation member to effect injection of the set dose, is axially movable with respect to the dose selector member 310 between a distal position and a proximal position. A biasing member 308, which is configured as a compression spring, biases the dose setting member 290 into the distal position during dose setting.

To effect ejection of a set dose, the user of the device 200 pushes the dose setting member 290 from the distal position into the proximal position. This transfers the dosing mechanism 230 from a dose setting state into a dose delivery state.

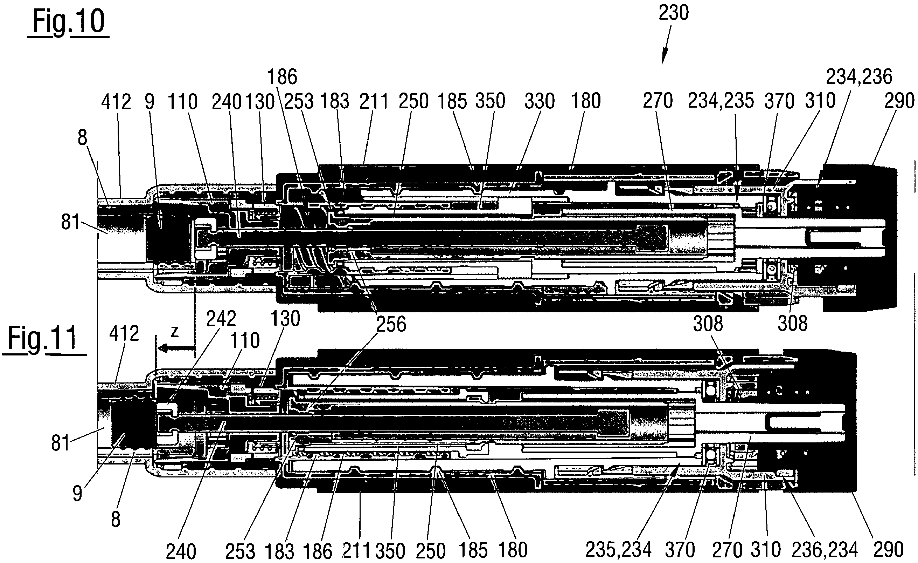

**Fig. 10** shows the dosing mechanism 230 after the dose has been set and the dosing mechanism 230 has been transferred from the dose setting state into the dose delivery state. Moving the dose setting member 290 into the proximal direction also causes the clutch member 270 to move into the proximal direction. Thereby, the first part 235 of the clutch mechanism 234 opens and the clutch elements 273 of the clutch member 270 are disengaged from the clutch elements 336 of the dosing member 330. Therefore, the dosing member 330 and the driver 350 are free to rotate with respect to the dose setting member 290, the clutch member 270 and the nut 250.

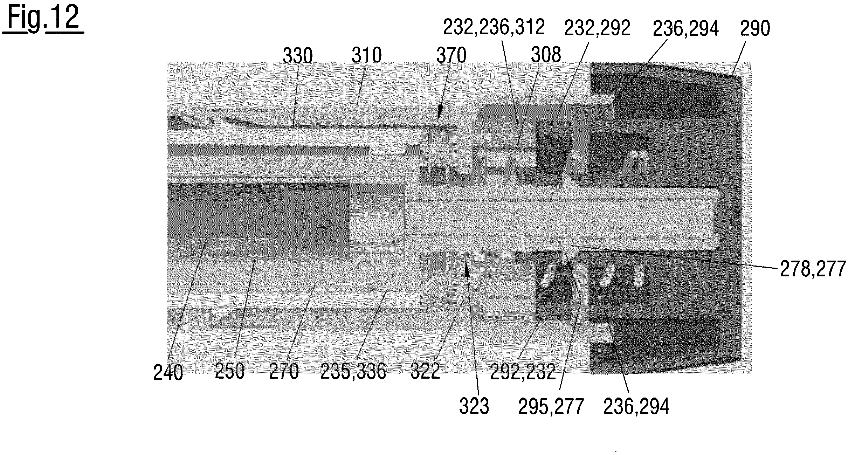

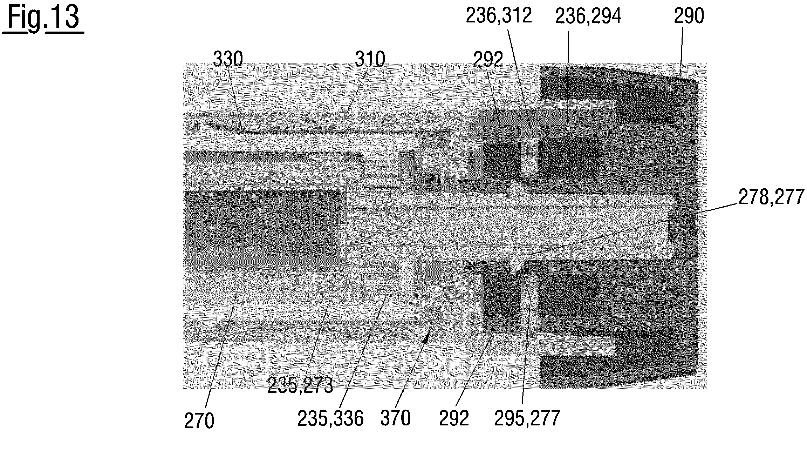

Proximal movement of the dose setting member 290 with respect to the dose selector member 310 at the same time causes a second part 236 of the clutch mechanism 234 to close and to rotationally lock the nut 250 with respect to the piston rod 240 and the housing 210. The second part 236 of the clutch mechanism 234 acts between the dose selector member 310 and the dose setting member 290 and is further described in connection with Fig. 12 and Fig. 13 below.

Further pushing the dose setting member 290 in the proximal direction then causes the dose selector member 310 to linearly move back into the housing 210. The dose selector member 310 thereby pushes against the dosing member 330, which causes the dosing member 330 to rotate due to its threaded engagement with the housing 210. Rotation of the dosing member 330 is transferred to the driver 350, which therefore also moves into the proximal direction due to its threaded engagement with the housing 210.

The difference in the pitches of the threaded connection between the dosing member 330 and the housing 210 and the threaded connection between the driver 350 and the housing 210 thereby causes a mechanical advantage that translates a first axial force exerted by the user and acting on the dosing member 330 into a second axial force exerted by the driver 350. With the dose delivery device 200, the second axial force is larger than the first axial force.

When moving in the proximal direction during dose delivery, the driver 350 pushes axially against the nut 250 and thereby advances the nut 250 in the proximal direction. Since the nut 250 is blocked from rotation with respect to the piston rod 240 during dose delivery due to its connection to the housing 210 via the clutch member 270, the dose setting member 290 and the dose selector member 310, the threaded connection between the nut 250 and the piston rod 240 axially fixes the nut 250 and the piston rod 240 with respect to each other during dose delivery. Therefore, the axially moving nut 250 urges the piston rod 240 to also move in the proximal direction and to thereby advances the plunger 9 to expel the drug from the drug compartment 81.

**Fig. 11** shows the dosing mechanism 230 after the dose has been delivered. The nut 250, the driver 350 and the dosing member 330 have returned to their initial positions while the piston rod 240 has been advanced in the proximal direction by the third distance z. Since the piston rod 250 presses against the plunger 9 via the piston disc 242, the plunger 9 has also been moved by the third distance z in the proximal direction.

**Fig. 12** shows the clutch mechanism 234 of the dosing mechanism 230 in the dose setting state and **Fig. 13** shows the clutch mechanism 234 in the dose delivery state.

In the dose setting state shown in Fig. 12, the dose setting member 290 and the clutch member 270 are in their distal position with respect to the dose selector member 310. The first part 235 of the clutch mechanism 234 is closed and rotationally fixes the clutch member 270 to the dosing member 330.

The second part 236 of the clutch mechanism 234 is configured to rotationally fix the dose setting member 290 to the dose selector member 310 during dose delivery. The second part 236 comprises clutch elements 294 that are provided at the dose setting member 290. As can be seen from Fig. 13, moving the dose setting member 290 into the proximal position brings the clutch elements 294 into engagement with teeth 312 of the dose selector member 311, thereby rotationally locking the dose setting member 290 to the dose selector member 311. The teeth 312 are provided on the inner surface of the distal part 311 of the dose selector member 310. The teeth 312 constitute clutch elements of the dose selector member 310. As can also be seen from Fig. 13, pressing the dose setting member 290 into the proximal position disengages the clutch elements 273 of the clutch member 270 from the clutch elements 336 of the dosing member 330.

Generally speaking, the clutch mechanism 234 rotationally locks the nut 250 to the dosing member 330 and to the driver 350 during dose setting, while it rotationally locks the nut 250 to the piston rod 240 and to the housing 210 during dose delivery.

The dosing mechanism 230 of the drug delivery device 200 further comprises a dose definition mechanism 232 that acts between two members of the dosing mechanism 230 that are rotationally movable with respect to each other during dose setting. The dose definition mechanism 232 defines distinct rotational positions of the dose setting member 290 with respect to the housing 210 that correspond to individual settable doses of the drug to be ejected by the dosing mechanism 230.

With the drug delivery device 200, the dose definition mechanism 232 acts between the dose selector member 310 and the dose setting member 290. Thereby, the dose definition mechanism 232 is realized by direct engagement between the dose setting member 290 and the dose selector member 310. With other embodiments of dose delivery devices according to the present disclosure, the dose definition mechanism 232 may also act between the dose selector member 310 and the dose setting member 290 via additional elements that are located between the dose selector member 310 and the dose setting member 290. Such an additional element could be, for example, the clutch member 270 and/or the dosing member 330.

As can also be seen from Fig. 12, the dose definition mechanism 232 comprises at least one element 292 that engages with at least one corresponding functional feature, namely with the one of the teeth 312, when the dose setting member 270 reaches a rotational position with respect to the housing 210 that corresponds to a respective dose defined by the functional feature 312. Engagement between the element 292 and the functional feature 312 then provides audible and/or tactile feedback to the user of the drug delivery device 200.

At least one of the element 292 and the functional feature 312 are configured as a flexible element that deflects in a radial direction upon engagement between the element 292 and the functional feature 312. With the drug delivery device 200, the element 292 is configured as such a flexible element.

With the teeth 312, the drug delivery device 200 comprises several functional features 312 that are circumferentially distributed around the longitudinal axis 207 to define a multitude of settable doses. Furthermore, the dose definition mechanism 232 of the drug delivery device 200 comprises a multitude of elements 292, namely four elements 292, that are distributed around the longitudinal axis 207. A relative position between the individual functional features 312 and the individual elements 292 is chosen in a way that at each rotational position of the dose setting member 290 with respect to the housing 210, which correspond to a settable dose, all elements 292 engage with one of the functional features 312. Other embodiments of the drug delivery device 200 may also comprise other numbers of elements 292, for example a single element 292.

With the drug delivery device 200, the functional features 312 are located on an inner surface of the dose selector member 310 and the elements 292 are located on an outer surface of the dose setting member 290. Furthermore, the element 292 and the three further elements 292 are configured as flexible arms. They constitute integral parts of the dose setting member 290 and are provided at a proximal end of the dose setting member 290.

The teeth 312 provided on the dose selector member 310 constitute both clutch elements of the second part 236 of the clutch mechanism 234 and functional features, that is dose stops, of the dose definition mechanism 232.

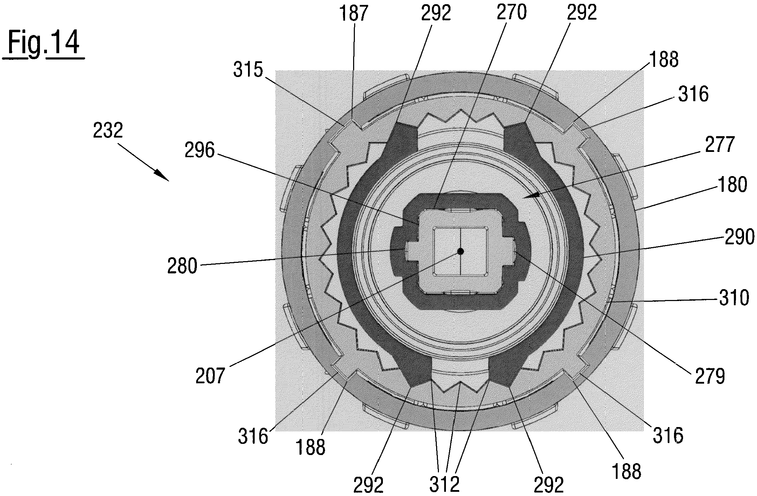

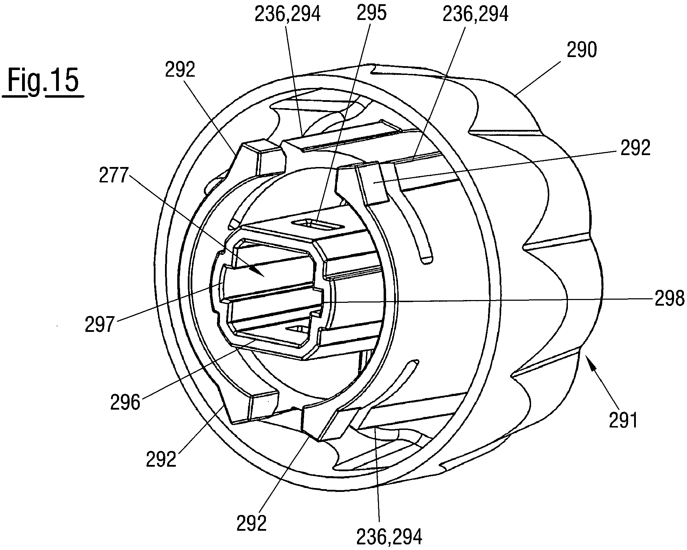

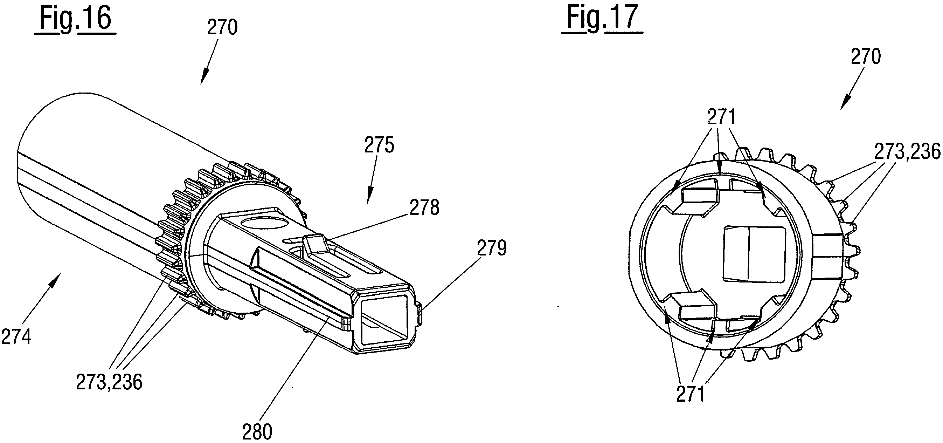

**Fig. 14** shows a radial cut through the dose definition mechanism 232 perpendicular to the longitudinal axis 207. **Fig. 15** shows a perspective view of a proximal side of the dose setting member 290 of the drug delivery device and **Fig. 16** shows a perspective view of a distal side of the clutch member 270.

As can be seen from Fig. 14, the dose definition mechanism 232 defines an uneven number of distinct rotational positions of the dose setting member 290 with respect to the housing 210 that correspond to settable doses, namely 27 rotational positions/settable doses. To ensure correct rotational alignment between the first part 235 and the second part 236 of the clutch mechanism 234, the dose setting member 290 is connected to the clutch member 270 by a connection 277 having a coding feature that only allows a single relative rotational orientation between the clutch member 270 and the dose setting member 290.

The connection 277 comprises a non-circular, namely rectangular, opening 296 within the dose setting member 290, the opening 296 receiving the non-circular, namely rectangular, distal part 275 of the clutch member 270. The coding feature then comprises a first longitudinal ridge 279 and a second longitudinal ridge 280, whereby the longitudinal ridges 279, 280 radially extend from opposite sides of the distal part 275 of the clutch member 270. The first ridge 279 is received in a corresponding first longitudinal groove 297 located within the opening 296 of the dose setting member 290 and the second ridge 280 is received within a corresponding second longitudinal groove 298 of the dose setting member 290. The first ridge 279 and the first groove 297 have a width that differs from the respective widths of the second ridge 280 and the second groove 297. With other embodiments of the drug delivery device 200, the coding feature of the connection 277 could also be realized in a different way for example by ridges provided on the dose setting member 290 and corresponding grooves provided at the clutch member 270.

To permanently and non-releasably couple the dose setting member 290 to the clutch member 270 during assembly of the drug delivery device 200, the clutch member 270 is locked to the dose setting member 290 by a snap-fit connection 277. As can be seen, for example, in Fig. 15 and Fig. 16, this snap-fit connection 277 comprises two flexible snap hooks 278 that are located at opposing sides of the distal part 275 of the clutch member 270. Upon insertion of the distal part 275 into the opening 296 of the dose setting member 290, the snap hooks 278 engage with corresponding recesses 295 provided in the side surfaces of the opening 296. With other embodiments, the non-releasable connection 277 could also be provided in different ways, for example by at least one snap-hook located at the dose setting member 290 and at least one corresponding recess located on the clutch member 270.

As it will be described in further detail below, axial positions of the dosing member 330 that correspond to a minimum and a maximum settable dose are defined by interaction between the dosing member 330 and the inner housing 180. A connection between the dose selector member 310 and the inner housing 180 is therefore configured in a way that these axial positions correspond to settable doses defined by the dose definition mechanism 232.

With the drug delivery device 200, such a connection, which is shown in Fig. 14, is achieved by restricting a relative rotational orientation between the dose selector member 310 and the inner housing 180 to a single orientation. The connection is established by a first longitudinal ridge 315, which is provided on the outer surface of the dose selector member 310 and which is received in a corresponding first longitudinal groove 187 provided on an inner surface of the inner housing 180. The first longitudinal ridge 315 has a width that is different than the width of three further longitudinal ridges 316 that are distributed over the remaining outer surface of the dose selector member 310. The further longitudinal ridges 316 engage with corresponding further longitudinal grooves 188 that are distributed over the remaining inner surface of the inner housing 180 and have corresponding widths that are different from the width of the longitudinal groove 187.

In general, the first longitudinal ridge 315 and the first longitudinal groove 187 form a first longitudinal splined connection and the further longitudinal ridges 316 and the further longitudinal grooves 188 form at least a second longitudinal splined connection, the first longitudinal splined connection having a different width than the second longitudinal splined connection. With other embodiments, the connection between the dose selector member 310 and the inner housing 180 could also be achieved in different ways, for example by splined connections having grooves located on the dose selector member 310 and ridges located on the inner housing 180.

**Fig. 17** shows a perspective view of a proximal side of the clutch member 270 of the drug delivery device 200. On the inner surface of its proximal part 274, the clutch member 270 has the longitudinal ridges 271 that engage with the longitudinal grooves 254 of the nut 250 to rotationally locked the clutch member 270 with respect to the nut 250 while at the same time allowing relative axial movement. Generally speaking, the longitudinal ridges 271 and the corresponding longitudinal grooves 254 form a splined connection between the clutch member 270 and the nut 250. With other embodiments, a rotationally fixed and axially movable connection between the clutch member 270 and the nut 250 could also be achieved by other means, for example, by longitudinal ridges provided on the nut 250 and corresponding grooves provided on the clutch member 270.

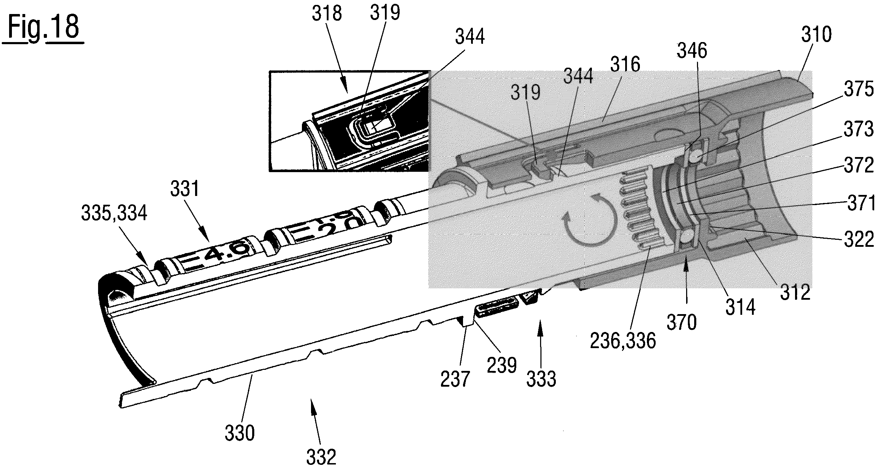

**Fig. 18** shows a longitudinal cut through the dosing member 330 and the dose selector member 310 of the drug delivery device 200. The drug delivery device 200 comprises a friction reduction mechanism that acts between the dosing member 330 and the dose selector member 310. The friction reduction mechanism is configured to reduce friction upon relative rotational movement between the dosing member 330 and the dose selector member 310.

The friction reduction mechanism comprises a ball bearing 370 which is provided between a distal end surface 346 of the dosing member 330 and a contact surface 314 of the dose selector member 310. The contact surface 314 is thereby provided by the proximal front surface of the radial inner wall 322 of the dose selector member 310.

When increasing the dose during dose setting, a distally directed axial force is transferred from the dosing member 330 via the ball bearing 370 to the dose selector member 310. When pushing the dose selector member 310 in the proximal direction during injection, a proximally directed axial force is transferred from the dose selector member 310 via the ball bearing 370 to the dosing member 330.

The ball bearing 370 comprises several balls 375 that are sandwiched between a distal disc 371 touching the contact surface 314 of the dose selector member 310 and a proximal disc 372 contacting the end surface 346 of the dosing member 330. Furthermore, the ball bearing 370 comprises a holder 372, which is sandwiched between the distal disc 371 and the proximal disc 372. The holder 372 surrounds the balls 375 in the radial direction and holds them into place.

The dose selector member 310 is axially fixed to the dosing member 330. Distal movement of the dose selector member 310 with respect to the dosing member 330 is prevented by a snap-fit connection. The snap-fit connection comprises a circumferential annular ridge 344 on an outer surface of the dosing member 330 and at least one, namely four, flexible members 319 formed on the dose selector member 310. When moving the dose selector member 313 in the proximal direction over the dosing member 330, the flexible members 319 snap over the annular ridge 344 and engage with a distal front surface of the annular ridge 344. With other embodiments, distal movement of the dose selector member 310 may also be achieved by a different connection, for example, by flexible members of the dosing member 330 engaging with an annular ridge of the dose selector member 310. Proximal movement of the dose selector member 310 with respect to the dosing member 330 is prevented by the contact surface 314 of the dose selector member 310 resting via the ball bearing 370 against the distal end surface 346 of the dosing member 330.

With other embodiments of the drug delivery device 200, the bearing element 370 could also be configured in other ways. For example, the bearing element 370 could also be configured as a disc bearing, such as a single annular disc made from a low-friction material, such as PTFE.

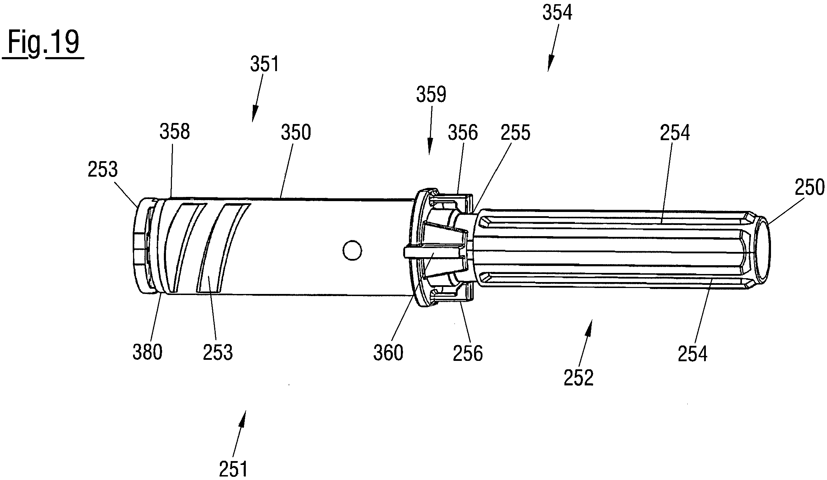

**Fig. 19** shows a perspective view of a connection 354 between the nut 250 and the driver 350 of the first drug delivery device 200. The connection 354 is configured to axially restrain the driver 350 with respect to the nut 250 and to allow relative rotational movement between the nut 250 and the driver 350.

The connection 354 comprises two flexible arms 356 that are formed at a distal end of the driver 350 and that radially protrude inwardly to engage with an annular detent 255 between the proximal and distal parts 251, 252 of the nut 250. When moving the driver 350 distally with respect to the nut 250, the flexible arms 356 abut against the distal side surface of the annular detent 255. A clearance is provided between the distal side surface and the flexible arm 356 to allow the nut 250 and the driver 350 to travel different distances into the distal direction during dose setting.

The drug delivery device 200 comprises a further friction reduction mechanism that is configured to reduce friction between the nut 250 and the driver 350 upon relative rotational movement with respect to each other during dose delivery. The further friction reduction mechanism comprises a bearing element 380 that is positioned between the driver 350 and the nut 250.

The bearing element 380 is located between a proximal front surface 358 of the driver 350 and a protrusion 253 located at the proximal end of the nut 250. The proximal protrusion 253 defines a rim that radially extends from the nut 250. When rotating into the inner sleeve 183 of the inner housing 180 during dose delivery, the driver 350 pushes via the further bearing element 380 against the protrusion 253 and thereby also pushes the nut 250 in the proximal direction.

The bearing element 380 is configured as a bearing disc made from a low-friction material, such as PTFE. With other embodiments, the bearing element 380 could also be configured as a different type of bearing, for example as a ball bearing.

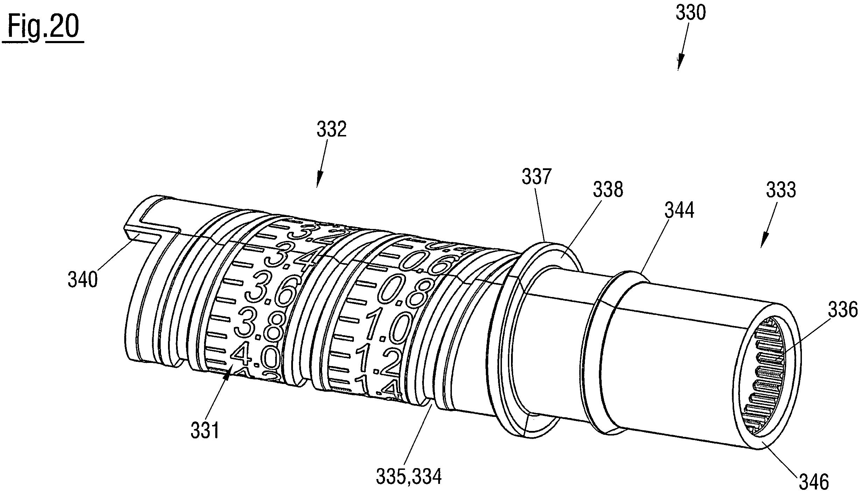

**Fig. 20** shows a perspective view of the dosing member 330 of the first drug delivery device 200. The dosing member 330 comprises a maximum dose stop 337 that is configured to engage with the inner housing 180 upon setting a maximum dose. Engagement of the maximum dose stop 337 with the inner housing 180 thereby limits further axial movement of the dosing member 330 in the distal direction and defines the axial and rotational position of the dosing member 330 that corresponds to the maximum dose settable by the dosing mechanism 230.

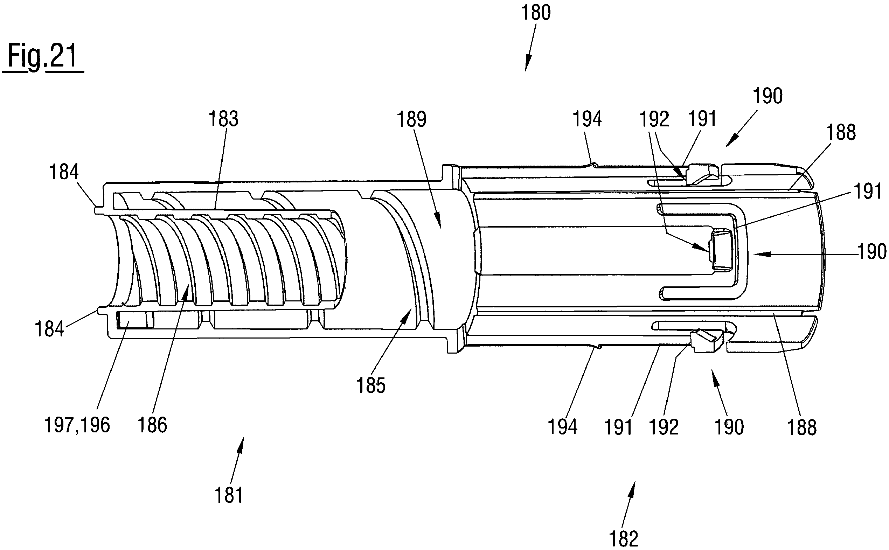

As can be seen from **Fig. 21****,** which shows the inner housing 180 in a longitudinal cut through the longitudinal axis 207, the inner housing 180 comprises at least one maximum stop feature 190, namely four maximum stop features 190. The maximum stop features 190 are formed as integral parts of the inner housing 180. They each comprise a flexible hook 191 that radially protrudes inwardly into a housing cavity 189 of the inner housing 180 that receives the dosing member 330. The flexible hooks 191 each comprise a limiting surface 192 that is orientated perpendicular to the longitudinal axis 207 and faces into the proximal direction.

Upon insertion of the dosing member 330 into the housing cavity 189, the flexible hooks 191 snap over the maximum dose stop 337 to subsequently limit axial movement of the dosing member 330 into the distal direction. When setting the maximum dose, a distal stopping surface 338 of the maximum dose stop 337 abuts against the limiting surfaces 192 of the maximum stop features 190. The distal stopping surface 338 is configured as a side surface of the maximum dose stop 337 and is orientated perpendicular to the longitudinal axis 207.

The dosing member 330 also comprises a zero dose stop 340 that defines the rotational and axial position of the dosing member 330 that corresponds to a zero dose or no set dose. The zero dose stop 340 is located at the proximal end of the dosing member 330. It is configured as a limiting surface that is orientated parallel to the longitudinal axis 207. The limiting surface forms a side surface of a cut-out at the proximal end of the dosing member 330.

When reaching the zero-dose position, the zero dose stop 340 engages with a zero stop feature 196 of the inner housing 180. The zero stop feature 196 is located at the proximal end of the housing cavity 189. Like the zero dose stop 340, the zero stop feature 196 is also configured as a limiting surface that is orientated parallel to the longitudinal axis 207. Furthermore, the limiting surface of the zero stop feature 196 is orientated parallel to the limiting surface of the zero dose stop 340.

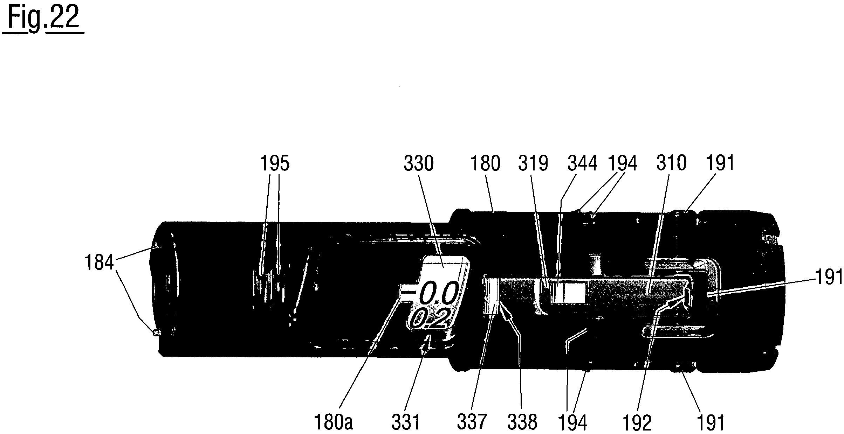

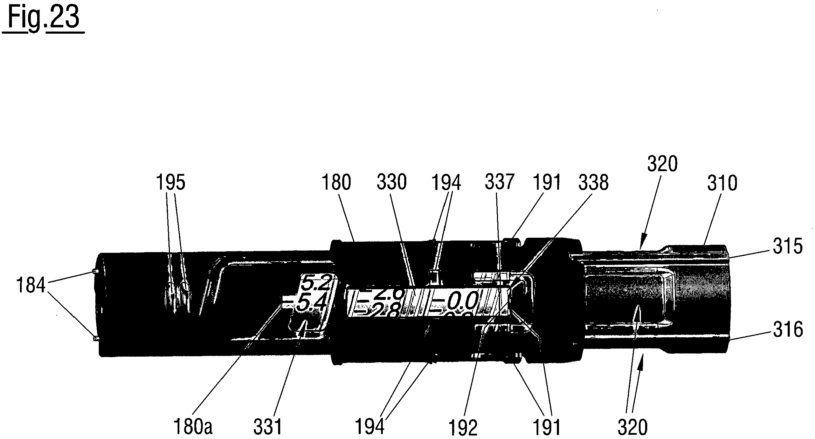

**Fig. 22** shows a perspective view of the inner housing 180 with the dosing member 330 in the zero-dose position and **Fig. 23** shows a perspective view of the inner housing 180 with the dosing member 330 in a maximum dose position.

The dosing member 330 is configured to perform two full rotations about the longitudinal axis 207 when moving from the zero-dose position to the maximum dose position. In the zero-dose position, a minimum dose marker is visible in the window 188a of the inner housing 180 indicating a set dose of 0.0, and in the maximum dose position, a maximum dose marker is visible in the window 188a indicating a set dose of 5.4.

The inwardly protruding maximum stop features 190 of the inner housing 180 are located inside longitudinal detents 320 of the dose selector member 310. This allows the limiting surfaces 192 to engage with the stopping surface 338 despite the dose selector member 310 surrounding the dosing member 330 in its distal part 333.

The inner housing 180 is both axially and rotationally locked with respect to the outer housing 211. As can be seen from Fig. 22 and Fig. 23, the inner housing 180 comprises protrusions 194 that are circumferentially distributed around the outer surface of the distal part of the inner housing 180. Furthermore, the inner housing comprises radial protrusions 195 that are located on the outer surface of the proximal part of the inner housing 180. With the embodiments shown in Fig. 22 and Fig. 23, two radial protrusions 195 are placed next to each other parallel to the longitudinal axis 207. The two protrusions 195 are both placed at the same circumferential position on the outer surface of the inner housing 180.

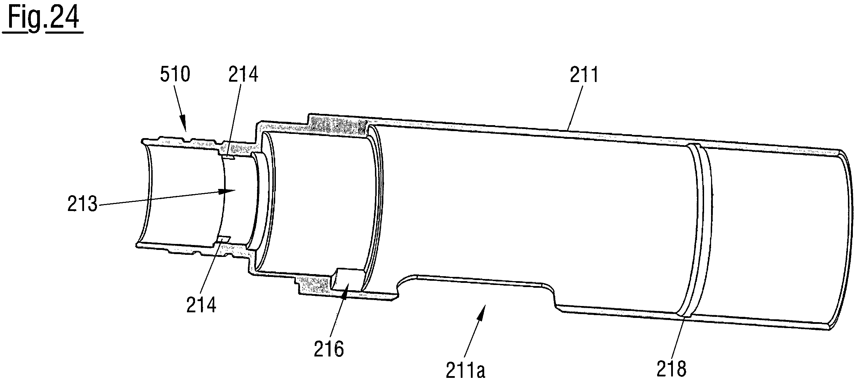

As can be seen from **Fig. 24****,** which shows a longitudinal cut through the outer housing 211 of the first drug delivery device 200, the outer housing 211 comprises, on its inner surface, a circumferential groove 218, which is located in the distal part of the outer housing 211. Furthermore, the outer housing 211 comprises a detent 216 in a proximal part of its inner surface.

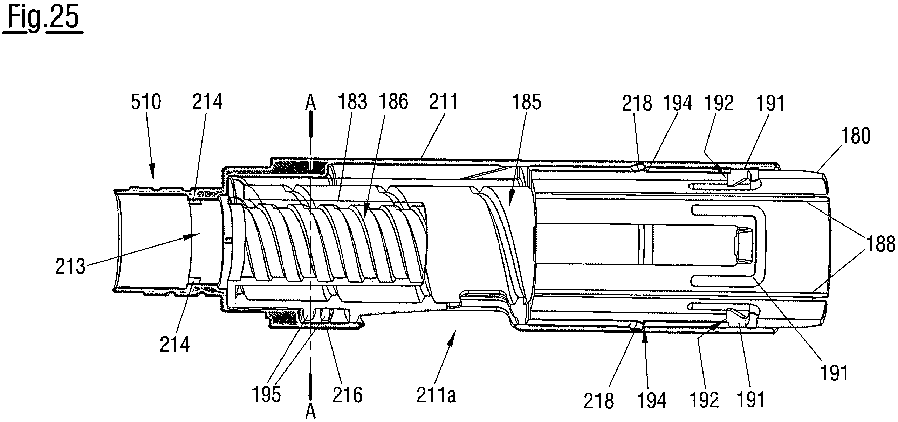

**Fig. 25** shows longitudinal cut of the inner housing 180 mounted within the outer housing 211 of the first drug delivery device 200. The protrusions 194 in the distal part of the inner housing 180 are configured to prevent axial movement of the inner housing 180 with respect to the outer housing 211 in the distal direction. They snap into the circumferential groove 218 when mounting the inner housing 180 inside the outer housing 211 by inserting the inner housing 180 into the outer housing 211 from its distal end. When pushing the inner housing 180 in the distal direction after full insertion, the protrusions 194 engage with the distal end surface of the circumferential groove 218 and thereby prevent axial movement. In the proximal direction, the inner housing 180 abuts against a step within the inner surface of the outer housing 211, which step is limiting proximal movement of the inner housing.

With other embodiments of the drug delivery device 200, axial movement of the inner housing 180 with respect to the other housing 211 may also be prevented by other means. For example, the outer housing 211 may comprise flexible elements that engage with grooves positioned on the outer surface of the inner housing 180.

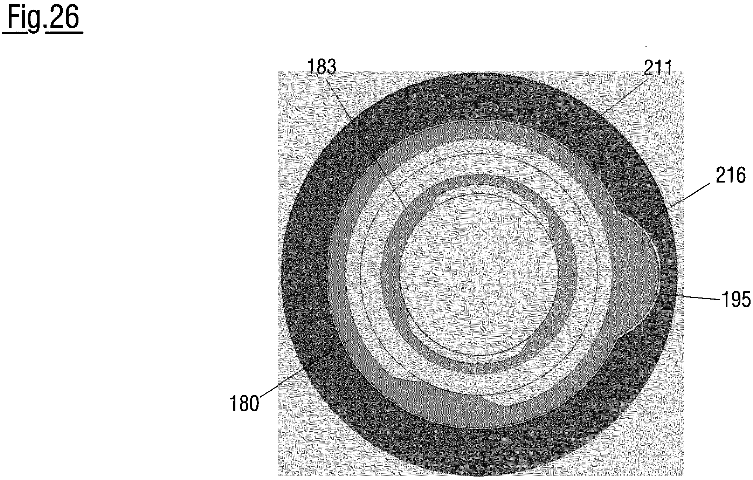

The radial protrusions 195 in the proximal part of the inner housing 180 are configured to prevent rotational movement of the inner housing 180 with respect to the outer housing 211. They engage with the detent 216 in the proximal part of the inner surface of the outer housing 211. This is further illustrated in **Fig. 26****,** which shows a radial cut through the outer and inner housing 211, 180 of the drug delivery device 200 through the line A-A shown in Fig. 25. With other embodiments of the drug delivery device 200, rotational movement of the inner housing 180 with respect to the other housing 211 may also be prevented by other means. For example, the outer housing 211 may comprise protrusions that engage with detents positioned on the outer surface of the inner housing 180.

The first drug delivery device 200 is configured to deliver a multitude of individual doses from the cartridge 8 attached to the device 200 via the cartridge holder 412. Furthermore, the drug delivery device 200 is configured as a reusable drug delivery device, which allows a user to replace an empty cartridge 8 by a new cartridge 8 after the last dose has been delivered from a given cartridge 8.

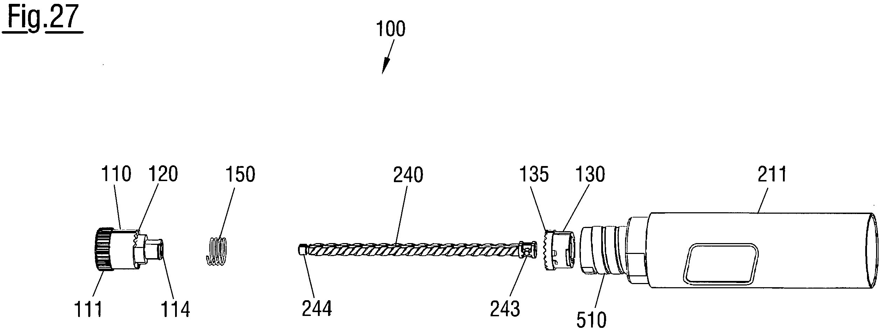

The resetting mechanism 100, which is shown in an exploded partial view **in** **Fig. 27****,** thereby allows to move the piston rod 240 back into the housing 210 after delivery of the last dose and disengagement of the cartridge holder 412 from the housing 210.

The resetting element 110, which guides the piston rod 240 in the non-circular opening 114, is mounted to the housing 210, namely the outer housing 211. Connection between the resetting element 110 and the housing 210 is achieved by a coupling part 130, which is both rotationally and axially fixed with respect to the housing 210. The coupling part 130 is configured as an insert received within the housing 210, namely within the outer housing 211.

A biasing element 150, which is configured as a compression spring, is mounted between the coupling part 130 and the resetting element 110 and therefore also between the housing 210 and the resetting element 110. The biasing element 150 biases the resetting element 110 in the proximal direction into a proximal position with respect to the housing 210 and the coupling part 130.

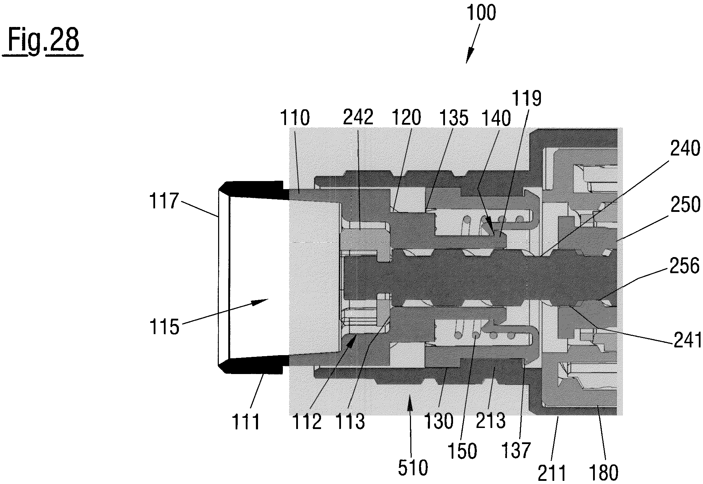

**Fig. 28** shows a longitudinal cut through the resetting mechanism 100 of the first drug delivery device 200 with the resetting element 110 in the proximal position. In this configuration, the resetting element 110 is rotationally movable with respect to the housing 210. The resetting element 110 comprises a gripping zone 111 at its proximal end, which may be gripped by the user of the device 200 to rotate the resetting element 110.

Due to the rotationally fixed connection between the resetting element 110 and the piston rod 240, the piston rod 214 is forced to rotate together with the resetting element 110 when the user rotates the resetting element 110. Engagement between the thread 241 of the piston rod 240 and the thread 256 of the nut 250 then forces the piston rod 240 to travel into the distal direction back into the housing 210 upon rotating the resetting element 110 in a resetting direction. In this way, the resetting element 110 is configured to move the piston rod 240 back into the housing 210 upon rotation by the user.

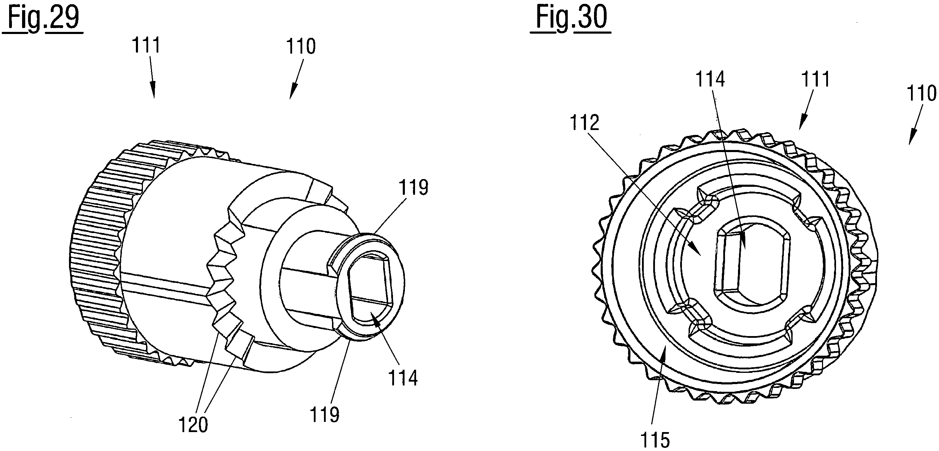

**Fig. 28** shows a distal perspective view of the resetting element 110, **Fig. 29** shows a proximal perspective view of the resetting element 110 and **Fig. 30** shows a proximal perspective view of the coupling part 130 of the resetting mechanism 110.

As can be seen from Fig. 28, a distal part of the resetting element 110 is received within the coupling part 130. In the proximal position shown in Fig. 28, further proximal movement of the resetting element 110 under the action of the biasing member 150 within the coupling part 130 is prevented by the resetting element 110 engaging with the coupling part 130. Thereby, a radial stop 119 located at the distal end of the resetting element 110 engages with a corresponding stop feature 141 on an inner surface of the coupling part 130. With other embodiments, further proximal movement of the resetting element 110 may also be achieved in other ways.

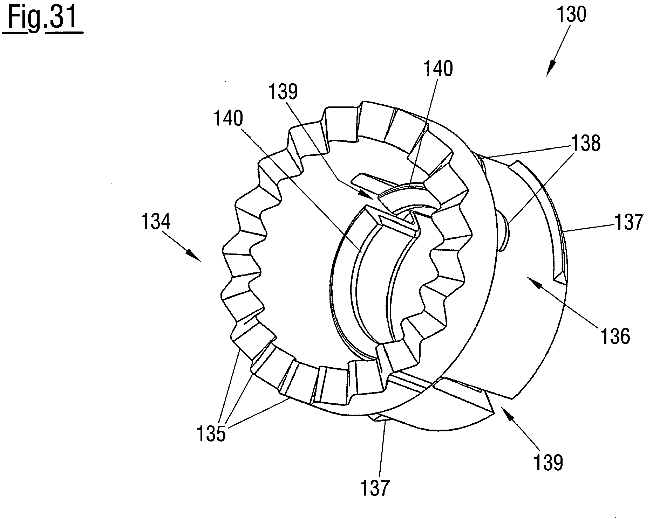

As can also be seen from **Fig. 28** and **Fig. 31****,** the coupling part 130 is axially locked with respect to the housing 210 by an annular notch 136 that is located on the outer surface of the coupling part 130, whereby the annular notch 136 is received in a corresponding collar 213 on an inner surface of the outer housing 211. The notch 136 is distally limited by a locking structure 137 that radially protrudes from the outer surface of the coupling part 130. Upon inserting the coupling part 130 into the outer housing 211 in the distal direction, the locking structure 137 flexes radially inwardly and snaps over the annular collar 213 of the outer housing 211. In this way, the coupling part 130 is axially fixed with respect to the housing 210 by a snap-fit connection. With other embodiments, exam movement between the coupling part 130 and the housing 210 could also be achieved with other means, for example by a large located on the housing and a collar or protrusion located at the coupling part 130.

To rotationally lock the coupling part 130 with respect to the housing 210, the coupling part 130 comprises protrusions 138 that are located within the notch 136. The protrusions 138 engage with corresponding detents 214 in the annular collar 213. These detents 214 are shown, inter alia, in Fig. 24. With other embodiments, rotation between the coupling part 130 and the housing 210 could also be achieved by other means, for example by protrusions provided at the housing 210 and corresponding detents provided at the coupling part 130

The locking structure 137 of the coupling part 130 comprises two portions that are separated by longitudinal slots 139. This allows the portions of the locking structure 137 to radially bend inwardly when mounting the coupling part 130 to the outer housing 211. After mounting the coupling part 130, the portions of the locking structure 137 are prevented from bending inwardly by engagement with the inner housing 180.

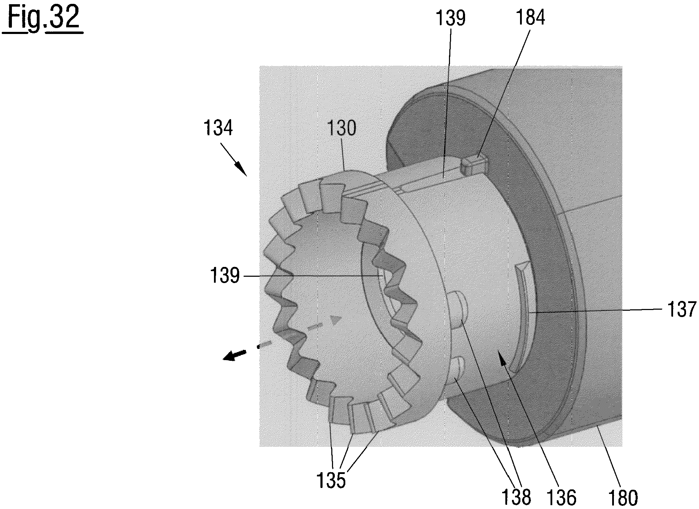

**Fig. 32** shows a perspective view of the coupling part 130 and the inner housing 180. The inner housing 180 comprises at its front surface two longitudinally protruding tappets 184, which are also visible, for example, in Fig. 23. The tappets 184 are received within the longitudinal slots 139 and thereby block the portions of the locking structure 137 from radially bending inwardly.

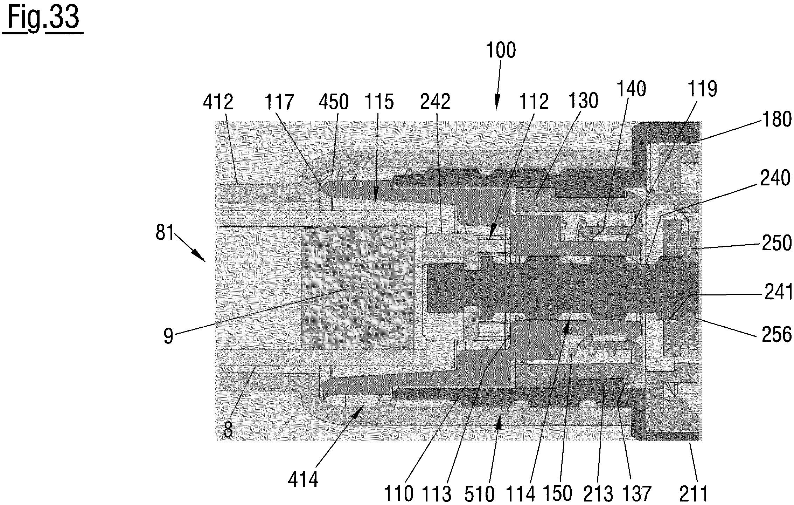

**Fig. 33** shows a longitudinal cut through the resetting mechanism 100 with the dispensing unit 410 attached the drug delivery device 200. When attaching the dispensing unit 410, the inner thread of the connection means 414 of the dispensing unit 410 is screwed onto the outer thread of the connection means 510 of the outer housing 210 until the distal end of the cartridge holder 412 rests against a step formed on the outer surface of the outer housing 211.

During mounting of the dispensing unit 410, the resetting element 110 is moved into the distal direction into its distal position to rotationally lock the resetting element 110 with respect to the housing 210. When being in its distal position, engagement features 120 of the resetting element 110 engage with corresponding engagement features 135 of the coupling part 130 and thereby rotationally lock the resetting element 110 with respect to the coupling part 135.

The engagement feature 120 of the resetting element 110 are configured as distally facing teeth. The engagement features 135 of the coupling part 130 are located at a coupling site, which is formed by a front surface of the coupling part 130. The engagement features 135 are configured as proximally facing teeth that match between distally facing teeth of the engagement feature 120 of the resetting element 110.

In the embodiment shown in Fig. 27 to Fig. 33 the engagement features 120, 135 are configured as symmetric teeth that have circumferential side surfaces that have the same slope. With other embodiments, the teeth of the engagement features 120, 135 may also be configured as asymmetric teeth. For example, the asymmetric teeth may have circumferential side surfaces with different slopes. Thereby, one side surface of the individual teeth may be orientated, for example, parallel to the longitudinal axis 207 and the respective other side surface may be inclined with respect to the longitudinal axis 207. Such asymmetric teeth may, for example, provide a saw-tooth profile.

With asymmetric engagement features 120, 135, side surfaces of the individual engagement features 120, 135 having a steeper slope than the respective other side surfaces may be configured to press against each other when the resetting element 110 is rotated in a circumferential direction that would screw the piston rod 240 back into the housing 210. This efficiently prevents a counter-rotation of the piston rod 240 with respect to the nut 250 during dose delivery.

As can be seen from Fig. 33, the cartridge holder 412 of the dispensing unit 410 directly engages with the resetting element 110 to push the resetting element 110 into the distal direction upon mounting the dispensing unit 410 onto the housing 210. Thereby, a proximally facing contact structure 117 of the resetting element 110 rests against a distally facing contact feature 450 of the cartridge holder 412.

The proximally facing contact structure 117 is configured as a proximal circumferential edge of the resetting member 110. The distally facing contact feature 450 is provided as a distally facing annular surface located at an inwardly protruding step of the cartridge holder 412.

With the cartridge holder 412 mounted to the housing 210, the cartridge 8 does not contact the resetting element 110. Therefore, the resetting element 110 is moved in the distal direction solely by its contact with the cartridge holder 412. The distal end of the cartridge 8 is received inside a cartridge cavity 115 of the resetting element 110, the cartridge cavity 115 being accessible from the proximal side of the resetting element 110.

The direct engagement between the cartridge holder 412 and the resetting element 110 allows, compared to an engagement between the cartridge 8 and the resetting element 110, to configure the engagement features 120, 135 with tighter axial tolerances and a smaller axial height. Typically, the individual cartridges 8 are made from glass and have larger variation in their longitudinal extent then individual cartridge holders 412, which are typically made from a plastic material. Therefore, the engagement features 120, 135 would have to have a comparably large axial height to provide a secure rotational locking between the resetting element 110 and the coupling part 130 irrespective of possible variations in the length of individual cartridges 8 due to manufacturing tolerances.

When being fully retracted into the housing 210, the plunger disc 242 of the piston rod 240 is located within a reception area 112 of the resetting element 110. The reception area 112 is configured as a further cavity that is accessible from the proximal side of the resetting element 110. Furthermore, the reception area 112 is located at the distal end of the cartridge cavity 115. In its fully retracted position, the plunger disc 242 of the piston rod 240 rests against an inner surface 113 of the reception area 112. This inner surface 113 forms the distal end surface of the reception area 112 and surrounds the opening 114 of the resetting element 110 that guides the piston rod 240.

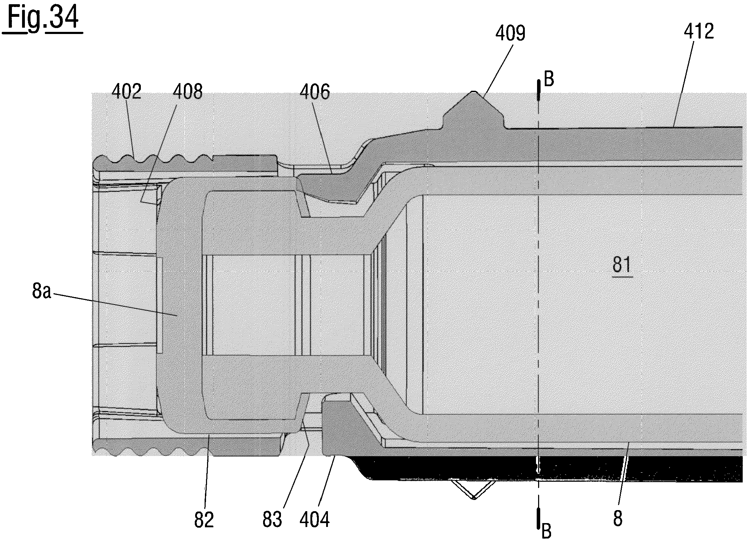

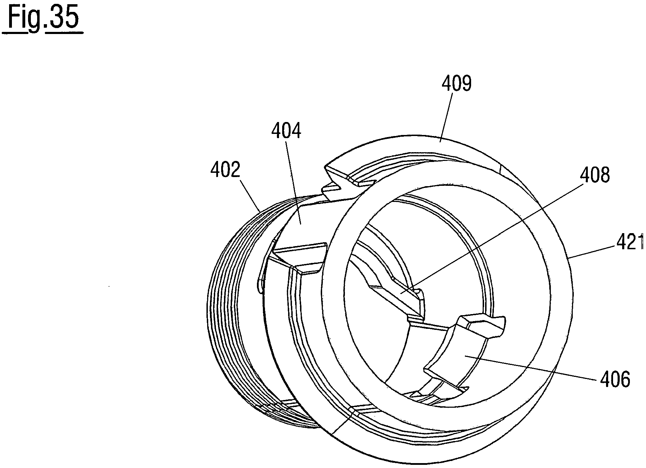

**Fig. 34** shows a longitudinal cut through a proximal end of the cartridge holder 412 attachable to the drug delivery device 200 with the cartridge 8 inserted into the cartridge holder 412. **Fig. 35** shows a perspective distal view of a radial cut through the proximal part of the cartridge holder 412. Inside the cartridge holder 412, the cartridge 8 is pushed against the stop 408 by a push element 406. The push element 406 engages with the distal surface 83 of the annular rim 82 of the cartridge 8. It is configured as a flexible member that snaps over the annular rim 82 when the cartridge 8 is inserted into the cartridge holder 412. The push feature 406 is configured as an integral part of the cartridge holder 412.

The proximal part of the cartridge holder 412 furthermore comprises an annular ridge 409 that radially extends from the outer surface of the cartridge holder 412. The annular ridge 409 is configured to be engaged by a locking arm of the cap 209, which is provided on an inner surface of the cap 209. Engagement between the locking arm and the annular ridge 409 releasably locks the cap 209 to the drug delivery device 200 after attachment.

According to the present disclosure, the drug delivery device 200 may be part of a set of several drug delivery devices and the dispensing unit 410 may be part of a set of several dispensing units, whereby each drug delivery device comprises connection means that only allow attachment of a dedicated dispensing unit and prevents attachment of all other dispensing units of the set and vice versa. The connection means are thereby configured as keyed connectors, which provide a one-to-one assignment between the individual dispensing units and the individual drug delivery devices.

The set of drug delivery devices may comprise further variants of the drug delivery device 200 that have at least one mutual member that is identical among the drug delivery device 200 and the further variants. The set may also comprise different types of drug delivery devices that do not share such a mutual member with the drug delivery device 200.

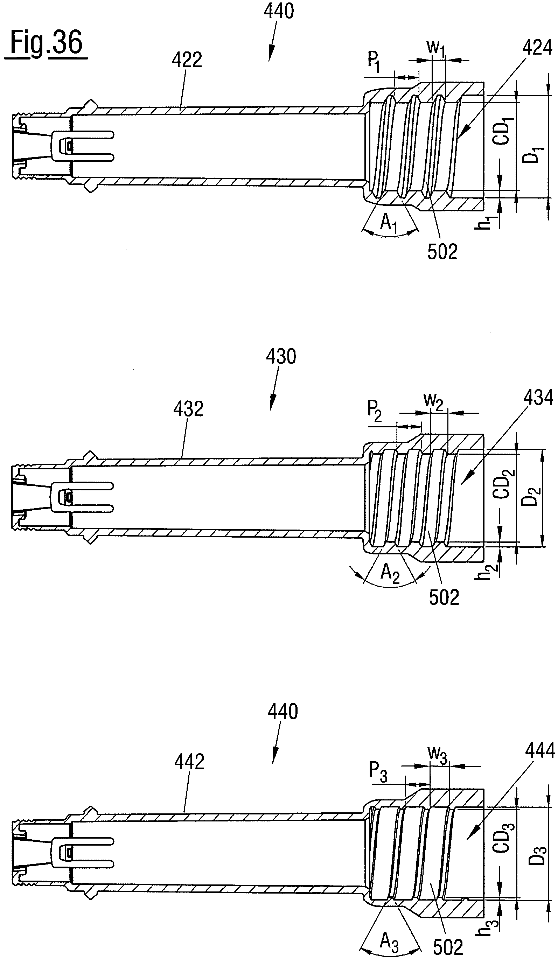

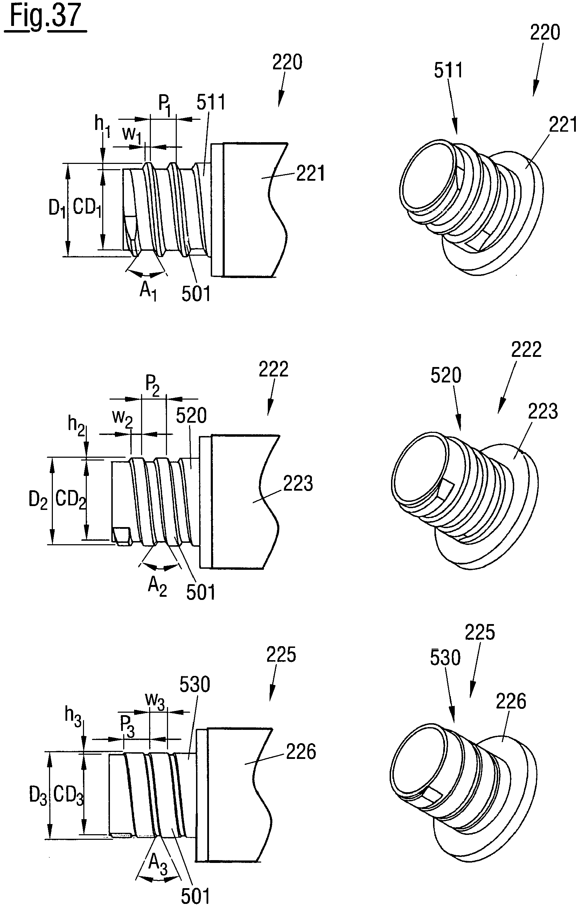

**Fig. 36** and **Fig. 37** show a set of three drug delivery devices and a set of three corresponding dispensing units according to the present disclosure. Each drug delivery device is connected to its corresponding dispensing unit by a keyed connection that prevents the respective drug delivery device from connecting to the other dispensing units and which, vice versa, prevents the corresponding dispensing unit from connecting to the other drug delivery devices.

Thereby, Fig. 36 shows a longitudinal cut through a first dispensing unit 420 attachable to a first housing 221 of a first drug delivery device 220 via first connection means 424 of a first cartridge holder 422, a longitudinal cut through a second dispensing unit 430 attachable to a second housing 231 of a second drug delivery device 232 via second connection means 434 of a second cartridge holder 432 of the second dispensing unit 430 and a longitudinal cut through a third dispensing unit 440 attachable to a third housing 226 of a third drug delivery device 225 via third connection means 444 of a third cartridge holder 442 of the third dispensing unit 440. Fig. 37 shows side views and perspective views of first connection means 511 of the first housing 221 of the first drug delivery device 220, of second connection means 520 of the second housing 223 of the second drug delivery device 223 and of third connection means 530 of the third housing 226 of the third drug delivery device 225.

The connection means 424, 434, 444 of the cartridge holders 422, 432, 442 and the corresponding connection means 511, 520, 530 of the drug delivery devices 220, 222, 225 form keyed connectors according to the present disclosure.

Thereby, the connection means 424, 434, 444 are of the same type and the connection means 511, 520, 530 are also of the same type.

The individual connection means 424, 434, 444 of the cartridge holders 422, 432, 442 each form female parts of the connections and the individual connection means 511, 520, 530 of the drug delivery devices 220, 222, 225 form corresponding male parts. All connection means 424, 434, 444, 511, 520, 530 are configured as threads, whereby the connection means 424, 434, 444 of the cartridge holders 422, 422, 432 form inner threads and the connection means 511, 520, 530 of the drug delivery devices 220, 222, 225 form outer threads.

The geometries of the threads 424, 434, 444, 511, 520, 530 are defined by several thread dimensions. The thread dimensions comprise a core diameter or minor diameter that specifies the minimum inner diameter of the female part of the connections, an outer diameter or major diameter that specifies the maximum inner diameter of the female part of the connections, a pitch that specifies a distance between adjacent ridges 501 or valleys 502 of the threads, a width of the ridges 501 provided on the male part of the threads, which corresponds to a width of the valleys 502 provided on the female part of the threads and an opening angle between sidewalls of adjacent ridges 501 of the male parts. A height of the ridges 501 of the male parts and a corresponding height of the valleys 502 of the female parts is given by the difference between the outer diameter and the core diameter.

Unless stated otherwise, the term "ridges" used in the present disclosure always refers to the ridges 501 of the male thread of a given threaded connection, irrespective of whether the part being described actually comprises a male thread or a female thread.

Keying is achieved by at least one of the thread dimensions, such as at least one of the core diameter, the outer diameter, the pitch, the width of the ridges 501 and the opening angle, being mutually different among the individual pairs of corresponding connection means 424, 434, 444, 511, 520, 530, of the cartridge holders 422, 432, 442 and drug delivery devices 220, 222, 225.

With the embodiments shown in Fig. 36 and Fig. 37, the only thread dimensions that differ among the individual dispensing units 420, 430, 440 and therefore also among the individual drug delivery devices 220, 222, 225 are the width and the height of the individual ridges 501 of the male parts and the corresponding widths and heights of the valleys 502 of the female parts. Thereby, the ridges 501 of the first connection means 511 have a first width W₁, the ridges 501 of the second connection means 520 have a second width W₂, and the ridges 501 of the third connection means 530 have a third width W₃. The first width W₁ is smaller than the second width W₂, and the second width W₂ is smaller than the third width W₃. Exemplarily, the second width W₂ is twice the first width W₁ and the third width W₃ is three times the first width wi.

Furthermore, the ridges 501 of the first connection means 510 have a first height hi, the ridges 501 of the second connection means 520 have a second height h₂, and the ridges 501 of the third connection means 530 have a third height h₃. The first height h₁ is larger than the second height h₂, and the second height h₂ is larger than the third height h₃. Thereby, the first height h₁ is twice the second height h₂ and the first height h₁ is three times the third height h₃.

The aforementioned differences in the heights h₁, h₂, h₃, combined with the aforementioned differences in the widths W₁, W₂, W₃ reliably prevent mounting of the individual dispensing units 420, 430, 440 to other than their corresponding drug delivery device 220, 222, 225 with the matching connection means 511, 520, 530.

The different heights h₁, h₂, h₃ result from different outer diameters with a first outer diameter D₁ of the first connection means 424, 520 being larger than a second outer diameter D₂ of the second connection means 434, 520 and the second outer diameter D₂ of the second connection means 434, 520 being larger than a third outer diameter D₃ of the third connection means 444, 530. The first connection means 424, 511 have a first core diameter CD₁, the second connection means 434, 520 have a second core diameter CD₂, and the third connection means 444, 530 have a third core diameter CD₃ and all core diameters CD₁, CD₂, CD₃ are equal.

With other embodiments, the different heights h₁, h₂, h₃ may also result from differing core diameters CD₁, CD₂, CD₃ and, optionally, also differing outer diameters D₁, D₂, D₃. According to still another embodiment, the core diameters CD₁, CD₂, CD₃ could be chosen to be mutually identical and also the outer diameters D₁, D₂, D₃ could be chosen to be mutually identical for all connections such that all devices 220, 222, 225 comprise threads 511, 520, 530 with ridges 501 of the same height.

With the embodiments shown in Fig. 36 and Fig. 37, a first pitch P₁ of the first connection means 424, 511, a second pitch P₂ of the second connection means 434, 520 and a third pitch P₃ of the third connection means 444, 530 are the same. Furthermore, a first angle A₁ of the first connection means 424, 511, a second angle A₂ of the second connection means 434, 520 and a third angle A₃ of the third connection means 444, 530 are also the same.

With the exemplary embodiments shown in Fig. 36 and Fig. 37, the individual thread dimensions are the following: CD₁=CD₂=CD₃=12.60 mm, D₁=14.70 mm, D₂=14.00 mm, D₃=13.30 mm, h₁=2.10 mm, h₂=1.40 mm, h₃=0.70 mm, W₁=0.65 mm, W₂=1.30 mm, W₃=1.95 mm, A₁=A₂=A₃=60°.

In one embodiment, the first, second and third drug delivery device 220, 222, 225 each are a variant of the drug delivery device 200 disclosed in connection with Fig. 1 to Fig. 35. As far as no differences are described or apparent from the Figures, the first, second and third drug delivery device 220, 222, 225 are then configured as it is disclosed in connection with the drug delivery device 200 and vice versa. Furthermore, the first, second and third dispensing unit 420, 430, 440 each are a variant of the dispensing unit 410 disclosed in connection with Fig. 1 to Fig. 35. As far as no differences are described or apparent from the Figures, the first, second and third dispensing unit 420, 430, 440 are then configured as it is disclosed in connection with the dispensing unit 410 and vice versa.

The second drug delivery device 222 and the first drug delivery device 220 share at least one mutual member that is identical among the first and second drug delivery device 220, 222 and the third drug delivery device 225 and the first drug delivery device 220 share at least one further mutual member that is identical among the first and third drug delivery device 220, 225. Thereby, the mutual member and the further mutual member are identical. With other embodiments, the mutual member and the further mutual member may also be different. Mutual members are thereby both mechanically identical, that is identical in shape, and identical in their appearance, such as in their color and printing.

The second drug delivery device 222 and the first drug delivery device 220 each comprise at least one distinguishing member that is different among the first and second drug delivery device 220, 222 and the third drug delivery device 225 and the first drug delivery device 220 each comprise at least one further distinguishing member that is different among the first and third drug delivery device 220, 225. Thereby, the distinguishing member and the further distinguishing member are the same functional member and therefore perform the same function during use of the dosing mechanism. With other embodiments, the mutual member and the further mutual member may also be different functional members.

Distinguishing members are at least different in their appearance, such as color and printing. Additionally, they may also be mechanically different, that is they may be different in shape. Despite being different in appearance and, optionally shape, the individual distinguishing members perform the same function during dose setting and dose delivery and thus constitute the same functional member among the individual drug delivery devices 220, 222, 225. The individual distinguishing elements are therefore designated by the same term in all drug delivery devices 200, 220, 222, 225.

The functional members constitute the individual parts of which the drug delivery devices 220, 225, 225 are assembled. While the individual parts may differ in their exact shape and appearance, for example to provide different dose increments among the individual drug delivery devices 220, 225, 225, they perform the same function and are located at the same positions within the dosing mechanisms 230 of the individual drug delivery devices 220, 225, 225. Furthermore, they interact and engage with the same further functional members of the dosing mechanism 230 among all drug delivery devices 220, 225, 225 of the set. Functional members may be composed of several sub-parts that are rigidly connected to each other to form a single mechanical part. With one embodiment of the present disclosure, a dosing member may, for example, constitute a functional member that is composed of two sub-parts, namely a dose sleeve and a snap element.

The first and second drug delivery device 220, 222 form a first set of drug delivery devices that mechanically differ only by their outer housings 221, 223, which carry the keyed connection means 510, 520. All other functional members of the drug delivery devices 220, 222 of the first set are mechanically identical. Therefore, the dosing mechanisms 240 and the dose definition mechanisms 232 of the two drug delivery devices 220, 222 are also the same. The two drug delivery devices 220, 222 are therefore configured to define identical rotational dose positions of the dose setting member 290 and to expel the same amount of liquid per settable dose increment.

One of the first set of drug delivery devices 220, 222 is configured to be used with its corresponding dispensing unit 420, 430 containing a drug having an active pharmaceutical ingredient in a first concentration and the other one of the first set of drug delivery devices 220, 222 is configured to be used with its corresponding dispensing unit 420, 430 containing the drug having the active pharmaceutical ingredient in a second concentration that is different from the first concentration.

Among the drug delivery devices 220, 222 of the first set, the piston rods 240, the plunger discs 242, the drivers 350, the nuts 250, the dose setting members 290, the first and second bearing elements 370, 380, the biasing members 308, the inner housings 180 and all elements of the resetting mechanism 110, namely the resetting elements 110, the coupling parts 130 and the biasing members 150, each form mutual members that are mutually identical both in appearance and shape among the two drug delivery devices 220, 222.

The dosing members 330 of the two drug delivery devices 220, 222 of the first set form a distinguishing member that differs in appearance but not in shape among the two drug delivery devices 220, 222. The difference in appearance thereby includes different numerals of the visual indicators 331, whereby the individual indicators 331 are located at the same positions on the dosing members 330 of the respective two drug delivery devices 220, 222.

The outer housings 211 of the two drug delivery devices 220, 222 of the first set form distinguishing elements that differ in shape due to the differences of their connection means 511, 520. Furthermore, the outer housings 211 differ in appearance, such as in color and/or labelling, to allow a user to clearly distinguish between the two devices 220, 222.

The dose selector members 310 and the caps 209 of the two drug delivery devices 220, 222 of the first set also form distinguishing members that differ in appearance but not in shape among the two drug delivery devices 220, 222. The difference in appearance thereby include different labelling on the dose selector member 310 and the cap 209. Furthermore, the caps 209 differ in color to match the colors of the respective body of their drug delivery device 220, 222. With other embodiments, the dose selector member 310 and/or the caps 209 could also be configured as mutual members. Furthermore, the caps 209 could also differ only in color and not in labelling or vice versa.

Each one of the first and second drug delivery device 220, 222 forms together with the third drug delivery device 225 a second set of drug delivery devices 200, 220, 225 that mechanically differ not only by their outer housings 211 but also by functional members of their dose definition mechanisms 232.

The dosing mechanism 230 of the third drug delivery device 225 is configured to provide a dialing resolution that is different from the dialing resolution of the first and second drug delivery device 220, 222. While the dosing mechanisms 230 of the first and second drug delivery device 220, 222 comprises the dose selector member 310 and the dose setting member 290 described in connection with Fig. 1 to Fig. 35, which are configured to define 27 settable dose positions, the third drug delivery device 225 comprises embodiments of the dose selector member 310 and the dose setting member 290 that are configured to define 18 settable dose positions.

The dose selector member 310 of the third drug delivery device 225 comprises 18 functional features 312 that are distributed around its inner surface. A position of the elastic elements 292 of the dose setting member 290 is thereby adapted to the larger distance between the individual functional features 312 to allow for reliable engagement between the elastic elements 292 and the functional features 312.

Since the dose definition mechanisms 332 of the third drug delivery device 225 define an even number of settable doses, the connection 277 between the clutch member 270 and the dose setting member 290 is configured to connect the clutch member 270 and the dose setting member 290 in two different relative rotational orientations that differ from each other by 180°. To achieve this, the first and second longitudinal grooves 297 and 298 of the dose setting member 290 and the corresponding first and second ridge 279, 280 of the clutch member 270 each have the same widths.

The clutch member 270 of the third drug delivery device 225 comprises 18 clutch elements 278, the circumferential positions of which are adapted to the circumferential positions of the functional features 312 of the dose selector member 310. Therefore, the number and circumferential positions of the clutch elements 278 of the clutch member 270 of the third drug delivery device 225 differs from the number and circumferential positions of the clutch elements 278 of the clutch member 270 of the first and second drug delivery device 220, 222.

The clutch members 270 of, on the one hand, the first and second drug delivery devices 220, 222 and of, on the other hand, the third drug delivery device 225 form distinguishing members that differ in shape among the second sets of drug delivery devices 220, 222 225. Likewise, the dose setting member 290 of, on the one hand, the first and second drug delivery devices 220, 222 and of, on the other hand, the third drug delivery device 225 also form distinguishing members that differ in shape among the second sets of drug delivery devices 220, 222 225.

The dosing member 330 of the third drug delivery device 225 comprises 18 clutch elements 336, the circumferential positions of which are adapted to the circumferential positions of the clutch elements 273 of the clutch member 270. Therefore, the dosing member 330 of the third drug delivery device 225 and each one of the dosing members 330 of the first and second drug delivery device 220, 222 form distinguishing members that differ in shape among the second sets of drug delivery devices 220, 222 225.

With all drug delivery devices 200, 220, 222, 225, the clutch elements 273 of the clutch member 270, the clutch elements 336 of the dosing member 330, the clutch elements 312 of the dose selector member 310 and the clutch elements 294 of the dose setting member 290 are rotationally aligned with respect to each other in a way that in each rotational position of the dose setting member 290, in which the clutch elements 273 of the clutch member 270 and the clutch members 336 of the dosing member 360 are aligned with each other to allow mutual engagement, also the clutch elements 294 of the dose setting member 290 and the clutch elements 312 of the dose selector member 310 are aligned with each other to allow mutual engagement.

The dosing member 330 of the third drug delivery device 225 furthermore differs from the dosing member 330 of the first and second drug delivery device 220, 222 in appearance, as the positions of the optical markers 331 on the dosing member 330 of the third drug delivery device 225 differ from the positions of the optical markers 331 on the dosing members 330 of the first and second drug delivery devices 220, 222 to reflect the different number of doses settable per revolution of the dose setting member 290.

The numbering of the individual optical markers 331 on the dosing member 330 of the first drug delivery device 220 differs from the numbering of the individual optical markers 331 on the dosing member 330 of the third drug delivery device 225.

This allows the first drug delivery device 220 to be used with a drug that has a first concentration of an active pharmaceutical ingredient and the third drug delivery device 225 to be used with a drug having a third concentration of an active pharmaceutical ingredient, whereby the product of the first concentration with the amount of liquid that is expelled by the first drug delivery device 220 per dose increment differs from the product of the third concentration with the amount of liquid that is expelled by the third drug delivery device 220 per dose increment.

The numbering of the individual optical markers 331 on the dosing member 330 of the second drug delivery device 222 equals the numbering of the individual optical markers 331 on the dosing member 330 of the second drug delivery device 222. This allows the second drug delivery device 220 to be used with a drug that has a second concentration of an active pharmaceutical ingredient and the third drug delivery device 225 to be used with a drug having a third concentration of an active pharmaceutical ingredient, whereby the product of the second concentration with the amount of liquid that is expelled by the second drug delivery device 222 per dose increment differs from the product of the third concentration with the amount of liquid that is expelled by the third drug delivery device 220 per dose increment.

Due to the differences in shape and appearance, the dosing member 330 constitutes a distinguishing member among the second sets of drug delivery devices 220, 222, 225.

In total, mutual members of the second sets of drug delivery devices 220, 222, 225 are the piston rod 240, the plunger disc 242, the nut 250, the driver 350, the bearing elements 370, 380, the biasing member 308, the inner housing 180 and all elements of the resetting mechanism 110, namely the resetting element 110, the coupling part 130 and the biasing member 150.

Distinguishing members that only differ in appearance but not in shape among the second sets of drug delivery devices 220, 222, 225 are the caps 209, each of which has a different color. Distinguishing members that differ both in appearance and in shape among the second sets of drug delivery devices 220, 222, 225 are the outer housings 211, each of which has a different color and a differently shaped connection means 511, 520, 530, the dosing members 330, the dose selector members 310, each of which has a different labelling and differently shaped functional features 312, the clutch members 270, which differ in the shapes of their clutch elements 273 and hence also in their appearance, and the dose setting members 290, which differ in the positions of their elastic elements 292 and their clutch elements 294 and hence also in their appearance.

The first drug delivery device 220 is configured to be used with a drug containing the active pharmaceutical ingredient in a concentration of 5 mg / 1.5 ml, the second drug delivery device 222 is configured to be used with drug containing the active pharmaceutical ingredient in a concentration of 10 mg / 1.5 ml and the third drug delivery device 225 is configured to be used with a drug containing the active pharmaceutical ingredient in a concentration of 15 mg / 1.5 ml. Both the first and second drug delivery device 220, 222 have a dialing resolution of 0.015 ml per dose increment and the third drug delivery device 225 has a dialing resolution of 0.010 ml per dose increment.

The optical markers 331 on the dosing member 330 of the first drug delivery device 220 then display dose increments of 0.05 mg and the optical markers 331 on the dosing members 330 of the second and third drug delivery device 222, 225 then each display dose increments of 0.10 mg. All drug delivery devices 220, 222, 225 allow for two full rotations of the dose setting member 290 during dose setting. With 27 dose increments per revolution of the dose setting member 290, the first drug delivery device 220 is configured to expel a maximum dose of the active pharmaceutical ingredient of 1.80 mg and the second drug delivery device 222 is configured to expel a maximum dose of the active pharmaceutical ingredient of 3.60 mg. Since the third drug delivery device 225 provides 18 dose increments per revolution of the dose setting member 290, it is configured to deliver a maximum dose of the active pharmaceutical ingredient of 5.40 mg.

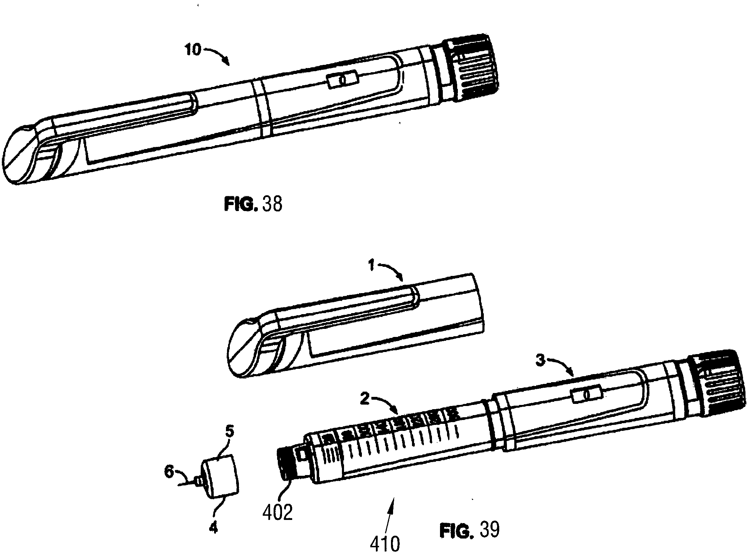

The keyed connectors according to the present disclosure are also applicable with other drug delivery devices, for example, injection devices. A further possible injection device is the pen-type, further drug delivery device 10 illustrated in **Fig. 38 to Fig. 40**. As far as no differences are described or apparent from the Figures, the further drug delivery device 10 is configured as it is disclosed in connection with the drug delivery device 200 and vice versa. The further drug delivery device 10 is also described in more detail in international applications WO2020/015980A1 and WO2019/011394A1, the disclosure of each of which is incorporated into this disclosure in its respective entirety by reference.

The further drug delivery device 10 has an outer housing 3 connected a dispensing unit 410 with a cartridge holder 2 holding a cartridge 8. The cartridge holder 2 has a needle connector 402. The injection device 10 has a dosing mechanism 30 and is illustrated in the zero-dose state as indicated by an optical marker 40 showing a zero through a window 3a of the outer housing 3. The outer housing 3 terminates at its proximal end in a keyed connection means 510, which has a thread form.

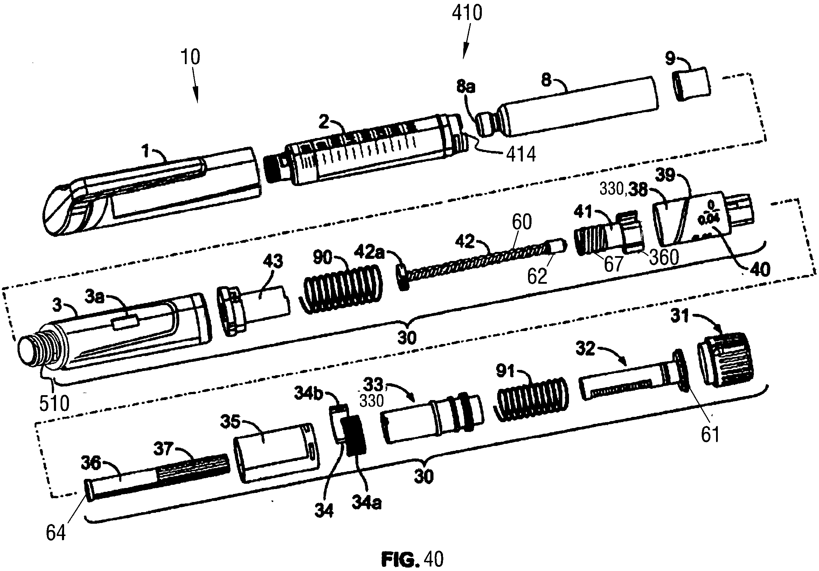

Fig. 40 schematically shows a simplified exploded view of the device 10 with a cap 1 removed to expose the cartridge holder 2 and the proximal needle connector 402. The pen needle 4 is typically attached to the needle connector 402 through a snap fit, thread, Luer-Lok, or other secure attachment with hub 5 such that a double ended needle cannula 6 can achieve a fluid communication with a drug contained in the cartridge 8 positioned within cartridge holder 2.

The particular design of the device 10 allows for setting of one or more of predetermined fixed doses through the interaction of a snap element 33 with a dose selector member 35. A rotation of a dose setting member 31 and the snap element 33 occurs during dose setting and is relative to outer housing 3. During the initiation of the dose delivery procedure the dose setting member 31 is pressed in the proximal direction causing it and the dose selector member 35 to move axially relative to the snap element 33. Like with the drug delivery device 200, the dose selector member 35 is axially movable and rotationally fixed with respect to the outer housing 3 of the further drug delivery device 10.

Part of the dosing mechanisms of most pen-type injectors, including device 10, is a piston rod 42 as illustrated in Fig. 3. Such piston rods usually have a non-circular cross-section and have two flat surfaces that are designed to prevent the piston rod 42 from rotating with respect to the outer housing 3 but allowing it to move linearly in the proximal direction. A nut 36 and a clutch member 32 are permanently splined to each other during assembly of the dosing mechanism 30 through a splined connection 37. The splined connection 37 ensures that the clutch member 32 and the nut 36 are always rotationally fixed to each other during both dose setting and dose delivery. This splined connection 37 also allows the clutch member 32 and the nut 36 to move axially relative to each other during both dose setting and dose delivery.

The proximal end of the nut 36 has an internal thread that matches a corresponding outer thread 60 of the piston rod 42. The distal end of the clutch member 32 is configured as a dose button 61 and is permanently attached to the distal end of the dose setting member 31 through engagement of connectors, which may be configured as snap locks, an adhesive and/or a sonic weld. This connection ensures that the clutch member 32 is both rotationally and axially fixed to the dose setting member 31 during both dose setting and dose delivery. Alternatively, the clutch member 32 and the dose setting member 31 could also be configured as a single member.

At the terminal proximal end of the piston rod 42 is a connector, which is configured as a snap fit, that connects with a plunger disc or foot 42a. At the distal end of the piston rod 42 is a stop feature 63 of the dosing mechanism 30, illustrated as an enlarged section. This enlarged section 63 is designed to stop the rotation of the nut 36 about the thread 60 when the amount of medicament remaining in the cartridge 8 is less than the next highest predetermined dose setting. In other words, if the user tries to set one of the predetermined fixed dose settings that exceeds the amount of medicament remaining in the cartridge 8, then the enlarged section 63 will act as a hard stop preventing the nut 36 from further rotation along the thread 60 as the user attempts to reach the desired predetermined fixed dose setting. With the drug delivery device 200, the stop feature 243 interacts with the nut 250 in the same way and therefore also prevents setting of a dose larger than the remaining dose within the cartridge 8.

The piston rod 42 is held in a non-rotational state relative to the outer housing 3 during both dose setting and dose delivery by a piston rod guide 43. The piston rod guide 43 is both rotationally and axially fixed to the outer housing 3. This fixation can be achieved when the piston rod guide 43 is a separate component from the outer housing 3 as illustrated or the piston rod guide 43 could be made integral with the outer housing 3, analogous to the inner sleeve 183 of the inner housing 180 of the drug delivery device 200. Also not shown in the Figures, the piston rod guide 43 is configured as a resetting mechanism that, like the resetting mechanism 100 of the drug delivery device 200, prevents rotation of the piston rod 42 with respect to the housing 3 during attachment of the dispensing unit 410 to the housing 3 of the drug delivery device 10 and that allows rotational movement of the piston rod 42 with respect to the housing 3 upon disengagement of the dispensing unit 410.

The piston rod guide 43 also engages the proximal end of a rotational biasing member 90, shown as a torsion spring, the function of which will be explained below. This connection of the rotational biasing member 90 to the piston rod guide 43 anchors one end of the rotational biasing member 90 in a rotationally fixed position relative to the outer housing 3.

The distal end of the rotational biasing member 90 is connected to a driver 41. The driver 41 is connected to and rotationally fixed with respect to an inner surface of a dosing member 330 through a splined connection on the distal outer surface of the driver 41. This splined connection comprises at least one, such as two longitudinal ridges that are located on the outer diameter of the driver 41 and that engage with corresponding grooves on the inner surface of the dosing member 330. On the proximal end of the driver 41 on the outer surface is a thread 67 that is engaged with a matching thread on the inner distal surface of the piston rod guide 43.

The dosing member 330 comprises two parts that are rotationally and axially fixed to each other, for example by a snap-fit connection. One part forms a dose sleeve 38 that is connected to the driver 41 through the splined connection, the other part forms the snap element 33. As such, the dosing member 330 forms a single functional member.

The dosing member 330, namely the dose sleeve 38 is threadedly engaged with the body 3 by a helical groove 39 located on the outer surface of the dosing member 330 that engages with a corresponding helical ridge located on the inner surface of the body 3. The thread between the driver 41 and the piston guide 43 has a significantly different pitch than the thread between the dosing member 330 and the outer housing 3. The axially sliding connection between the nut 36 and the clutch member 32 allows to compensate for the differences in the pitch of the thread between the inner surface of the nut 36 and the outer surface of the piston rod 42 and the pitch of the thread between the dosing member 330 and the body 3. The thread between the driver 41 and the piston guide 43 has basically the same pitch as the thread between the piston rod 42 and the nut 36.

The nut 36 and the driver 41 rotate together both during dose setting and dose cancellation and, as such, they perform essentially the same axial movement. However, these movements are independent from each other, i. e., the nut 36 is turned by the clutch member 32 and performs an axial movement due to the thread to the piston rod 42, while the driver 41 is rotated by the dosing member 330 and performs an axial movement due to the thread to the piston guide 43. The driver 41 is rotating during injection also, and so it actively moves in the proximal direction during injection. But, the nut 36 does not rotate during injection and as such does not perform an active axial movement. The nut 36 is only moving in the proximal direction during injection because it is being pushed axially by the driver 41, which surrounds the nut 36 and abuts against a protrusion 64 located at the proximal end of the nut 36. The rotating driver 41 pushing the non-rotating nut 36 causes the injection because the piston rod 42 is pushed forward due to the threaded engagement with the nut 36.

Because the torsion spring 90 is attached to the driver 41 and the driver 41 is rotationally fixed to the dosing member 330, rotation of the dosing member 330 in a first direction during dose setting will wind the torsion spring 90 such that it exerts a counter rotational force on the dosing member 330 in an opposite second direction. This counter rotational force biases the dosing member 330 to rotate in a dose canceling direction.

The function of the complete further drug delivery device 10 and the dosing mechanism 30 will now be described. The further drug delivery device 10 is provided to a user as reusable or semi-reusable device. A semi-reusable means that only the dosing mechanism 30 housed in outer housing 3 is reused each time a new dispensing unit having a cartridge holder 2 containing a new cartridge 8 of medicament is connected to the outer housing 3. A reusable device would allow reattachment of an old or previously used cartridge holder 2 where the user has inserted a new full cartridge 8 of medicament. In one configuration according to the present disclosure, the device 10 has the semi-reusable design where each time the medicament in the cartridge 8 is expelled or emptied, the user would be required to disconnect the cartridge holder 2 containing the empty cartridge 8 that is not removable from the cartridge holder 2. As such, the user would dispose of both the cartridge holder 2 and the empty cartridge 8 together. A new cartridge holder 2 and cartridge 8 assembly would be connected to the outer housing 3 provided that the keyed connection means 510 on the outer housing 3 matches a keyed connection means 414 provided on the distal end of the cartridge holder 2.

With the further drug delivery device 10, the dose sleeve 38 and the snap element 33 are axially and rotationally fixed with each other via a snap-fit connection. Therefore, the dose sleeve 38 and the snap element 33 constitute a single functional element, namely the dosing member 330. With other embodiments of the further drug delivery device 10, the dosing member 330 could also be configured as a single component or member.

A housing of the further drug delivery device 10 comprises the outer housing 3 and the piston guide 43, which are rotationally and axially fixed with respect to each other.

Like the drug delivery device 200, the further drug delivery device 10 comprises a clutch mechanism 237. During dose setting, the clutch mechanism 237 rotationally fixes the nut 36 with respect to the driver 41 and the dosing member 330 and allows rotation of the nut 36 with respect to the housing 3, 43. During dose delivery, the clutch mechanism 237 rotationally fixes the nut 36 with respect to the dose selector member 35 and the housing 3, 43 and allows relative rotation between the nut 36 on the one hand and the driver 41 and the dosing member 330 on the other hand.

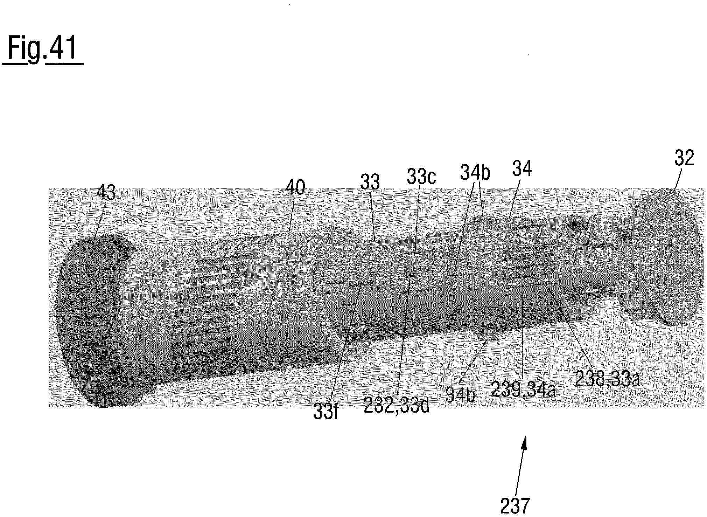

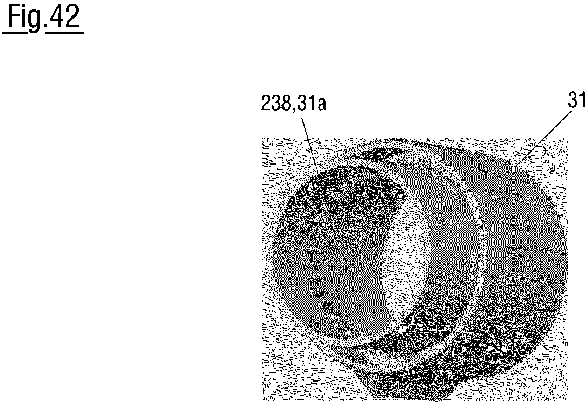

As can be seen from Fig. 41 and Fig. 42, a first part 238 of the clutch mechanism 237 comprises clutch elements 33a that are configured as radially extending teeth and that are provided on an outer surface at a distal end of the snap element 33 of the dosing member 330. A second part 239 of the clutch mechanism 237 comprises clutch elements 34a that are configured as radially extending teeth and that are provided on an outer surface at a distal end of a connector 34.

The connector 34 is located within an annular recess of the dosing member 330 and is thereby rotationally movable and axially fixed with respect to the dosing member 330. The connector 34 is axially movable and rotationally fixed with respect to the dose selector member 35. This is exemplarily achieved by radially protruding ridges 34b of the connector 34b that are received in corresponding longitudinal grooves on an inner surface of the dose selector member 35. The rotationally fixed connection to the dose selector member 35 also rotationally fixes the connector 34 to the housing 3, 34 of the further drug delivery device 10.

The dosing member 330 surrounds the clutch member 32 and the clutch member 32, together with the dose setting member 31 and the dose selector member 35, is axially movable with respect to the dosing member 330. Thereby, the dose setting member 31 and the clutch member 32 are biased into the distal direction by a compression spring 91 (shown in Fig. 40) that acts between the dosing member 330 and the clutch member 32. Axial movement of the clutch member 32 and the nut 31 is allowed until the dose setting member 31 abuts the dosing member 330 via the dose selector member 35.

During dose setting, the clutch member 32 and the nut 31 are in their distal position with respect to the dosing member 330. In this position, the dose setting member 31 is rotationally coupled to the dosing member 330 via the first part 238 of the clutch mechanism 237 that comprises the clutch elements 33a at the distal end of the snap element 33 of the dosing member 330 and corresponding clutch elements 31a on an inner surface of the dose setting member 31, which are shown in Fig. 42. When rotating the dose setting member 31 during dose setting, the dosing member 330 is also rotated via the closed first part 238 of the clutch mechanism 237 between the dose setting member 31 and the dosing member 330 and screwed out of the outer housing 3. This forces the dose selector member 35 and the dose setting member 31 to also move in the distal direction. Rotation of the dosing member 330 also forces a corresponding rotation of the driver 41, which is therefore also screwed out of the piston guide 43.

Since the nut 36 is rotationally fixed with respect to the clutch member 32, rotation of the dose setting member 31 also causes rotation of the nut 36 during dose setting. Thereby, the nut 36 is screwed along the piston rod 42 and also moves into the distal direction. The pitches of the threads of the piston rod 42 and the driver 41 are adapted so that the nut 36 and the driver 41 essentially move the same axial distance upon rotation. Thereby, the nominal pitch of the connection between the driver 41 and the piston guide 43 is slightly higher than the nominal pitch of the thread between the piston rod 42 and the nut 36 to prevent mutual blocking of the nut 36 and driver 41 irrespective of manufacturing tolerances.

To eject a set dose, the dose setting member 31, the clutch member 33 and the dose selector member 35 are moved into their proximal position with respect to the dosing member 330. This releases the first part 238 of the clutch mechanism 237 between the snap element 33 of the dosing member 330 and the dose setting member 31 and engages the second part 239 of the clutch mechanism 237, which is realized between the dose setting member 31 and the connector 34 that surrounds the dosing member 330. Upon engagement of the second part 239 of the clutch mechanism in 237, the clutch elements 31a of the dose setting member 31 engage with the clutch elements 34a of the connector 34.

Engagement of the second part 239 of the clutch mechanism 237 rotationally locks the dose setting member 31 and the clutch member 32 to the connector 34 and, via the ridge 34a of the connector 34, also to the dose selector member 35 and the housing 3, 43. Therefore, the nut 36 is rotationally locked with respect to the housing 3, 43 and the piston rod 42 during dose delivery. This locking is achieved via the nut 36, the clutch member 32, the dose setting member 31, the connector 34 and the dose selector member 35.

Disengagement of the first part 238 of the clutch mechanism 237 allows rotational movement between the nut 36 and the driver 41 during dose delivery.

When further pushing the dose setting member 31 into the proximal direction, the dose selector member 35 abuts against the dosing member 330 and forces the dosing member 330 to move into the proximal direction. Due to the threaded connection between the dosing member 330 and the outer housing 3, the dosing member 330 rotates when moving into the proximal direction. This rotation is transferred to the driver 41, which is screwed into the proximal direction into the piston guide 43 and therefore also moves axially in the proximal direction. The driver 41 thereby abuts and advances the nut 36, which is now rotationally fixed to the outer housing 3 and the piston rod 42 via the clutch member 32, the dose setting member 31, the connector 34 and the dose selector member 35. Therefore, both the piston rod 42 and the nut 36 are rotationally fixed with respect to each other and axial advancement of the nut 36 causes a corresponding axial advancement of the piston rod 42, thus expelling the set dose.

The further drug delivery device 10 comprises a dose definition mechanism 232 that acts between the snap element 33 of the dosing member 330 and the dose selector member 35. During dose setting, the dosing member 330 rotates with respect to the dose selector member 35. On its outer surface, the dosing member 330 has a flexible arm 33c with a radial protrusion 33d, which forms an elastic element and engages with dose stops 35a on the inner surface of the dose selector 35. The dose stops 35 form functional features of the dose definition mechanism 232.

The circumferential positions of the individual dose stops 35a thereby define individual relative rotational positions between the dose setting member 31 and the housing 3, 43 that correspond to settable doses. To prevent the dialing of intermediate doses in between the individual dose stops 35a, the torsion spring 90 is provided between the piston guide 43 and the driver 41. This torsion spring 90 is loaded when increasing the set dose and causes the dosing member 330 to rotate back to the last set dose in cases where the dose setting member 31 is released while the protrusion 33d on the dosing member 330 is positioned in between two dose stops 35a.

With the further drug delivery device 10, the dose setting member 31 is limited to perform less than one full rotation upon dose setting. The further drug delivery device 10 comprises a stop mechanism that defines a maximum and minimum rotational position of the dose setting member 31 during dose setting.

The stop mechanism acts between the snap element 33 of the dosing member 330 and the dose selector member 35. It comprises a further protrusion 33f that is located on the outer surface of the dosing member 330 and that radially protrudes towards the dose selector member 35. The dose selector member 35 comprises a maximum stop feature 35b that is located on an inner surface of the dose selector member 33 and that is configured as a side surface of a step located on the inner surface. Furthermore, the dose selector member 35 comprises a zero stop feature 35c that is located also on the inner surface of the dose selector member 33. The zero stop feature 35c is exemplarily configured as an opposing side surface of the step.

The further protrusion 33f of the dosing member 330 is configured to abut the maximum stop feature 35b upon rotation of the dose setting member 31 into a rotational position that corresponds to or exceeds a maximum settable dose and thereby prevents further rotation of the dose setting member 31. Likewise, the further protrusion 33f of the dosing member 330 is configured to abut the zero stop feature 35c upon rotation of the dose setting member 31 into a rotational position that corresponds to a zero dose setting and thereby prevents further rotation of the dose setting member 31.

Like the dose delivery device 200, the further dose delivery device 10 may be provided in several variants that are distinguished by their connection means 510 to be configured to only connect to a dedicated variant of the dispensing unit 410. The connection means 510 may thereby be configured as it is disclosed in connection with Fig. 36 and Fig. 37.

In one embodiment, the several variants of the further drug delivery device 10 comprises as distinguishing members the outer housing 3, the cap 1, the dose sleeve 38 and the dose selector member 35. The outer housings 3 differ in shape due to the differences in the connection means 510 and also in appearance due to different colors and/or labeling. The dose selector members 35 differ in shape due to different numbers and/or different positions of the dose stops 35a, which allows to realize different dialing resolutions or settable doses. Alternatively or additionally, the dose selector members 35 may also differ in the position of the maximum stop feature 35c. The dose sleeves 38 are mechanically identical among the individual variance but differ in appearance due to different positions and/or numbering of their optical markers. The caps 1 are identical in shape but differ in their appearance, like color and/or labelling. With other embodiments, the caps 1 could also be configured as mutual members.

Mutual members of the variants of the further drug delivery devices 10 then may be all other elements of the dosing mechanism 30.

The invention is further described with reference to the following embodiments.
1. Set of at least two drug delivery devices comprising at least a first injection device and a second injection device, each one of the first and second injection device comprising
   a housing,
   a dosing mechanism within said housing comprising
   a dose setting member for setting an injection dose,
   a dose indication member for visual indication of the set dose, and
   a piston rod for ejecting the set dose from the cartridge attached to the housing
   wherein the dosing mechanisms of the first drug delivery device and the second drug delivery device each comprise a first set of mutual members that are mechanically identical in both the first drug delivery device and the second drug delivery device, the first set comprising at least one mutual member,
   wherein the dosing mechanisms of the first drug delivery device and the second drug delivery device each comprise a second set of distinguishing members that differ among the first and second drug delivery device, the second set comprising at least one distinguishing member,
   wherein the first drug delivery device comprises first connection means for attaching a first cartridge to the housing of the first drug delivery device and the second drug delivery device comprises second connection means for attaching a second cartridge to the housing of the second drug delivery device,
   wherein the first and second connection means differ from each other so that the second cartridge is not attachable to the first connecting means and the first cartridge is not attachable to the second connecting means.
2. The set of at least two drug delivery devices according to embodiment 1, wherein the dosing mechanisms of the first drug delivery device and the second delivery device each comprise, for example consist of, a same set of functional members that includes the first set and the second set.
3. The set of at least two drug delivery devices according to embodiment 2, wherein all functional members of the dose definition mechanism of the first drug delivery device are mechanically identical to the functional members of the dosing mechanism of the second drug delivery device.
4. The set of at least two drug delivery devices according to one of the preceding embodiments,
   wherein the first connection means and the second connection means both comprise a female part or a male part.
5. The set of at least two drug delivery devices according to one of the preceding embodiments,
   wherein the first connection means and/or the second connection means are designed as threads.
6. The set of at least two drug delivery devices according to embodiment 5, wherein the thread of the first connection means has a pitch that is identical to the pitch of the thread of the second connection means.
7. The set of at least two drug delivery devices according to embodiment 5 or 6,
   wherein ridges of the thread of the first connection means have different dimensions than ridges of the second connection means.
8. The set of at least two drug delivery devices according to embodiment 7, wherein the ridges of the thread of the first connection means have a width that differs from a width of the ridges of the thread of the second connection means.
9. The set of at least two drug delivery devices according to embodiment 7 or 8,
   wherein the ridges of the thread of the first connection means have a height that differs from a height of the ridges of the second connection means.
10. The set of at least two drug delivery devices according to one of embodiments 5 to 9,
   wherein the threads of the first and second connection means have the same minor diameter and/or the same major diameter.
11. The set of at least two drug delivery devices according to any one of the preceding embodiments,
   wherein the dosing mechanisms each comprise a dose definition mechanism that acts between the dose setting member and the housing and wherein the dose definition mechanisms are configured to define dose positions that are settable with the dose setting members,
   wherein the dose definition mechanisms define relative positions, for example relative rotational positions, of the dose setting member with respect to the housing that correspond to the settable dose positions.
12. The set of least two drug delivery devices according to embodiment 11, wherein the dose definition mechanisms of the first and second drug delivery device define the same number of settable doses, for example per revolution of the dose setting member.
13. The set of least two drug delivery devices according to embodiment 11, wherein the dose definition mechanisms of the first and second drug delivery device define different numbers of settable doses, for example per revolution of the dose setting member.
14. The set of least two drug delivery devices according to one of embodiments 11 to 13,
   wherein the dose definition mechanisms of the first and/or second drug delivery device define an uneven number of settable doses, for example per revolution of the dose setting member.
15. The set of at least two drug delivery devices according to one of embodiments 11 to 14,
   wherein the dosing mechanism further comprises a dose selector sleeve which is rotationally fixed to the housing and axially movable with respect to the housing,
   wherein the dose definition mechanism is provided between the dose selector sleeve and the dose setting member.
16. The set of at least two drug delivery devices according to one of embodiments 11 to 15,
   wherein the dose definition mechanisms comprise elastic elements which are in connection with functional features, for example teeth, such that upon actuation of the dose setting member the elastic elements snap into the functional features to provide an audible and/or tactile feedback for a user.
17. The set of at least two drug delivery devices according to embodiment 16, wherein the first drug delivery device and/or the second drug delivery device comprises an even number of elastic elements, for example four elastic elements.
18. The set of at least two drug delivery devices according to one of the preceding embodiments,
   wherein the dose indication member comprises optical markers visualizing the dose set upon actuation of the dose setting member,
   wherein the optical markers of the first drug delivery device are different from the optical markers of the second drug delivery device.
19. The set of at least two drug delivery devices according to one of the preceding embodiments,
   wherein the dose indication member of the first drug delivery device is mechanically identical to the dose indication member of the second drug delivery device.
20. The set of at least two drug delivery devices according to one of embodiments 1 to 18,
   wherein the dose indication member of the first drug delivery device is mechanically different from the dose indication member of the second drug delivery device.
21. The set of at least two drug delivery devices according to any one of the preceding embodiments,
   wherein the dose setting mechanisms further comprise a nut and a clutch mechanism,
   wherein the nut is configured to axially advance the piston rod during dose delivery,
   wherein the clutch mechanism is configured to rotationally couple the nut to a dose setting sleeve in distinct relative rotational positions and to rotationally decouple the nut from the housing during dose setting,
   wherein the clutch mechanism is configured to rotationally decouple the nut from the dose setting sleeve and to rotationally couple the nut to the housing in distinct relative rotational positions during dose delivery,
   wherein, for example, the dose setting sleeve is the dose indication member.
22. The set of at least two drug delivery devices according to embodiment 21, wherein the clutch mechanism is provided between the dose setting member and the dose setting sleeve.
23. The set of at least two drug delivery devices according to one of embodiments 21 and 22,
   wherein the relative rotational positions in which the nut and the dose setting sleeve are coupled to each other during dose setting differ from each other among the first and second drug delivery device and/or
   wherein the relative rotational positions in which the nut and the housing are coupled to each other during dose delivery differ among the first and second drug delivery device.
24. The set of at least two drug delivery devices according to one of embodiments 21 and 22,
   wherein the relative rotational positions in which the nut and the dose setting sleeve are coupled to each other during dose setting of the first and second drug delivery device are identical and/or
   wherein the relative rotational positions in which the nut and the housing are coupled to each other during dose delivery of the first and second drug delivery device are identical.
25. The set of at least two drug delivery devices according to one of embodiments 21 to 24,
   wherein the clutch mechanisms each comprise a clutch member that is connected to the respective dose setting member,
   wherein the dose setting member comprises a first set of clutch elements and the clutch mechanism comprises a second set of clutch elements, wherein a connection between the clutch member and the dose setting member is configured to restrict a relative rotational orientation of the clutch elements of the dose setting member with respect to the clutch elements of the clutch member.
26. The set of at least two drug delivery devices according to embodiment 25, wherein the connection between the clutch member and the dose setting member of the first delivery device is mechanically different from the connection between the clutch member and the dose setting member of the second delivery device.
27. The set of at least two drug delivery devices according to embodiment 26, wherein the connection between the clutch member and the dose setting member of the first delivery device is mechanically identical to the connection between the clutch and the dose setting member of the second delivery device.
28. The set of at least two drug delivery devices according to one of embodiments 25 to 27,
   wherein the connection of the first and/or second drug delivery device is configured to restrict the relative rotational orientation to a single relative rotational orientation.
29. The set of at least two drug delivery devices according to one of embodiments 25 to 27,
   wherein the connection of the first and/or second drug delivery device is configured to restrict the relative rotational orientation to two relative rotational orientations that differ by 180°.
30. The set of at least two drug delivery devices according to any one of the preceding embodiments,
   wherein the dosing mechanisms further each comprise an inner body to support the respective dosing mechanism,
   wherein the inner body is in particular in threaded connection with the dose setting sleeve and with at least one member from said first subset of functional members.
31. The set of at least two drug delivery devices according to any one of the preceding embodiments,
   wherein the dosing mechanisms of the first and second drug delivery device have a dialling resolution that is the same for both devices such that they are configured to expel the same amount of fluid per dose increment settable by the dose setting member, for example 0.010 ml or 0.015 ml.
32. The set of at least two drug delivery devices according to any one of embodiments 1 to 30,
   wherein the dosing mechanisms of the first and second drug delivery device have a dialling resolution that is different for both devices such that they are configured to expel different amounts of fluid per dose increment settable by the dose setting members, for example 0.010 ml and 0.015 ml.
33. The set of at least two drug delivery devices according to any one of the preceding embodiments,
   wherein the piston rods of the first and second drug delivery device comprise a gearing ratio between the movement of the piston rod with respect to the axial movement of a dose actuation member that is identical among the first and second drug delivery device.
34. A set of dispensing units comprising a first dispensing unit and a second dispensing unit,
   each dispensing unit configured to have a drug compartment containing a drug to be delivered by one of the drug delivery devices of the sets according to one of the preceding embodiments,
   wherein the first dispensing unit comprises first connection means that are different from second connection means of the second dispensing unit, wherein the first connection means are configured to only connect to the first connection means of the first drug delivery device and not to the second connection means of the second drug delivery device, and wherein the second connection means are configured to only connect to the second connection means of the second drug delivery device and not to the first connection means of the first drug delivery device.
35. A set of dispensing units according to embodiment 34,
   wherein the first connection means and the second connection means are both configured as a female part or
   wherein the first and second connection means are both configured as a male part.
36. A set of dispensing units according to one of embodiments 34 and 35, wherein the first and second connection means are designed as threads.
37. A set of dispensing units according to embodiment 36,
   wherein the thread of the first connection means has a pitch that is identical to the pitch of the thread of the second connection means.
38. A set of dispensing units according to one of embodiments 36 and 37, wherein ridges of the thread of the first connection means have different dimensions than ridges of the thread of the second connection means.
39. A set of dispensing units according to embodiment 38,
   wherein the ridges of the thread of the first connection means have a width that differs from a width of the ridges of the thread of the second connection means.
40. A set of dispensing units according to one of embodiments 38 to 39, wherein the ridges of the thread of the first connection means have a height that differs from a height of the ridges of the second connection means.
41. A set of dispensing units according to one of embodiments 36 to 40, wherein the threads of the first and second connection means have the same minor diameter and/or the same major diameter.
42. A set of dispensing units according to one of embodiments 34 to 41, wherein the dispensing units comprise cartridge holders for receiving a cartridge which comprises the drug compartment filled with the drug.
43. A set of dispensing units according to embodiment 42,
   wherein the first connection means and the second connection means are provided at the cartridge holder.
44. A set of dispensing units according to one of embodiments 42 to 43, wherein the cartridge holder comprises a connector that is configured to non-releasably, for example permanently, connect the cartridge to the cartridge holder after insertion of the cartridge into the cartridge holder.
45. A set of dispensing units according to embodiment 44,
   wherein the connector is designed as a snap fit connection, for example as a snap hook.
46. A set of dispensing units according to one of embodiments 44 and 45, wherein the connector is provided at a needle end of the dispensing unit.
47. A set of dispensing units according to one of embodiments 44 and 46, wherein the connector is configured to engage with an annular rim of the cartridge, for example to abut against a distal surface of the annular rim that faces away from a needle end of the cartridge holder.
48. A set of dispensing units according to one of embodiments 42 to 47,
   wherein the cartridge holder comprises a push element, for example a flexible member, that is configured to push the cartridge into the cartridge holder after insertion.
49. A set of dispensing units according to embodiment 48,
   wherein the push element is configured to push the cartridge against a stop that is located at a needle end of the cartridge holder.
50. A set of dispensing units according to one of embodiments 34 to 49, wherein the first dispensing unit comprises a fluid containing a drug, for example insulin or HGH, in a first concentration and the second dispensing unit comprises a fluid containing the drug in a second concentration that is different from the first concentration.
51. A kit comprising at least the first drug delivery device of the set according to one of embodiments 1 to 33 and the first dispensing unit of the set according to one of embodiments 34 to 50 and the second drug delivery device of the set according to one of embodiments 1 to 33 and the second dispensing unit of the set according to one of embodiments 34 to 50.
52. The kit according to embodiment 51,
   wherein a dialling resolution of the first drug delivery device differs from a dialling resolution of the second drug delivery device, and
   wherein the first dispensing unit comprises a fluid containing a drug, for example insulin or HGH, in a first concentration and the second dispensing unit comprises a fluid containing the drug in a second concentration that is different from the first concentration.

According to one embodiment of the invention said set of mutual members of the first and second drug delivery device may comprise one or more of the following: cap clip, driver, clutch member, inner body, dose setting member, nut and piston rod.

The corresponding sets of distinguishing members may then comprise one or more of the following: housing, cap body, cartridge holder, dose indication member and dose selector sleeve. In this connection it is noted that said distinguishing members may only be distinguished by their appearance but not by their functionality.

According to another embodiment of the invention the set of mutual members of the first and second drug delivery device may comprise one or more of the following: cap body, driver, inner body, nut and piston rod.

The corresponding sets of distinguishing members may then comprise one or more of the following: housing, cap body, cartridge holder, dose indication member, dose selector sleeve, clutch member and dose setting member. In this connection it is further noted that the distinguishing members may not only be distinguished by their appearance as well as their mechanical functionalities.

According to a third embodiment of the invention the set of mutual members of the first and second drug delivery device may comprise one or more of the following: cap clip, driver, inner body, nut and piston rod.

The corresponding sets of distinguishing members may then comprise one or more of the following: housing, cap body, cartridge holder, dose indication member, dose selector sleeve, clutch member, dose setting member. In this connection it is noted that the distinguishing members may differ in their appearance as well as in their mechanical functionalities.

Further embodiments of the present disclosure relate to:
1. A set of two or more dosing mechanisms and two or more cartridges comprising:
   a first dosing mechanism having a proximal end comprising a first keyed connection means;
   a second dosing mechanism having a proximal end comprising a second keyed connection means;
   a first holder configured to accept and hold a first cartridge containing a first drug, where a distal end of the first holder comprises a third keyed connection means;
   a second holder configured to accept and hold a second cartridge containing a second medicament, where a distal end of the second holder comprises a fourth keyed connection mans and the second medicament is different from the first medicament at least in concentration;
   wherein the first keyed connection means will not form a connection with the fourth keyed connection means and the second keyed connection means will not form a connection with the third keyed connection means, and
   wherein the first keyed connection means can engage and form a connection with the third keyed connection means and the second keyed connection means can engage and form a connection with the fourth keyed connection means.
2. The set of embodiment 1,
   wherein each of the first and second holders has an open proximal end configured to allow axial movement of a piston rod contained within one of the first or second dosing mechanisms such that the piston rod moves beyond the proximal end into the first holder when the first holder is attached to the first dosing mechanism.
3. The set of one of the preceding embodiments,
   wherein the first and second keyed connection means each comprise a thread form.
4. The set of embodiment 3,
   wherein the thread forms each have the same core diameter.
5. The set of one of embodiments 3 and 4,
   wherein the thread forms each have the same outer diameter.
6. The set of one of embodiments 3 to 5,
   wherein the thread form of the first keyed connection means is of opposite hand to the thread form of the second keyed connection means.
7. The set of one of embodiments 3 to 6,
   wherein the thread form of the first keyed connection means has a first pitch and the thread form of the second keyed connection means has a second pitch,
   wherein the first pitch and the second pitch are the same.
8. The set of one of embodiments 3 to 7,
   wherein the thread form of the first keyed connection means has a first thread width w₁, w₂, w₃ and the thread form of the second keyed connection means has a second thread width w₁, w₂, w₃,
   wherein the first thread width w₁, w₂, w₃ differs from the second thread width W1, W2, W3.
9. The set of embodiment 8,
   wherein the second thread width w₁, w₂, w₃ is two times or three times larger than the first thread width w₁, w₂, w₃.
10. The set of one of embodiments 3 to 9,
   wherein the thread form of the first keyed connection means has a first thread height h₁, h₂, h₃ and the thread form of the second keyed connection means has a second thread height h₁, h₂, h₃,
   wherein the first thread height h₁, h₂, h₃ differs from the second thread height h₁, h₂, h₃.
11. The set of embodiment 8,
   wherein the first thread height w₁, w₂, w₃ is two times or three times larger the second thread height w₁, w₂, w₃.
10. The set of one of embodiments 3 to 9,
   wherein the thread form of the first keyed connection means has a first thread geometry and the thread form of the second keyed connection means has a second thread geometry.
   wherein the first thread geometry is different than the second geometry.
11. The set of one of embodiments 3 to 10,
   wherein the first and second keyed connection means each comprise a male thread form.

### Reference numeral list

- 1: cap
- 2: cartridge holder
- 3: outer housing
- 3a: window
- 4: pen needle
- 5: hub
- 6: cannula
- 8: cartridge
- 8a: sealing means
- 9: piston
- 10: further drug delivery device
- 30: dosing mechanism
- 31: dose setting member
- 32: clutch member
- 33: snap element
- 33a: clutch elements
- 34: connector
- 34a: clutch elements
- 34b: ridges
- 33c: flexible arm
- 33d: protrusion
- 33f: further protusion
- 35: dose selector member
- 35a: dose stop
- 35b: maximum stop feature
- 35c: minimum stop feature
- 36: nut
- 37: splined connection
- 38: dosing member
- 40: optical marker
- 41: driver
- 42: piston rod
- 42a: disc
- 43: piston guide
- 60: outer thread
- 61: dose button
- 67: thread
- 63: stop feature
- 64: protrusion
- 81: drug compartment
- 82: annular rim
- 83: distal surface
- 85: annular detent
- 90: rotational biasing member
- 100: resetting mechanism
- 110: resetting element
- 111: gripping zone
- 112: reception area
- 113: inner surface
- 114: opening
- 115: cartridge cavity
- 117: contact structure
- 119: stop
- 120: engagement features
- 130: coupling part/insert
- 134: coupling site
- 135: engagement features
- 136: notch
- 137: first locking structure
- 138: protrusions
- 139: slot
- 140: second locking structure
- 150: biasing element
- 180: inner housing
- 180a: window
- 181: proximal part
- 182: distal part
- 183: inner sleeve
- 184: tappet
- 185: dose thread
- 186: drive thread
- 187: groove
- 188: further groove
- 189: housing cavity
- 190: maximum stop feature
- 191: hook
- 192: limiting surface
- 194: protrusion
- 195: bulge
- 196: zero stop feature
- 197: zero stop surface
- 200: drug delivery device
- 205: proximal end
- 206: distal end
- 207: longitudinal axis
- 209: cap
- 210: housing
- 211: outer housing
- 211a: window
- 213: collar
- 214: detents
- 216: detent
- 218: groove
- 220: first drug delivery device
- 221: first housing
- 222: second drug delivery device
- 223: second housing
- 225: third drug delivery device
- 226: third housing
- 230: dosing mechanism
- 232: dose definition mechanism
- 234: clutch mechanism
- 235: first part
- 236: second part
- 237: clutch mechanism
- 238: first part
- 239: second part
- 240: piston rod
- 241: thread
- 242: plunger disc
- 243: stop feature
- 244: disc connector
- 250: nut
- 251: proximal part
- 252: distal part
- 253: proximal protrusion
- 254: longitudinal groove
- 255: annular detent
- 256: thread
- 270: clutch member
- 271: longitudinal ridges
- 273: clutch elements
- 274: proximal part
- 275: distal part
- 277: connection
- 278: snap hook
- 279: first ridge
- 280: second ridge
- 290: dose setting member
- 292: elastic elements
- 294: clutch elements
- 295: recess
- 296: opening
- 297: first longitudinal groove
- 298: second longitudinal groove
- 308: biasing member
- 310: dose selector member
- 311: distal part
- 312: functional features
- 314: contact surface
- 315: ridge
- 316: further ridge
- 317: proximal part
- 318: connector
- 319: flexible member
- 320: detent
- 322: inner wall
- 323: opening
- 330: dosing member
- 331: optical marker
- 332: proximal part
- 333: distal part
- 334: threaded connection
- 335: outer thread
- 336: clutch elements
- 337: maximum dose stop
- 338: stopping surface
- 340: zero dose stop
- 341: groove
- 343: connector
- 344: annular ridge
- 346: distal end surface
- 350: driver
- 351: proximal part
- 352: threaded connection
- 353: thread
- 354: connection
- 356: flexible arm
- 358: front surface
- 359: distal part
- 360: spline
- 370: first bearing element
- 371: distal disc
- 372: holder
- 373: proximal disc
- 375: ball
- 380: second bearing element
- 402: needle connector
- 404: snap hook
- 406: push element
- 408: stop
- 409: ridge
- 410: dispensing unit
- 412: cartridge holder
- 414: connection means
- 420: first dispensing unit
- 422: first cartridge holder
- 424: first connection means
- 430: second dispensing unit
- 432: second cartridge holder
- 434: second connection means
- 440: third dispensing unit
- 442: third cartridge holder
- 444: third connection means
- 450: contact feature
- 501: ridge
- 502: valleys
- 510: connection means
- 511: first connection means
- 520: second connection means
- 530: third connection means
- P₁: first pitch
- w₁: first width
- h₁: first height
- CD₁: first core diameter
- D₁: first outer diameter
- A₁: first angle
- P₂: second pitch
- w₂: second width
- h₂: second height
- CD₂: second core diameter
- D₂: second outer diameter
- A₂: second angle
- P₃: third pitch
- w₃: third width
- h₃: third height
- CD₃: third core diameter
- D₃: third outer diameter
- A₃: third angle

## Claims

1. Set of at least two drug delivery devices (10, 200, 220, 222, 225) comprising at least a first drug delivery device and a second drug delivery device, each one of the first and second drug delivery device comprising
a housing (3, 43, 210, 221, 223, 226),
a dosing mechanism (30, 230) within said housing (3, 43, 210, 221, 223, 226) comprising
a dose setting member (31, 290) for setting an injection dose, a dose indication member (330) for visual indication of the set dose, and a piston rod (42, 240) that is configured to move in a proximal direction out of the housing (3, 43, 210, 221, 223, 226) for ejecting the drug, the piston rod (42, 240) further being operatively coupled to the dose setting member (31, 290),
wherein the first drug delivery device (10, 200, 220, 222, 225) and the second drug delivery device (10, 200, 220, 222, 225) each comprise a first set of mutual members that are identical in both the first drug delivery device (10, 200, 220, 222, 225) and the second drug delivery device (10, 200, 220, 222, 225), the first set comprising at least one mutual member,
wherein the first drug delivery device (10, 200, 220, 222, 225) and the second drug delivery device (10, 200, 220, 222, 225) each comprise a second set of distinguishing members that differ among the first and second drug delivery device (10, 200, 220, 222, 225), the second set comprising at least one distinguishing member,
wherein the first drug delivery device (10, 200, 220, 222, 225) comprises first connection means (510, 511, 520, 530) for attaching a first dispensing unit (410, 420, 430, 440) to the housing (3, 43, 210, 221, 223, 226) of the first drug delivery device (10, 200, 220, 222, 225) and the second drug delivery device (10, 200, 220, 222, 225) comprises second connection means (510, 511, 520, 530) for attaching a second dispensing unit (410, 420, 430, 440) to the housing (3, 43, 210, 221, 223, 226) of the second drug delivery device (10, 200, 220, 222, 225), wherein the first and second connection means (510, 511, 520, 530) differ from each other so that the second dispensing unit (410, 420, 430, 440) is not attachable to the first connection means (510, 511, 520, 530) and the first dispensing unit (410, 420, 430, 440) is not attachable to the second connection means (510, 511, 520, 530).

2. The set of at least two drug delivery devices (10, 200, 220, 222, 225) according to claim 1, wherein the dosing mechanisms (30, 230) of the first drug delivery device (10, 200, 220, 222, 225) and the second drug delivery device (10, 200, 220, 222, 225) each comprise, for example consist of, a same set of functional members that includes the first set and the second set.

3. The set of at least two drug delivery devices (10, 200, 220, 222, 225) according to any one of the preceding claims,
wherein the dosing mechanisms (30, 230) each comprise a dose definition mechanism (232) that acts between the dose setting member (31, 290) and the housing (3, 43, 210, 221, 223, 226) and wherein the dose definition mechanisms (232) are configured to define dose positions that are settable with the dose setting members (31, 290),
wherein the dose definition mechanisms (232) define relative positions, for example relative rotational positions, of the dose setting member (31, 290) with respect to the housing (3, 43, 210, 221, 223, 226) that correspond to the settable dose positions,
wherein, for example, the dose definition mechanisms (232) of the first and second drug delivery device (10, 200, 220, 222, 225) define the same number of settable doses, for example per revolution of the dose setting member (31, 290), or
wherein, for example, the dose definition mechanisms (232) of the first and second drug delivery device (10, 200, 220, 222, 225) define different numbers of settable doses, for example per revolution of the dose setting member (31, 290).

4. The set of at least two drug delivery devices (10, 200, 220, 222, 225) according to claim 3,
wherein the dosing mechanism (30, 230) further comprises a dose selector member (35, 310) which is rotationally fixed to the housing (3, 43, 210, 221, 223, 226) and axially movable with respect to the housing (3, 43, 210, 221, 223, 226),
wherein the dose definition mechanism (232) is provided between the dose selector member (35, 310) and the dose setting member (31, 290).

5. The set of at least two drug delivery devices (10, 200, 220, 222, 225) according to one of claims 3 or 4,
wherein the dose definition mechanisms (232) comprise elastic elements (33c, 33d, 292) which are in connection with functional features (35a, 312), for example dose stops, such that upon actuation of the dose setting member (31, 290) the elastic elements (33c, 33d, 292) snap into the functional features (35a, 312) to provide an audible and/or tactile feedback for a user,
wherein, for example, the first drug delivery device (10, 200, 220, 222, 225) and/or the second drug delivery device (10, 200, 220, 222, 225) can comprise an even number of elastic elements (33c, 33d, 292), for example four elastic elements (33c, 33d, 292).

6. The set of at least two drug delivery devices (10, 200, 220, 222, 225) according to one of the preceding claims,
wherein the dose indication member (330) comprises optical markers (331) visualizing the dose set upon actuation of the dose setting member (31, 290),
wherein the optical markers (331) of the first drug delivery device (10, 200, 220, 222, 225) are different from the optical markers (331) of the second drug delivery device (10, 200, 220, 222, 225),
in particular wherein the dose indication member (330) of the first drug delivery device (10, 200, 220, 222, 225) can be mechanically identical to the dose indication member (330) of the second drug delivery device (10, 200, 220, 222, 225), or
wherein the dose indication member (330) of the first drug delivery device (10, 200, 220, 222, 225) can be mechanically different from the dose indication member (330) of the second drug delivery device (10, 200, 220, 222, 225).

7. The set of at least two drug delivery devices (10, 200, 220, 222, 225) according to any one of the preceding claims,
wherein the dosing mechanisms (30, 230) further comprise a nut (36, 250) and a clutch mechanism (234, 237),
wherein the nut (36, 250) is configured to axially advance the piston rod (42, 240) during dose delivery,
wherein the clutch mechanism (234, 237) is configured to rotationally couple the nut (36, 250) to a dosing member (330) in distinct relative rotational positions and to rotationally decouple the nut (36, 250) from the housing (3, 43, 210, 221, 223, 226) during dose setting,
wherein the clutch mechanism (234, 237) is configured to rotationally decouple the nut (36, 250) from the dosing member (330) and to rotationally couple the nut (36, 250) to the housing (3, 43, 210, 221, 223, 226) in distinct relative rotational positions during dose delivery, wherein, for example, the dosing member (330) is the dose indication member (330) and/or
wherein, for example, the clutch mechanism (234, 237) is provided between the dose setting member (31, 290) and the dosing member (330).

8. The set of at least two drug delivery devices (10, 200, 220, 222, 225) according to claim 7,
wherein the relative rotational positions in which the nut (36, 250) and the dosing member (330) are coupled to each other during dose setting differ from each other among the first and second drug delivery device (10, 200, 220, 222, 225) and/or
wherein the relative rotational positions in which the nut (36, 250) and the housing (3, 43, 210, 221, 223, 226) are coupled to each other during dose delivery differ among the first and second drug delivery device (10, 200, 220, 222, 225), or
wherein the relative rotational positions in which the nut (36, 250) and the dosing member (330) are coupled to each other during dose setting of the first and second drug delivery device (10, 200, 220, 222, 225) are identical and/or
wherein the relative rotational positions in which the nut (36, 250) and
the housing (3, 43, 210, 221, 223, 226) are coupled to each other during dose delivery of the first and second drug delivery device (10, 200, 220, 222, 225) are identical.

9. The set of at least two drug delivery devices (200, 220, 222, 225) according to one of claims 7 or 8,
wherein the clutch mechanisms (234) each comprise a clutch member (270) that is connected to the respective dose setting member (290), wherein the dose setting member (290) comprises a first set of clutch elements (294) and the clutch member (270) comprises a second set of clutch elements (273),
wherein a connection (277) between the clutch member (270) and the dose setting member (290) is configured to restrict a relative rotational orientation of the clutch elements (294) of the dose setting member (290) with respect to the clutch elements (273) of the clutch member (270),
wherein the connection (277) between the clutch member (270) and the dose setting member (290) of the first drug delivery device (200, 220, 222, 225) is mechanically different from the connection (277) between the clutch member (270) and the dose setting member (290) of the second drug delivery device (200, 220, 222, 225).

10. The set of at least two drug delivery devices (200, 220, 222, 225) according to one of claims 7 or 8,
wherein the clutch mechanisms (234) each comprise a clutch member (270) that is connected to the respective dose setting member (290), wherein the dose setting member (290) comprises a first set of clutch elements (294) and the clutch member (270) comprises a second set of clutch elements (273),
wherein a connection (277) between the clutch member (270) and the dose setting member (290) is configured to restrict a relative rotational orientation of the clutch elements (294) of the dose setting member (290) with respect to the clutch elements (273) of the clutch member (270),
wherein the connection (277) between the clutch member (270) and the dose setting member (290) of the first drug delivery device (200, 220, 222, 225) is mechanically identical to the connection (277) between the clutch member (270) and the dose setting member (31, 290) of the second delivery device (200, 220, 222, 225).

11. The set of at least two drug delivery devices (200, 220, 222, 225) according to one of claims 9 and 10,
wherein the connection (277) of the first and/or second drug delivery device (200, 220, 222, 225) is configured to restrict the relative rotational orientation to a single relative rotational orientation, or wherein the connection (277) of the first and/or second drug delivery device (10, 200, 220, 222, 225) is configured to restrict the relative rotational orientation to two relative rotational orientations that differ by 180°.

12. The set of at least two drug delivery devices (10, 200, 220, 222, 225) according to any one of the preceding claims,
wherein the dosing mechanisms (30, 230) further comprise an inner body (43, 180) to support the dosing mechanism (30, 230) and to transfer the movement of the dose setting member (31, 290) to the piston rod (42, 240),
wherein the inner body (180) is, for example, in threaded connection with the dosing member (330) and with at least one member from said first subset of mutual members.

13. The set of at least two drug delivery devices (10, 200, 220, 222, 225) according to any one of the preceding claims,
wherein the dosing mechanisms (30, 230) of the first and second drug delivery device (10, 200, 220, 222, 225) have a dialling resolution that is the same for both drug delivery device (10, 200, 220, 222, 225) such that they are configured to expel the same amount of fluid per dose increment settable by the dose setting member (31, 290), for example 0.010 ml or 0.015 ml, or
wherein the dosing mechanism (30, 230) of the first and second drug delivery device (10, 200, 220, 222, 225) have a dialling resolution that is different for both drug delivery device (10, 200, 220, 222, 225) such that they are configured to expel a different amount of fluid per dose increment settable by the dose setting members (31, 290), for example 0.010 ml and 0.015 ml.

14. The set of at least two drug delivery devices (10, 200, 220, 222, 225) according to any one of the preceding claims,
wherein the piston rod (42, 240) is rotationally fixed with respect to the housing (3, 43, 210, 221, 223, 226) at least during dose delivery.

15. A set of dispensing units (410, 420, 430, 440) comprising a first dispensing unit (410, 420, 430, 440) and a second dispensing unit (410, 420, 430, 440),
each dispensing unit (410, 420, 430, 440) configured to have a drug compartment (81) containing a drug to be delivered by one of the drug delivery devices (10, 200, 220, 222, 225) of the sets according to one of the preceding claims,
wherein the first dispensing unit (410, 420, 430, 440) comprises first connection means (414, 424, 434, 444) that are different from second connection means (414, 424, 434, 444) of the second dispensing unit (410, 420, 430, 440),
wherein the first connection means (414, 424, 434, 444) are configured to only connect to the first connection means (510, 511, 520, 530) of the first drug delivery device (10, 200, 220, 222, 225) and not to the second connection means (510, 511, 520, 530) of the second drug delivery device (10, 200, 220, 222, 225) and,
wherein the second connection means (414, 424, 434, 444) are configured to only connect to the second connection means (510, 511, 520, 530) of the second drug delivery device (10, 200, 220, 222, 225) and not to the first connection means (510, 511, 520, 530) of the first drug delivery device (10, 200, 220, 222, 225).

16. The set of dispensing units (410, 420, 430, 440) according to claim 15, wherein the dispensing units (410, 420, 430, 440) comprise cartridge holders (2, 412, 422, 432, 432) for receiving a cartridge which comprises the drug compartment (81) filled with the drug,
in particular wherein the first connection means (510, 511, 520, 530) and the second connection means (510, 511, 520, 530) can be provided at the cartridge holder (2, 412, 422, 432, 432), and/or
wherein the cartridge holder (2, 412, 422, 432, 432) comprises a connector (404) that is configured to non-releasably, for example permanently, connect the cartridge to the cartridge holder (2, 412, 422, 432, 432) during use of the dispensing unit (410, 420, 430, 440),
wherein, for example, the connector (404) is designed as a snap fit connection, for example as a snap hook, and/or
wherein the connector (404) is configured to engage with an annular rim (82) of the cartridge (8), for example to abut against a distal surface (83) of the annular rim (82) that faces away from a needle end of the cartridge holder (2, 412, 422, 432, 432).

17. The set of dispensing units (410, 420, 430, 440) according to claim 16, wherein the cartridge holder (2, 412, 422, 432, 432) comprises a push element (406), for example a flexible member, that is configured to push the cartridge (8) into the cartridge holder (2, 412, 422, 432, 432) after insertion,
in particular wherein the push element (406) can be configured to push the cartridge (8) against a stop (408) that is located at a needle end of the cartridge holder (2, 412, 422, 432, 432).
